(19) Europäisches Patentamt / European Patent Office / Office européen des brevets

(11) **EP 3 627 513 A1**

(12) **EUROPEAN PATENT APPLICATION**

(43) Date of publication:
**25.03.2020 Bulletin 2020/13**

(51) Int Cl.:
***G16B 20/20*** *(2019.01)* ***G16B 30/20*** *(2019.01)*

(21) Application number: **19193572.5**

(22) Date of filing: **26.08.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(30) Priority: **31.08.2018 JP 2018163953**<br><br>(71) Applicants:<br>• **SYSMEX CORPORATION**<br>  **Kobe-shi**<br>  **Hyogo 651-0073 (JP)** | • **Riken Genesis Co., Ltd.**<br>  **Tokyo 141-0032 (JP)**<br><br>(72) Inventors:<br>• **Washio, Takanori**<br>  **Tokoyo, 141-0032 (JP)**<br>• **Watanabe, Reiko**<br>  **Hyogo, 651-0073 (JP)**<br><br>(74) Representative: **Hoffmann Eitle**<br>  **Patent- und Rechtsanwälte PartmbB**<br>  **Arabellastraße 30**<br>  **81925 München (DE)** |

(54) **ANALYSIS METHOD, INFORMATION PROCESSING APPARATUS, GENE ANALYSIS SYSTEM, AND NON-TRANSITORY STORAGE MEDIUM**

(57) Provided is an analysis method including: obtaining sequence information of nucleic acid contained in a measurement sample prepared by mixing a sample that contains subject-derived nucleic acid and a sample that contains non-subject-derived nucleic acid such that the measurement sample contains a previously determined amount of nucleic acid; and performing analysis on the obtained sequence information, wherein the performing of the analysis includes performing analysis on sequence information of the subject-derived nucleic acid in the obtained sequence information, and not performing, on sequence information of the non-subject-derived nucleic acid in the obtained sequence information, at least a part of the analysis to be performed on the sequence information of the subject-derived nucleic acid.

FIG. 4

**Description**

FIELD OF THE INVENTION

The present invention relates to an analysis method, an information processing apparatus, and the like that analyze base sequences of genes.

BACKGROUND

Conventionally, technologies that analyze base sequences of genes have been utilized as important analysis techniques in the fields of basic study, clinical study, medical care, and the like. In recent years, panel tests using gene panels that allow comprehensive checking of abnormalities in genes of a subject (for example, a patient) by use of next generation sequencing (NGS) have been developed. Such panel tests are expected to play an important role in individualized medical care. Here, individualized medical care denotes medical care in which an appropriate therapeutic strategy is selected for each patient in consideration of characteristics such as the genetic background, the physiological condition, and the state of the disease of the patient.

Among technologies that analyze base sequences of genes, NGS is an indispensable technology for comprehensively detecting abnormalities in base sequences in genes. For example, "An introduction to Next-Generation Sequencing Technology", [online], Illumina, Inc. [searched on August 30, 2018], Internet <https://www.illumina.com/content/dam/illumina-marketing/documents/products/illumina_sequencing_introduction.pdf> describes a technique of simultaneously analyzing base sequences of genes derived from samples of a plurality of subjects by use of NGS.

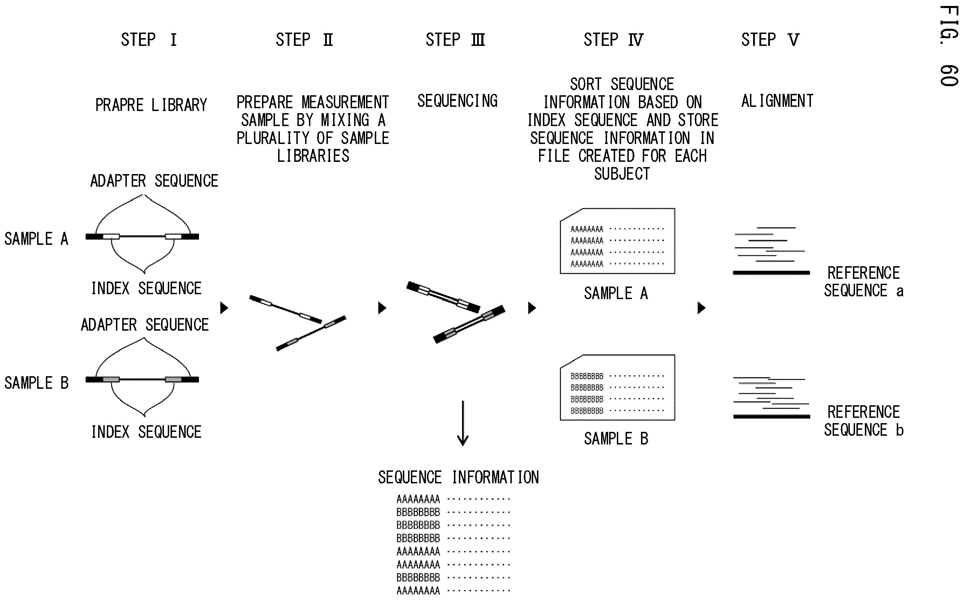

When base sequences of genes of a plurality of subjects are simultaneously analyzed by use of NGS, the analysis is performed through steps I to V shown in FIG. 60, for example. FIG. 60 shows one example of steps performed in order to simultaneously analyze base sequences of genes of a plurality of subjects.

In step I, a sample A and a sample B are fragmented, and a library A of the sample A and a library B of the sample B are prepared. Here, the "sample A" may be genes derived from a tissue collected from a subject A, and the "sample B" may be genes derived from a tissue collected from a subject B, for example. In this step, adapter sequences are added to fragments of the sample A and the sample B. In this step, an index sequence 1 ("AAAAAAAA" in the drawing) is added to fragments (for example, DNA fragments) of the sample A, and an index sequence 2 ("BBBBBBBB" in the drawing) is added to fragments (for example, DNA fragments) of the sample B. The "adapter sequence" is an oligonucleotide that is added to each fragment in order to capture the fragment so that sequencing reaction is caused in a flow cell for a sequencer that performs sequencing. The "index sequence" is an oligonucleotide that has a length of several bases to several tens of bases and that is added to each fragment in order to distinguish sequence information derived from fragments of the sample A, from sequence information derived from fragments of the sample B in a later step IV.

Subsequently, in step II, the library A and the library B are mixed together, and the mixture is applied to a flow cell. In step III, sequencing reaction occurs on the flow cell, and sequence information is obtained. The obtained sequence information includes base sequence data of fragments of the sample A and base sequence data of fragments of the sample B.

Next, in step IV, sequence information is sorted on the basis of the index sequence included therein, and the sorted sequence information is stored in a file created for each sample. Then, in step V, sequence information is read out from each file, and alignment is performed for each of the sample A and the sample B.

When base sequences of genes of a plurality of subjects are simultaneously analyzed by use of NGS, each step is determined on the basis of a protocol that is recommended for the sequencer to be used and the gene panel to be used. In addition, various reference values determined on the basis of the recommended protocol are set for an existing analysis program that is to be used in the analysis of the base sequences of genes.

For example, a suitable protocol is recommended in accordance with the specification of a flow cell that suits the sequencer to be used, and in accordance with the amounts and the like of a primer and a probe included in the gene panel. Therefore, in steps I and II shown in FIG. 60, libraries that contain a previously determined amount of nucleic acid are prepared so that the total amount of nucleic acid to be subjected to one sequencing run becomes the optimum amount. Then, the prepared libraries are applied to the flow cell.

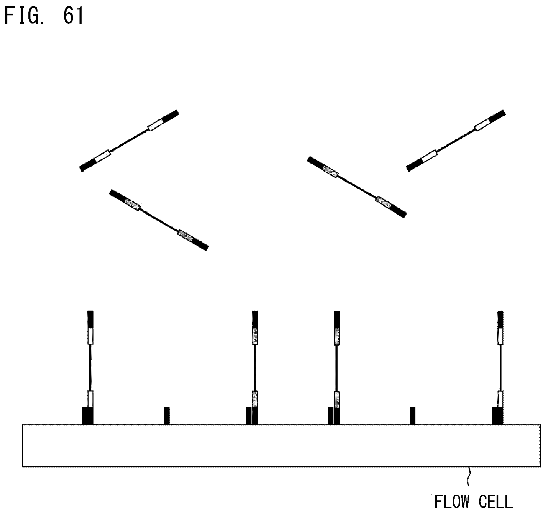

For example, as shown in FIG. 61, a certain number of oligo DNA (for example, several-ten thousand molecules) that function as capture molecules for capturing DNA fragments whose base sequences are to be read are immobilized on the surface of the flow path of the flow cell. Sequencing reaction is caused on the nucleic acid, in the nucleic acid having been applied to the flow cell, which has been captured by the oligo DNA on the flow cell. Accordingly, sequence information is obtained. Therefore, if the amount of nucleic acid applied to the flow cell is increased, the number of nucleic acid fragments captured by the oligo DNA on the flow cell is increased. If the amount of nucleic acid applied to the flow cell is decreased, the number of nucleic acid fragments captured by the oligo DNA on the flow cell is decreased. That is, if the number of nucleic acid fragments captured on the flow cell varies, the density on the flow cell of DNA fragments that are in contact with the primer and the probe in the sequencing reaction on the flow cell varies. The amounts of the primer and the probe to be subjected to the sequencing reaction are constant.

Therefore, if the density on the flow cell of DNA fragments varies, results of sequencing reaction will vary. Accordingly, measurement results of constant quality cannot be obtained. In order to prevent occurrence of variation in the results of sequencing reaction, it is necessary to apply, to the flow cell, samples that are mixed such that the amount of nucleic acid to be applied to the flow cell becomes a previously determined amount of nucleic acid.

However, there are conceivable cases where an ideal number of samples that should be applied to the flow cell cannot be obtained, such as when the number of subject-derived samples is small. In addition, in some cases, it could become necessary to perform analysis again on only some of the samples that have already been analyzed. If the number of samples to be subjected to one sequencing run varies, the data amount of sequence information obtained per sample will vary. This is because, since the total amount of nucleic acid contained in the libraries to be applied to a flow cell needs to be constant, the amount of nucleic acid per sample in the total amount of nucleic acid molecules applied to the flow cell will vary.

For example, when sequencing is performed using a number of samples (for example, 16 samples) that is 1/3 an ideal number of samples (for example, 48 samples), the amount of nucleic acid per sample is three times the amount obtained when sequencing is performed using the ideal number of samples. As a result, the data amount of sequence information obtained per sample is likely to be three times the data amount obtained when sequencing is performed using the ideal number of samples.

In order to keep the quality of the analysis result of gene base sequences constant, it is desirable that the data amount of sequence information obtained per sample does not vary for each sequencing run. However, if the data amount of sequence information obtained per sample varies due to variation of the number of samples subjected to a sequencing run, it becomes necessary, in accordance with the result, to modify the existing analysis program used in analysis of gene base sequences, for example.

In order to utilize NGS in the medical field to help determination of diagnoses and therapies for diseases of subjects, it is important to output analysis results of a quality that is always constant. Thus, it is desirable that, even when the number of samples to be subjected to one sequencing run varies, the data amount of sequence information obtained per sample is kept constant, and the existing analysis program is used as it is.

SUMMARY OF THE INVENTION

The scope of the present invention is defined solely by the appended claims, and is not affected to any degree by the statements within this summary.

In order to solve the above problem, an analysis method according to one aspect of the present invention includes: obtaining sequence information of nucleic acid contained in a measurement sample prepared by mixing a sample that contains subject-derived nucleic acid and a sample that contains non-subject-derived nucleic acid such that the measurement sample contains a previously determined amount of nucleic acid (S1); and performing analysis on the obtained sequence information (S3). The performing of the analysis includes performing analysis on sequence information of the subject-derived nucleic acid in the obtained sequence information (S52), and not performing, on sequence information of the non-subject-derived nucleic acid in the obtained sequence information, at least a part of the analysis to be performed on the sequence information of the subject-derived nucleic acid (S53).

Here, the "subject" means, for example, a patient or the like who has a gene test such as a panel test. The "measurement sample" means a sample to be prepared so as to be subjected to sequencing. The "previously determined amount of nucleic acid" means the amount of nucleic acid determined on the basis of a protocol recommended for a sequencer 2 to be used and reagents to be used. That is, the "previously determined amount of nucleic acid" is an amount of nucleic acid realized when the number of samples is not smaller than the recommended number of samples to be subjected to one sequencing run. The "number of samples" means the number of samples whose sequence information is individually obtained. For example, in a case where one sample that contains nucleic acid extracted from a tissue and one sample that contains nucleic acid extracted from blood are prepared for one subject, the number of samples per subject is 2.

According to the above configuration, the measurement sample is prepared by mixing a sample that contains subject-derived nucleic acid, and a sample that contains non-subject-derived nucleic acid such that the measurement sample contains a previously determined amount of nucleic acid. Then, analysis is performed on sequence information of the subject-derived nucleic acid in the obtained sequence information, and at least a part of the analysis to be performed on the sequence information of the subject-derived nucleic acid is not performed on sequence information of the non-subject-derived nucleic acid.

If the above analysis method is employed, even when the number of samples that contain subject-derived nucleic acid for preparing a measurement sample varies in gene tests, analysis of constant quality can be efficiently performed on the sequence information of the subject-derived nucleic acid.

The analysis method may be configured such that the sequence information of the subject-derived nucleic acid includes an index sequence, and analysis is performed on sequence information that includes the index sequence, in the sequence information of the nucleic acid of the measurement sample.

The analysis method may be configured such that the sequence information of the subject-derived nucleic acid includes a plurality of pieces of sequence information of nucleic acid derived from a plurality of subjects, and sequence information of nucleic acid derived from a different subject includes a different index sequence.

The analysis method may be configured such that the sequence information of the non-subject-derived nucleic acid does not include an index sequence.

The analysis method may be configured such that analysis is performed on sequence information that includes an index sequence in the sequence information of the nucleic acid of the measurement sample (S52), and at least a part of the analysis to be performed on the sequence information that includes the index sequence is not performed on sequence information that does not include the index sequence (S53).

The analysis method may be configured such that the sequence information of the nucleic acid of the measurement sample includes sequence information that includes a first index sequence and sequence information that includes a second index sequence different from the first index sequence, and analysis is performed on the sequence information that includes the first index sequence, and at least a part of the analysis to be performed on the sequence information that includes the first index sequence is not performed on the sequence information that includes the second index sequence.

In the above configuration, the analysis may include obtaining information related to a gene of the subject, on the basis of the sequence information of the subject-derived nucleic acid. In the above configuration, information related to a gene of the subject may include a gene name corresponding to the sequence information and mutation information of the gene.

The preparing of the measurement sample (S304b) may include preparing a measurement sample having further added thereto a quality control sample for evaluating quality of sequence information, and the analysis method may further include performing a process for obtaining information related to quality of the measurement sample from sequence information of the quality control sample (S 110).

The analysis method may be configured such that nucleic acid of the quality control sample is identical to the non-subject-derived nucleic acid, and may further include performing a process for obtaining information related to quality, on at least a part of the sequence information of the non-subject-derived nucleic acid in the sequence information of the nucleic acid of the measurement sample.

Irrespective of the number of samples that contain subject-derived nucleic acid and that have been used in the preparing of the measurement sample (S304b), an amount of nucleic acid derived from each sample in the measurement sample may be substantially identical.

A data amount of sequence information per sample in the sequence information, of the nucleic acid of the measurement sample, obtained in the obtaining of the sequence information (S1) may be substantially identical.

Accordingly, the quality of the analysis of sequence information of subject-derived nucleic acid can be kept at a constant level.

A data amount of sequence information per sample in the sequence information, of the nucleic acid of the measurement sample, obtained in the obtaining of the sequence information (S1) may account for a predetermined proportion in a data amount of the sequence information of the nucleic acid of the measurement sample, irrespective of the number of samples that contain subject-derived nucleic acid and that have been used in the preparing of the measurement sample.

Here, the predetermined proportion is a value that is determined in accordance with the number, of samples containing subject-derived nucleic acid, which is recommended for preparing a measurement sample, for example.

When the number of samples that contain subject-derived nucleic acid and that have been used in the preparing of the measurement sample has been changed, variation in a data amount of sequence information per sample in the sequence information of the nucleic acid of the measurement sample may be in a range of $\pm 10\%$.

According to the above configuration, the quality of the analysis result of gene base sequences can be kept in a range allowable for a test result of a gene test such as a panel test.

A data amount of the sequence information of the non-subject-derived nucleic acid in the sequence information obtained in the obtaining of the sequence information (S1) may be greater than or equal to a data amount of sequence information per sample in the sequence information, of the nucleic acid of the measurement sample, obtained in the obtaining of the sequence information (S1).

Even when the data amount of the sequence information of the non-subject-derived nucleic acid is increased, the quality of the sequence information of the subject-derived nucleic acid is not influenced.

The amount of the nucleic acid may be the number of moles of the nucleic acid. The number of moles of nucleic acid can be calculated on the basis of measurement values such as absorbance at 260 nm, an average molecular weight, a molar absorption coefficient of nucleic acid, and the like.

The obtaining of the sequence information of the nucleic acid contained in the measurement sample (S1) may include obtaining sequence information of nucleic acid of the measurement sample, captured by capture molecules for capturing the nucleic acid. Each capture molecule may include a base sequence complementary to at least a part of the nucleic acid contained in the measurement sample.

The sequence information may be a base sequence of the nucleic acid read by a sequencer.

In order to solve the above problem, an information processing apparatus (1) according to another aspect of the present invention includes a controller (11) and a storage unit (12). The controller (11) is programmed to obtain sequence information of nucleic acid of a measurement sample prepared by mixing a sample that contains subject-derived nucleic acid and a sample that contains non-subject-derived nucleic acid such that the measurement sample contains a previously determined amount of nucleic acid, and store the sequence information into the storage unit (12), and perform analysis on the obtained sequence information. The controller is programmed to perform analysis on sequence information of the subject-derived nucleic acid in the obtained sequence information, and not to perform, on sequence information of the non-subject-derived nucleic acid in the obtained sequence information, at least a part of the analysis to be performed on the sequence information of the subject-derived nucleic acid.

According to the above configuration, the information processing apparatus (1) obtains sequence information, performs analysis on sequence information of subject-derived nucleic acid, and does not perform, on sequence information of non-subject-derived nucleic acid, at least a part of the analysis to be performed on the sequence information of the subject-derived nucleic acid. Accordingly, for example, even when the number of samples that contain subject-derived nucleic acid for preparing a measurement sample varies in gene tests, analysis of constant quality can be efficiently performed.

The analysis to be performed on the obtained sequence information may include an alignment process (S12) for mapping the obtained sequence information on a reference sequence, and may be configured such that the alignment process is not performed on the sequence information of the non-subject-derived nucleic acid.

For example, in a case where "PhiX DNA" (Illumina, Inc.) which is nucleic acid derived from bacteriophage is used as the sample that contains non-subject-derived nucleic acid, there is no need to perform the alignment process. According to the above configuration, unnecessary processes can be appropriately omitted.

The analysis to be performed on the obtained sequence information may include a mutation extraction process (S14) for extracting a mutation of nucleic acid, and may be configured such that the mutation extraction process is not performed on the sequence information of the non-subject-derived nucleic acid.

For example, when a quality control sample for evaluating the quality of sequence information is used as the sample that contains non-subject-derived nucleic acid, there is no need to perform the mutation extraction process. According to the above configuration, unnecessary processes can be appropriately omitted.

In order to solve the above problem, a gene analysis system (100) according to another aspect of the present invention includes a sequencer (2) configured to read sequence information of nucleic acid of a measurement sample prepared by mixing a sample that contains subject-derived nucleic acid and a sample that contains non-subject-derived nucleic acid such that the measurement sample contains a previously determined amount of nucleic acid; and an information processing apparatus (1) configured to perform analysis on the sequence information that has been obtained. The information processing apparatus performs analysis on sequence information of the subject-derived nucleic acid in the obtained sequence information, and does not perform, on sequence information of the non-subject-derived nucleic acid in the obtained sequence information, at least a part of the analysis to be performed on the sequence information of the subject-derived nucleic acid.

According to the above configuration, the sequencer (2) performs sequencing on a measurement sample prepared by mixing a sample that contains subject-derived nucleic acid and a sample that contains non-subject-derived nucleic acid such that the measurement sample contains a previously determined amount of nucleic acid. Then, the information processing apparatus (1) obtains sequence information, performs analysis on sequence information of the subject-derived nucleic acid, and does not perform, on sequence information of the non-subject-derived nucleic acid in the obtained sequence information, at least a part of the analysis to be performed on the sequence information of the subject-derived nucleic acid.

Accordingly, for example, even when the number of samples that contain subject-derived nucleic acid for preparing a measurement sample varies in gene tests, analysis of constant quality can be efficiently performed.

In order to solve the above problem, a program according to another aspect of the present invention is configured to cause a computer to perform: obtaining sequence information of nucleic acid of a measurement sample prepared by mixing a sample that contains subject-derived nucleic acid and a sample that contains non-subject-derived nucleic acid such that the measurement sample contains a previously determined amount of nucleic acid (S1); and performing analysis on the obtained sequence information (S52). In the performing of the analysis, the computer performs analysis on sequence information of the subject-derived nucleic acid in the obtained sequence information (S52), and does not perform, on sequence information of the non-subject-derived nucleic acid in the obtained sequence information, at least a part of the analysis to be performed on the sequence information of the subject-derived nucleic acid (S53).

According to this configuration, for example, even when the number of samples that contain subject-derived nucleic acid for preparing a measurement sample varies in gene tests, analysis of constant quality can be efficiently performed.

A non-transitory computer-readable storage medium having the above program stored therein is also included in the scope of the present invention.

An analysis method according to another aspect of the present invention includes: obtaining sequence information of nucleic acid contained in a measurement sample prepared by mixing a sample that contains subject-derived nucleic acid and a sample that contains non-subject-derived nucleic acid such that the measurement sample contains a previously determined amount of nucleic acid; selecting sequence information of the subject-derived nucleic acid among the obtained sequence information; and performing analysis on the selected sequence information.

The present invention can also be described as follows.

An analysis method according to one aspect of the present invention includes: obtaining sequence information of nucleic acid contained in a measurement sample prepared by mixing a sample that contains subject-derived nucleic acid and a sample that contains non-subject-derived nucleic acid such that the measurement sample contains a previously determined amount of nucleic acid (S1); and outputting sequence information in which a data amount of sequence information per sample that contains subject-derived nucleic acid is a predetermined amount irrespective of the number of samples that contain subject-derived nucleic acid and that have been used in preparing the measurement sample (S2).

Here, the "subject" means, for example, a patient or the like who has a gene test such as a panel test. The "measurement sample" means a sample to be prepared so as to be subjected to sequencing. The "previously determined amount of nucleic acid" means the amount of nucleic acid determined on the basis of a protocol recommended for a sequencer 2 to be used and reagents to be used. That is, the "previously determined amount of nucleic acid" is an amount of nucleic acid realized when the number of samples is not smaller than the recommended number of samples to be subjected to one sequencing run. The "predetermined amount" means the data amount of sequence information per sample obtained when the measurement sample is prepared by use of a recommended number of samples.

According to the above configuration, the measurement sample is prepared so as to contain a previously determined amount of nucleic acid by mixing a sample that contains subject-derived nucleic acid and a sample that contains non-subject-derived nucleic acid. Then, sequence information of the nucleic acid of the measurement sample is obtained, and sequence information in which the data amount of sequence information per sample is the predetermined amount is outputted.

In order to keep the reliability of the analysis result of the sequence information constant, the quality of the sequence information needs to be appropriately evaluated. If the above analysis method is employed, even when the number of samples that contain subject-derived nucleic acid for preparing a measurement sample varies in gene tests, the variation in the data amount of sequence information per sample can be kept in a predetermined range, and an analysis result of constant quality can be outputted. Thus, even when the number of samples that contain subject-derived nucleic acid for preparing a measurement sample to be subjected to one sequencing run is smaller than the recommended number of samples, the variation in the data amount of sequence information per sample can be kept in a predetermined range.

In the preparing of the measurement sample (S304b), the amount of nucleic acid derived from each sample that contains subject-derived nucleic acid in the measurement sample may be substantially identical.

In the outputting of the sequence information (S2), the data amount of the sequence information per sample may account for a predetermined proportion in a data amount of the obtained sequence information of the nucleic acid of the measurement sample, irrespective of the number of samples that contain subject-derived nucleic acid and that have been used in the preparing of the measurement sample.

When the number of samples that contain subject-derived nucleic acid and that have been used in the preparing of the measurement sample has been changed, variation in the data amount of the sequence information per sample may be in a range of ±10%.

According to the above configuration, the quality of the analysis result of gene base sequences can be kept in a range allowable for a test result of a gene test such as a panel test.

A data amount of sequence information of the non-subject-derived nucleic acid in the sequence information obtained in the obtaining of the sequence information (S1) may be greater than or equal to the data amount of the sequence information per sample.

Even when the data amount of the sequence information of the non-subject-derived nucleic acid is increased, the quality of the sequence information of the subject-derived nucleic acid is not influenced.

In the above configuration, the predetermined proportion is not dependent on the number of samples that contain subject-derived nucleic acid and that have been used in the preparing of the measurement sample (S304b).

A first measurement sample may be prepared by mixing samples that contain nucleic acid derived from a first subject group and a sample that contains non-subject-derived nucleic acid, and a second measurement sample may be prepared by mixing samples that contain nucleic acid derived from a second subject group and a sample that contains non-subject-derived nucleic acid. The number of subjects of the first subject group and the number of subjects of the second subject group may be different from each other.

Even when the number of samples that contain nucleic acid derived from a subject group to be used in preparing a measurement sample varies for each measurement sample, the variation of the data amount of sequence information per sample is kept in a predetermined range. Thus, the quality of the sequence information of subject-derived nucleic acid is not influenced.

An amount of the non-subject-derived nucleic acid in the measurement sample may be changed in accordance with the number of samples that contain subject-derived nucleic acid and that have been used in the preparing of the measurement sample (S304b).

The amount of the nucleic acid may be the number of moles of the nucleic acid. The number of moles of nucleic acid can be calculated on the basis of measurement values such as absorbance at 260 nm, an average molecular weight, a molar absorption coefficient of nucleic acid, and the like.

In the preparing of the measurement sample (S304b), an amount of nucleic acid contained in each measurement sample may be the previously determined amount of nucleic acid.

Variation in an amount of nucleic acid per sample included in the measurement sample may be in a range of ±10%.

According to the above configuration, the quality of the analysis result of gene base sequences can be kept in a range allowable for a test result of a gene test such as a panel test.

An amount of the non-subject-derived nucleic acid contained in the measurement sample may be greater than or equal to an amount of nucleic acid per sample contained in the measurement sample.

Accordingly, even when the number of samples that contain subject-derived nucleic acid is not sufficient in the preparing of a measurement sample, the insufficient amount can be compensated for by the non-subject-derived nucleic acid.

In order to solve the above problem, an information processing apparatus (1) according to another aspect of the present invention includes a controller (11) and a storage unit (12). The controller (11) is programmed to: obtain sequence information of nucleic acid contained in a measurement sample prepared by mixing a sample that contains subject-derived nucleic acid and a sample that contains non-subject-derived nucleic acid such that the measurement sample contains a previously determined amount of nucleic acid, and store the sequence information into the storage unit (12); and output sequence information in which a data amount of sequence information per sample that contains subject-derived nucleic acid is a predetermined amount irrespective of the number of samples that contain subject-derived nucleic acid and that have been used in preparing the measurement sample.

According to the above configuration, the information processing apparatus (1) analyzes sequence information in which the data amount of sequence information per sample is the predetermined amount irrespective of the number of samples that contain subject-derived nucleic acid and that have been used in preparing the measurement sample.

Thus, for example, even when the number of samples that contain subject-derived nucleic acid for preparing a measurement sample varies in gene tests, the variation in the data amount of sequence information per sample can be kept in a predetermined range, and an analysis result of constant quality can be outputted.

In order to solve the above problem, a gene analysis system (100) according to another aspect of the present invention includes: a sequencer (2) configured to read sequence information of nucleic acid of a measurement sample prepared by mixing a sample that contains subject-derived nucleic acid and a sample that contains non-subject-derived nucleic acid such that the measurement sample contains a previously determined amount of nucleic acid; and an information processing apparatus (1) configured to obtain the sequence information and output a result of analyzing the sequence information. Irrespective of the number of samples that contain subject-derived nucleic acid and that have been used in preparing the measurement sample, a data amount of sequence information per sample in the sequence information is a predetermined amount.

According to the above configuration, the sequencer (2) performs sequencing on a measurement sample prepared by mixing a sample that contains subject-derived nucleic acid and a sample that contains non-subject-derived nucleic acid such that the measurement sample contains a previously determined amount of nucleic acid. Then, the information processing apparatus (1) analyzes sequence information in which the data amount of sequence information per sample is a predetermined amount irrespective of the number of samples that contain subject-derived nucleic acid and that have been used in preparing the measurement sample.

Thus, for example, even when the number of samples that contain subject-derived nucleic acid for preparing a measurement sample varies in gene tests, the variation in the data amount of sequence information per sample can be kept in a predetermined range, and an analysis result of constant quality can be outputted.

In order to solve the above problem, a program according to another aspect of the present invention causes a computer to perform: obtaining sequence information of nucleic acid in a measurement sample prepared by mixing a sample that contains subject-derived nucleic acid and a sample that contains non-subject-derived nucleic acid such that the measurement sample contains a previously determined amount of nucleic acid, wherein, in the sequence information, a data amount of sequence information per sample accounts for a predetermined proportion in a data amount of sequence information of nucleic acid of the measurement sample (S1); analyzing the sequence information (S109); and outputting an analysis result (S111). In the obtaining of the sequence information of the nucleic acid (S1), a data amount of sequence information per sample that contains subject-derived nucleic acid is a predetermined amount irrespective of the number of samples that contain subject-derived nucleic acid and that have been used in preparing the measurement sample.

According to this configuration, for example, even when the number of samples that contain subject-derived nucleic acid for preparing a measurement sample varies in gene tests, the variation in the data amount of sequence information per sample can be kept in a predetermined range, and an analysis result of constant quality can be outputted.

A non-transitory computer-readable storage medium having the above program stored therein is also included in the scope of the present invention.

BRIEF DESCRIPTION OF THE DRAWINGS

FIG. 1 is a flow chart showing the outline of the flow of a process in an analysis method according to one embodiment of the present invention;

FIG. 2 shows a configuration example of a system that includes an information processing apparatus which creates a report including an analysis result obtained by an analysis method according to one embodiment of the present invention, a sequencer, and an auxiliary apparatus having functions of managing and storing sequence information obtained from the sequencer;

FIG. 3 shows a configuration example of a system that includes the information processing apparatus and the sequencer;

FIG. 4 is a flow chart showing the outline of the flow of a process in which the information processing apparatus shown in FIG. 3 performs analysis on sequence information of subject-derived nucleic acid;

FIG. 5 shows a configuration example of a gene analysis system including the sequencer and the information processing apparatus;

FIG. 6 is a sequence diagram showing an example of major processes performed in the gene analysis system;

FIG. 7 shows one example of a label attached to a container storing a sample;

FIG. 8 shows another example of a label attached to a container storing a sample;

FIG. 9 shows an example of the structure of data stored in a management server;

FIG. 10 shows an example of a configuration of the information processing apparatus;

FIG. 11 is a flow chart showing one example of the flow of a process for receiving an input of information related to a gene panel;

FIG. 12 shows an example of a GUI to be used for inputting information related to a gene panel;

FIG. 13 shows an example of a data structure of a gene-panel-related information database;

FIG. 14 shows another example of a GUI used for inputting information related to a gene panel;

FIG. 15 shows another example of a GUI used for inputting identification information for identifying a disease;

FIG. 16 shows an example of a GUI used when an operator updates information related to a gene panel;

FIG. 17 shows another example of a GUI used when an operator updates information related to a gene panel;

FIG. 18 is a flow chart showing one example of the flow of a process for analyzing gene base sequences of samples;

FIG. 19 is a flow chart describing one example of the procedure of pretreatment for analyzing gene base sequences of samples;

FIG. 20 is a flow chart describing another example of the procedure of pretreatment for analyzing gene base sequences of samples by a sequencer;

FIG. 21 is a flow chart describing another example of the procedure of pretreatment for analyzing gene base sequences of samples by a sequencer;

FIG. 22 is a flow chart describing another example of the procedure of pretreatment for analyzing gene base sequences of samples by a sequencer;

FIG. 23A illustrates one example of a quality control sample;

FIG. 23B illustrates one example of a quality control sample;

FIG. 23C illustrates one example of a quality control sample;

FIG. 23D illustrates one example of a quality control sample;

FIG. 24 shows an example of a data structure of the gene-panel-related information database;

FIG. 25A shows a specific example of a quality control sample;

FIG. 25B shows a specific example of a quality control sample;

FIG. 26A illustrates an example of a step of fragmenting a sample;

FIG. 26B illustrates an example of a step of providing an index sequence and an adapter sequence;

FIG. 27 illustrates one example of a hybridization step;

FIG. 28 illustrates one example of a step of collecting DNA fragments to be analyzed;

FIG. 29 is a flow chart describing one example of the procedure of preparing a measurement sample to be applied to a flow cell;

FIG. 30 shows one example of a measurement sample sheet created at the time of preparation of a measurement sample that is to be subjected to sequencing;

FIG. 31 illustrates a method for preparing a measurement sample by mixing a plurality of libraries of subject-derived samples to be analyzed;

FIG. 32 illustrates one example of a method for preparing a measurement sample when the number of subject-derived samples to be analyzed is insufficient;

FIG. 33 illustrates another example of a method for preparing a measurement sample when the number of subject-derived samples to be analyzed is insufficient;

FIG. 34 illustrates one example of a step of subjecting DNA fragments to a flow cell;

FIG. 35 is a flow chart describing one example of the procedure of analyzing base sequences of sample DNA by a sequencer;

FIG. 36 illustrates one example of a step of amplifying DNA fragments to be analyzed;

FIG. 37 illustrates one example of a sequencing step;

FIG. 38 is a flow chart showing one example of the flow of processes performed by the information processing apparatus when a measurement sample is prepared according to the method shown in FIG. 32;

FIG. 39 is a flow chart showing one example of the flow of processes performed by the information processing apparatus when a measurement sample is prepared according to the method shown in FIG. 33;

FIG. 40 is a flow chart describing one example of the flow of analysis performed by the information processing apparatus;

FIG. 41 shows one example of a file format for sequence information;

FIG. 42A illustrates alignment performed by a data adjustment unit;

FIG. 42B shows one example of a format for a result of alignment performed by the data adjustment unit;

FIG. 43 shows an example of the structure of a reference sequence database;

FIG. 44 shows an example of known mutations (that are not wild-type sequences) to be incorporated into reference sequences included in the reference sequence database;

FIG. 45 is a flow chart describing in detail one example of a step of alignment;

FIG. 46A shows one example of score calculation;

FIG. 46B shows another example of score calculation;

FIG. 47 shows one example of a format for a result file generated by a mutation identification unit;

FIG. 48 shows one example of the structure of a mutation database;

FIG. 49 shows in detail an example of the structure of mutation information in the mutation database;

FIG. 50A is a table indicating correspondence relationship between genes to be analyzed and position information;

FIG. 50B shows a state where mutations that do not correspond to information related to a gene panel are excluded from a result file;

FIG. 51 is a flow chart showing one example of a process in which a drug search unit generates a list of drugs related to mutations;

FIG. 52 shows an example of a data structure of a drug database;

FIG. 53 shows an example of a data structure of the drug database;

FIG. 54 is a flow chart showing one example of a process in which the drug search unit generates a list that includes information regarding drugs related to mutations;

FIG. 55 shows an example of a data structure of the drug database;

FIG. 56 is a flow chart showing one example of a process in which the drug search unit generates a list that includes information regarding clinical trials of drugs;

FIG. 57 shows one example of a report to be created;

FIG. 58 shows one example of a quality evaluation index;

FIG. 59 is a flow chart showing one example of the flow of a gene test;

FIG. 60 shows one example of steps performed for simultaneously analyzing gene base sequences derived from a plurality of subjects; and

FIG. 61 is a schematic diagram showing a state where DNA fragments applied to a flow cell have been captured by capture molecules.

DETAILED DESCRIPTION OF THE PREFERRED EMBODIMENTS

[Embodiment 1]

An analysis method according to one embodiment of the present disclosure is a method for outputting an analysis result of constant quality in gene tests. When this analysis method is applied, even if the number of samples subjected to one sequencing run is smaller than a recommended number of samples, it is possible to prevent the data amount of sequence information per sample from significantly varying and exceeding a predetermined range, thereby being able to output an analysis result of constant quality.

(Flow of gene test)

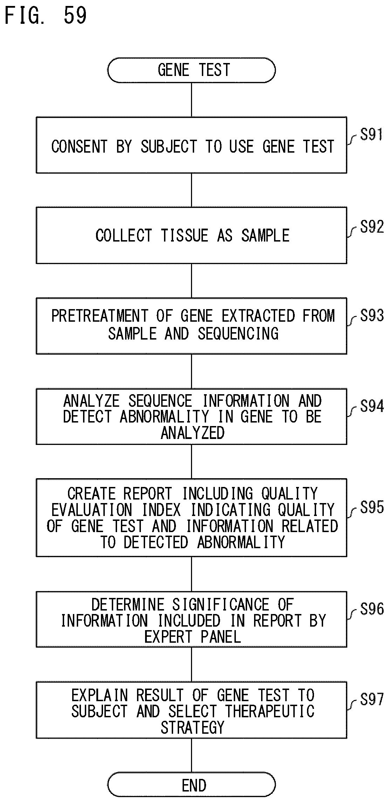

First, how a gene test is performed is described with reference to FIG. 59. FIG. 59 is a flow chart showing one example of the flow of a gene test.

In a case where it would be advantageous for a subject to have a gene test in order to determine a diagnosis and a therapeutic strategy for the subject, the attending physician of the subject explains this to the subject, and obtains consent of the subject to use a gene test (step S91). When the subject consents, a tissue and blood of the subject, which are each used as a sample in the gene test, are collected (step S92). Each collected sample is stored in a predetermined container.

Next, pretreatment of genes extracted from the sample, and sequencing are performed (step S93). Then, sequence information obtained as a result of the sequencing is analyzed, any abnormality in each gene to be analyzed is detected (step S94), and a report that includes a quality evaluation index indicating the quality of the gene test and information related to the detected abnormality is created (step S95).

Then, the significance of the information included in the report is determined by an expert panel composed of a plurality of specialists of gene tests (step S96). The attending physician of the subject explains to the subject the result of the gene test on the basis of the report, and selects a therapeutic strategy after having a discussion with the subject (step S97).

(Outline of analysis method)

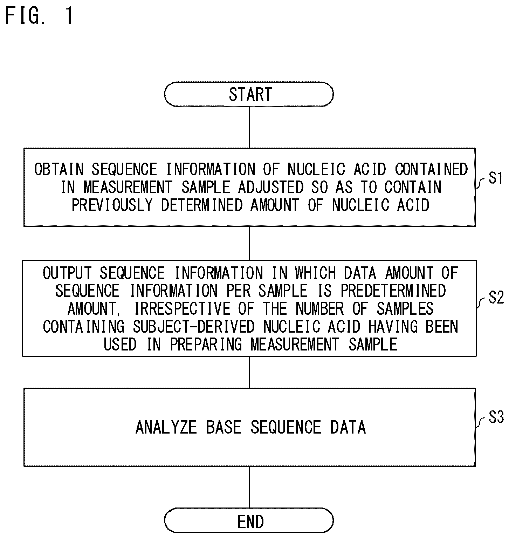

The outline of the analysis method according to one embodiment of the present disclosure is described with reference to FIG. 1. FIG. 1 is a flow chart showing the flow of processes in the analysis method according to one embodiment of the present disclosure.

Step S1 is a step of obtaining sequence information of nucleic acid contained in a measurement sample prepared so as to contain a previously determined amount of nucleic acid. The measurement sample can be prepared by use of a sample that contains subject-derived nucleic acid. The sample containing subject-derived nucleic acid is obtained by extracting nucleic acid such as DNA and RNA from blood, a tissue, and the like collected from a subject (for example, a patient) by use of a known method, for example. Sequencing includes processes of reading the base sequence of fragments (DNA fragments in a case where DNA is to be analyzed) of one or a plurality of genes to be analyzed which have been collected in the pretreatment, and generating sequence information.

The measurement sample means a sample prepared so as to be subjected to sequencing performed by a sequencer 2. When the amount of nucleic acid of the subject-derived samples is less than a previously determined amount of nucleic acid, the measurement sample is prepared so as to contain the previously determined amount of nucleic acid, by mixing a non-subject-derived sample thereto.

Here, the "non-subject-derived nucleic acid" means nucleic acid or the like derived from a virus, a microorganism, a plant, or an insect, for example. As the "non-subject-derived nucleic acid", "PhiX DNA" or the like that is provided by Illumina, Inc. can be suitably used, for example. PhiX DNA is bacteriophage-derived nucleic acid. PhiX DNA has a small molecular weight and is highly diverse in sequence.

The "previously determined amount of nucleic acid" means the amount of nucleic acid determined on the basis of a protocol recommended for the sequencer 2 to be used and reagents to be used. That is, the "previously determined amount of nucleic acid" is the amount of nucleic acid realized when the number of samples is not smaller than the recommended number of samples to be subjected to one sequencing run. The "previously determined amount of nucleic acid" is to ensure that the quality of the analysis result of base sequences obtained as a result of sequencing is at a certain level or higher. The "previously determined amount of nucleic acid" may be an amount specified between an upper limit amount and a lower limit amount.

The sequence information is sequence information of nucleic acid captured by capture molecules provided on the surface of the flow path in a flow cell, for example. Except for an operation of applying the measurement sample to a predetermined flow cell, and an operation of setting the flow cell on the sequencer 2, obtainment of sequence information is performed by the sequencer 2. In a certain case, capture molecules for capturing nucleic acid are immobilized on the surface of a solid phase in a predetermined flow cell or the like recommended to be used in the sequencer 2. The capture molecules include base sequences that are complementary to at least a part of nucleic acid contained in the measurement sample.

Some of the operations included in step S1 above (for example, the operation of applying the measurement sample to the flow cell, and the operation of setting the flow cell on the sequencer 2) are performed by an operator of the sequencer 2 or a person in charge of the test. However, some of the operations in step S1 may be performed by one or a plurality of working robots as described below.

- (Working robot 1) When samples containing subject-derived nucleic acid that are to be used in preparation of a measurement sample, and a sample that contains non-subject-derived nucleic acid are set, and the molar concentration of nucleic acid contained in each sample is inputted, a working robot 1 automatically dispenses each sample to prepare a measurement sample so as to contain a previously determined amount of nucleic acid.
- (Working robot 2) A working robot 2 applies the prepared measurement sample to the flow cell.
- (Working robot 3) A working robot 3 transports and sets the flow cell to a predetermined position in the sequencer 2.

Step S2 is a step of outputting sequence information in which the data amount of sequence information per sample is a predetermined amount, irrespective of the number of samples containing subject-derived nucleic acid that have been used in preparing the measurement sample. Here, the "predetermined amount" may be an amount specified between an upper limit amount and a lower limit amount. The predetermined data amount is a data amount that accounts for a predetermined proportion in the data amount of sequence information obtained in step S1. This step is a part of sequencing and is performed by the sequencer 2.

For example, if the number, of samples containing subject-derived nucleic acid, recommended for preparation of a measurement sample is 3, the data amount of sequence information per sample accounts for about 1/3 (i.e., about 33%) in the data amount of the obtained sequence information. If the number, of samples containing subject-derived nucleic acid, recommended for preparation of a measurement sample is 8, the data amount of sequence information per sample is about 1/8 (i.e., about 12.5%) in the data amount of the obtained sequence information. Thus, the predetermined proportion is a value that changes according to the number, of samples containing subject-derived nucleic acid, recommended for preparation of a measurement sample.

When the number of samples containing subject-derived nucleic acid that have been used in preparation of the measurement sample has been changed, the variation in the data amount of sequence information per sample is preferably in a range of ±10%. In this case, the data amount of sequence information of non-subject-derived nucleic acid in the obtained sequence information may be at least an amount or more that corresponds to the data amount of sequence information of nucleic acid per sample contained in the sequence information.

Step S3 is a step of analyzing sequence information and outputting an analysis result. This step is performed by an information processing apparatus 1. The information processing apparatus 1 is a computer that performs analysis on sequence information, to be analyzed, which has been generated and outputted by the sequencer 2 on the basis of the base sequence data having been read. The base sequence data means polynucleotide sequence data obtained by sequencing, and is base sequence data outputted by the sequencer 2.

In order to keep the quality of the analysis result of gene base sequences constant, the quality of sequence information needs to be appropriately evaluated. However, if the data amount of sequence information obtained per sample varies, it also becomes necessary to vary, in accordance with the variation of the data amount, the indexes for evaluating the quality of sequence information.

For example, one of the indexes for evaluating the quality of sequence information is depth. The depth is a quality evaluation index based on the total number of pieces of sequence information obtained by reading each base contained in each gene to be analyzed. In general, a reference value for depth is finely set in advance for a case where sequencing is performed using an ideal number of samples, and the quality of sequence information is evaluated according to whether or not the depth is not less than a given reference value. In order to keep the quality of the analysis result of gene base sequences constant, the reference value for depth in an existing analysis program to be used in analysis of gene base sequences needs be changed in accordance with variation of the number of samples to be subjected to one sequencing run.

If steps S1 to S3 shown in FIG. 1 are employed, even when the number of samples subjected to one sequencing run in a gene test is smaller than the recommended number of samples, the variation in the data amount of sequence information per sample can be kept in a predetermined range and an analysis result of constant quality can be outputted.

In addition, if the data amount of sequence information obtained per sample varies, it becomes necessary to vary, in accordance with the variation, the criteria for detecting abnormalities in genes to be analyzed.

For example, gene abnormalities to be detected in a panel test include polymorphisms such as single nucleotide polymorphism (SNP) and copy number variation (CNV). In order to prevent occurrence of variation in the accuracy of detecting polymorphisms due to variation of the data amount of sequence information obtained per sample, it is necessary to set criteria for detecting polymorphisms in the existing analysis program to be used in analysis of gene base sequences, in accordance with the number of samples to be subjected to one sequencing run.

If steps S1 to S3 shown in FIG. 1 are employed, even when the number of samples to be subjected to one sequencing run varies, the data amount of sequence information obtained per sample is constant. Therefore, the existing analysis program can be used as it is.

(System configuration example)

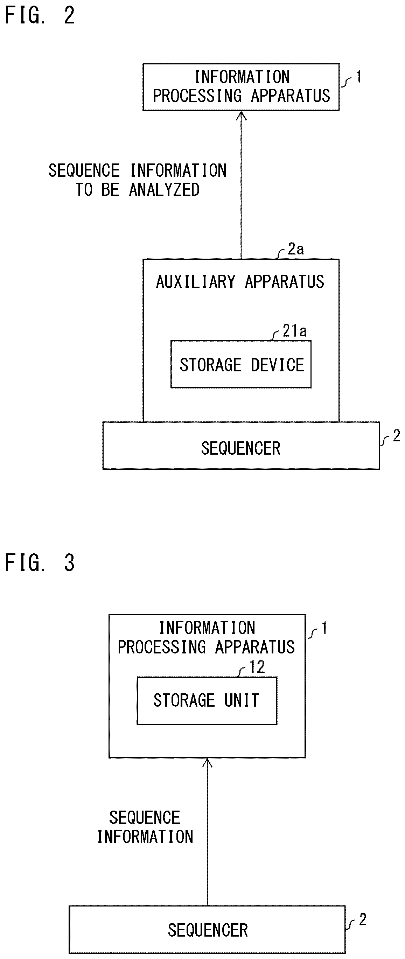

FIG. 2 shows a configuration example of a system that includes: the information processing apparatus 1 which creates a report including an analysis result obtained by an analysis method according to one embodiment of the present disclosure; the sequencer 2; and an auxiliary apparatus 2a having functions of managing and storing sequence information obtained from the sequencer 2. The sequence information generated by the sequencer 2 is stored in a storage device 21a of the auxiliary apparatus 2a connected to the sequencer 2, for example. The information processing apparatus 1 may be configured to obtain sequence information to be analyzed from the storage device 21a. The information processing apparatus 1 analyzes the sequence information generated by the sequencer 2 and analyzes the presence or absence of gene abnormalities. The auxiliary apparatus 2a may sort sequence information obtained from the sequencer 2 for each subject or each sample, and store the sorted sequence information into the storage device 21a. The information processing apparatus 1 may receive sequence information to be analyzed from the auxiliary apparatus 2a via a communication line.

The information processing apparatus 1 may be configured to have the functions of the auxiliary apparatus 2a shown in FIG. 2. In this case, as shown in FIG. 3, the system may be configured to include the information processing apparatus 1 and the sequencer 2. Sequence information generated by the sequencer 2 is stored in a storage unit 12 of the information processing apparatus 1. The information processing apparatus 1 may obtain the sequence information generated by the sequencer 2, sort the sequence information for each subject or each sample, and store the sorted sequence information into the storage unit 12.

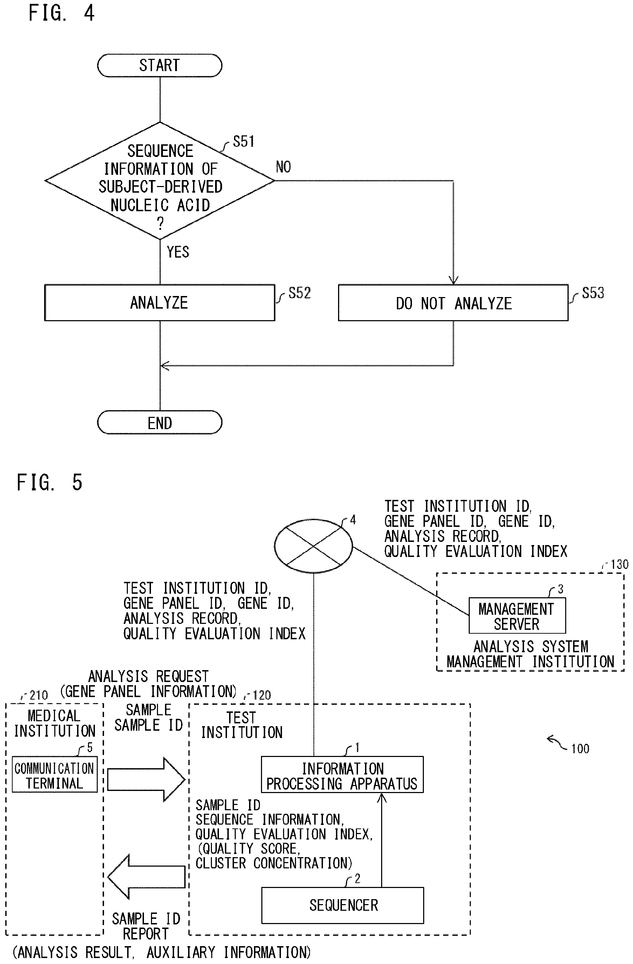

The flow of the process in which the information processing apparatus 1 having the functions of the auxiliary apparatus 2a performs analysis on sequence information of subject-derived nucleic acid is described with reference to FIG. 4. FIG. 4 is a flow chart showing the outline of the flow of the process in which the information processing apparatus 1 shown in FIG. 3 performs analysis on sequence information of subject-derived nucleic acid. The information processing apparatus 1 performs analysis on only sequence information of subject-derived nucleic acid in the sequence information obtained from the sequencer 2. That is, when the sequence information is sequence information of subject-derived nucleic acid (YES in step S51), the information processing apparatus 1 performs analysis (step S52), and when the sequence information is sequence information of non-subject-derived nucleic acid (NO in step S51), the information processing apparatus 1 does not perform at least a part of the analysis to be performed on the sequence information of subject-derived nucleic acid (step S53).

In order to allow determination whether or not the sequence information is sequence information of subject-derived nucleic acid, the sequence information of subject-derived nucleic acid in the sequence information obtained in step S1 of FIG. 1 includes an index sequence. Therefore, it is possible to selectively extract the sequence information of subject-derived nucleic acid from the sequence information obtained from the sequencer 2, to perform analysis.

In the following, one embodiment of the present disclosure is described in detail.

(Application example of gene analysis system 100)

First, the outline of the gene analysis system 100 including the information processing apparatus 1 according to one embodiment of the present disclosure is described with reference to FIG. 5. FIG. 5 shows a configuration example of the gene analysis system 100 including the sequencer 2 and the information processing apparatus 1. The gene analysis system 100 is a system for analyzing gene sequence information, and includes the information processing apparatus 1, the sequencer 2, and a management server 3.

The gene analysis system 100 shown in FIG. 5 is applied in an analysis system management institution 130 which manages general analyses performed in a test institution 120. The gene analysis system 100 is also applied in the test institution 120 which analyzes a provided sample in response to an analysis request from a medical institution 210 and which provides an analysis result to the medical institution 210.

The test institution 120 tests/analyzes the sample provided from the medical institution 210, creates a report based on the analysis result, and provides the report to the medical institution 210. In the example shown in FIG. 5, the test institution 120 is provided with, but not limited to, the sequencer 2, the information processing apparatus 1, and the like. For example, the test institution 120 may include a facility in which the information processing apparatus 1 is installed, and another facility in which the sequencer 2 is installed.

The analysis system management institution 130 manages general analyses that are performed in each test institution 120 that uses the gene analysis system 100. The analysis system management institution 130 may be the same institution as the test institution 120.

The medical institution 210 is an institution in which doctors, nurses, pharmacists, and the like perform medical activities such as providing diagnosis, therapy, and dispensation to patients, and examples of the medical institution 210 include hospitals, clinics, and pharmacies.

Although FIG. 5 shows an example case where the test institution 120 and the medical institution 210 are separate institutions, the gene analysis system 100 can be suitably used in an integrated facility of the test institution 120 and the medical institution 210, such as a university hospital.

(Process in application example of gene analysis system 100)

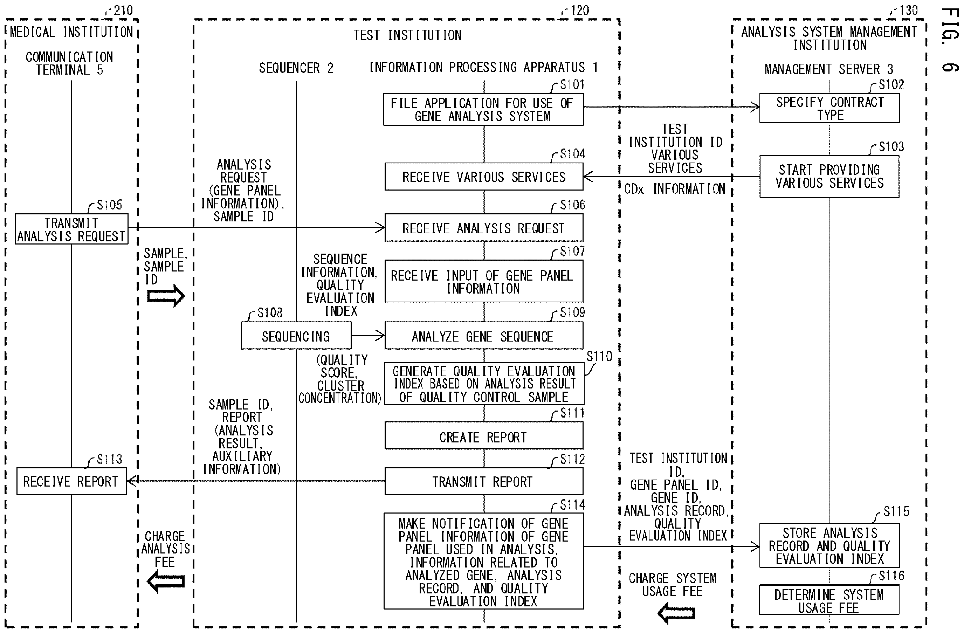

Next, the flow of processes performed in an application example of the gene analysis system 100 shown in FIG. 5 is more specifically described with reference to FIG. 6. FIG. 6 is a sequence diagram showing an example of major processes performed in the gene analysis system 100. The processes shown in FIG. 6 are only part of processes performed in each institution.

<Filing application for use of gene analysis system and start of use>

First, a test institution 120 that is going to use the gene analysis system 100 introduces the information processing apparatus 1. Then, the test institution 120 files an application for use of the gene analysis system 100 to the analysis system management institution 130 (step S101). S101 can be omitted. For example, in a case where the analysis system management institution 130 is identical to the test institution 120, S101 is omitted.

The test institution 120 and the analysis system management institution 130 can conclude in advance a desired contract with regard to use of the gene analysis system 100, from among a plurality of contract types. For example, service contents provided from the analysis system management institution 130 to the test institution 120, a method of determination of a system usage fee charged to the test institution 120 by the analysis system management institution 130, a method of payment for a system usage fee, and the like may be selected from a plurality of different contract types. The management server 3 of the analysis system management institution 130 specifies the content of the contract concluded with the test institution 120, in response to the application filed from the test institution 120 (step S102). S102 can be omitted. For example, in a case where the analysis system management institution 130 is identical to the test institution 120, S102 is omitted.

Next, the management server 3, managed by the analysis system management institution 130, provides a test institution ID to the information processing apparatus 1 of the test institution 120 having concluded the contract, and starts providing various services (step S103). S103 can be omitted. For example, in a case where the analysis system management institution 130 is identical to the test institution 120, S103 is omitted. In a case where the analysis system management institution 130 is identical to the test institution 120, the test institution ID and various services are managed by the test institution 120 itself.

The information processing apparatus 1 receives information, programs, and the like for controlling the analysis process of gene base sequences, creation of a report based on the analysis result, and the like, from the management server 3. Accordingly, the test institution 120 becomes able to receive various services from the analysis system management institution 130. The information processing apparatus 1 can output an analysis result, a report, and the like based on the inputted information related to a gene panel (hereinafter, also referred to as gene panel information). In a case where the analysis system management institution 130 is identical to the test institution 120, the test institution 120 itself manages information, programs, and the like for controlling the analysis process of gene base sequences, creation of a report based on the analysis result, and the like.

In many cases, a gene panel includes a set of reagents such as a primer and a probe. The gene panel may be used for analyzing polymorphisms, such as mutation, single nucleotide polymorphism (SNP), and copy number variation (copy number abnormality) (CNV), that have occurred in genes. The gene panel may be used for outputting information regarding the amount of mutations in the entirety of genes to be analyzed (also referred to as Tumor Mutation Burden, or the like), and for calculation of the methylation frequency.

Herein, a "gene panel" means a gene panel that allows batch analysis of a plurality of abnormalities in a plurality of genes, and that allows a test of samples related to a plurality of diseases. Such a gene panel is also referred to as a "multi-panel" or a "large panel", and is used for analyzing genes that are related to a plurality of diseases. In such a gene panel, base sequences read from exon regions each having a base length of 10 Mb (10 million bases) or greater are to be analyzed.

<Analysis request to test institution 120>

In the medical institution 210, a doctor or the like collects a sample such as blood and a tissue of a lesion site of a subject as necessary. When analysis of the collected sample is requested to the test institution 120, an analysis request is transmitted from a communication terminal 5 provided in the medical institution 210, for example (step S105). When requesting analysis of a sample to the test institution 120, the medical institution 210 transmits an analysis request and provides the test institution 120 with a sample ID provided for each sample. The sample ID provided for each sample associates the sample with, for example, information regarding the subject from whom the sample has been collected (for example, patient ID), and identification information for identifying the disease of the subject (for example, disease name and disease ID). A subject ID, a disease ID, and the like may be transmitted, together with the sample ID, from the medical institution 210 to the test institution 120. In the test institution 120, the sample ID and the subject ID are associated with the disease ID, to be managed.

In the following, an example case in which the medical institution 210 requests a panel test analysis to the test institution 120 is described. The panel test is not limited to laboratory tests, but includes tests for research use.

Herein, a "subject" means a human subject. However, the concept of the present disclosure can be applied to a genome derived from an organism such as any animal other than a human, and is useful also in the fields such as medical care, veterinary medicine, and zoological science.

When a gene panel test is requested from the medical institution 210, a desired gene panel may be designated. Therefore, gene panel information can be included in the analysis request transmitted from the medical institution 210 in step S105 shown in FIG. 6. Here, the gene panel information may be any information that can be used for specifying a gene panel, and may be, for example, the gene panel name, the names of genes to be analyzed in the panel test, and the like.

<Analysis in test institution 120>

The information processing apparatus 1 receives the analysis request from the medical institution 210 (S106). Further, the information processing apparatus 1 receives a sample from the medical institution 210, which is the transmission source of the analysis request. In the medical institution 210 (and the test institution 120), the subject name, the subject ID, the disease name, the disease ID, the sample ID, and the like are recorded/managed in association with one another.

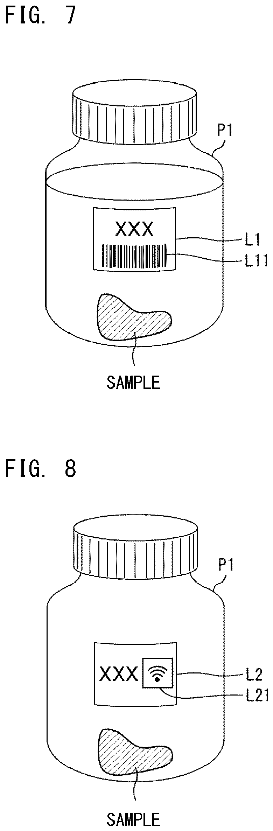

Each sample provided from the medical institution 210 is stored in a container as shown in FIG. 7 and FIG. 8. FIG. 7 shows one example of a label L1 attached to a container P1 storing a sample. A label L1 indicating a subject ID, a sample ID, and the like is attached to each container P1 storing a sample. A recording means such as a bar code L11 is indicated on the label L1. By reading the recording means such as the bar code L11 indicated on the label L1, it is possible to obtain the subject ID of the subject, the sample ID, the disease ID which is identification information of the disease, and the like. Reading of the bar code L11 may be performed by a person in charge of the test, or may be automatically performed by the information processing apparatus 1.

Alternatively, as shown in FIG. 8, a label L2 indicating a subject ID, a sample ID, and the like may be attached to each container P1, and a recording means such as an RFID tag L21 may be attached to the label L2. By reading the recording means such as the RFID tag L21 attached to the label L2, it is possible to obtain the subject ID of the subject, the sample ID, and the disease ID which is identification information of the disease of the subject. In FIG. 7 and FIG. 8, examples of a container that stores a tissue collected from a subject are shown. The container that stores blood collected from a subject has the same configuration as described above.

There are a plurality of gene panels that can be used in analysis that the test institution 120 is requested to perform by the medical institution 210, and a gene group to be analyzed is fixed for each gene panel. The test institution 120 can selectively use a plurality of gene panels so as to suit the purpose of the analysis. That is, for a first sample provided from the medical institution 210, a first gene panel can be used in order to analyze a first gene group to be analyzed, and for a second sample, a second gene panel can be used in order to analyze a second gene group to be analyzed.

The information processing apparatus 1 receives, from an operator, an input of gene panel information of a gene panel to be used in order to analyze the sample (step S107).

In the test institution 120, the received sample is subjected to pretreatment using the gene panel, and sequencing is performed by use of the sequencer 2 (step S108).

In addition, in the test institution 120, separately from sequencing performed on subject-derived samples, a predetermined quality control sample corresponding to the gene panel is subjected to pretreatment using the gene panel, and sequencing is performed by use of the sequencer 2 (step S108), whereby accuracy control is performed.

The result obtained by subjecting the quality control sample to a gene test including pretreatment, sequencing, sequence analysis, and the like is used as a quality evaluation index of the panel test.

Each gene panel may be associated with one or a plurality of quality control samples. Alternatively, for example, for each gene panel, a corresponding quality control sample may be prepared in advance. Further, a quality control sample may be measured individually, or may be measured together with a sample provided from the medical institution 210.

The pretreatment is a series of processes for preparing a measurement sample. The pretreatment corresponds to steps S1 to S2 in FIG. 1. The pretreatment includes processes of fragmenting genes such as DNA contained in each sample and collecting the fragmented genes. The sequencing corresponds to step S3 in FIG. 1. The sequencing includes a process of reading base sequences of fragments of one or a plurality of DNAs to be analyzed that have been collected in the pretreatment. In the sequence information including base sequence data read in the sequencing performed by the sequencer 2, sequence information to be analyzed is inputted to the information processing apparatus 1. The pretreatment includes processes of fragmenting genes such as DNA contained in each subject-derived sample and the non-subject-derived sample, and collecting the fragmented genes.

The sequencer 2 may output, to the information processing apparatus, sequence information including a quality score which is a quality evaluation index for the step of reading gene base sequences. The sequencer 2 may output, to the information processing apparatus 1, a cluster concentration which is a quality evaluation index for a step of amplifying DNA fragments to be analyzed. The "quality score" and the "cluster concentration" are described later.

The information processing apparatus 1 obtains sequence information from the sequencer 2 and analyzes gene base sequences (step S109).

The quality control sample is also processed in the same steps as those performed in the panel test on the sample provided from the medical institution 210. Thus, gene sequence information of the quality control sample is also analyzed in the same manner as that of the sample provided from the medical institution 210. On the basis of the result of analyzing the quality control sample, a quality evaluation index for evaluating the quality of the panel test is generated.

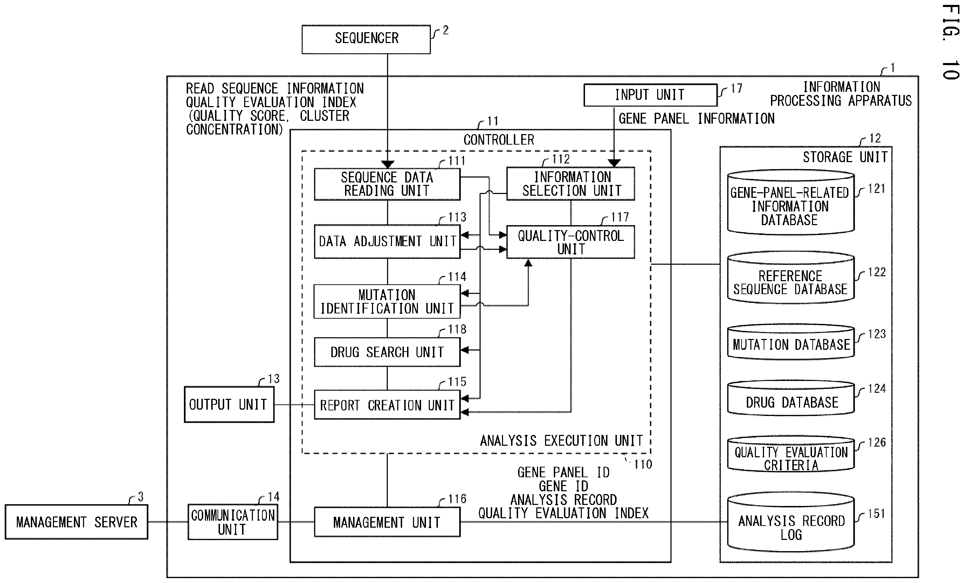

Next, the information processing apparatus 1 evaluates the quality of the panel test on the basis of the quality evaluation index generated by a quality-control unit 117 (step S110). Specifically, the information processing apparatus 1 can evaluate the quality of each panel test on the basis of a result of comparison between the generated quality evaluation index and an evaluation criterion set for each quality evaluation index stored in quality evaluation criteria 126 shown in FIG. 10.

The quality control sample is a sample that contains non-subject-derived nucleic acid. The information processing apparatus 1 may perform a process for obtaining information related to the quality, on at least a part of sequence information of the sample that contains non-subject-derived nucleic acid, in the sequence information of nucleic acid of the measurement sample. In this case, at least a part of sequence information of the sample that contains non-subject-derived nucleic acid is used as a substitute for sequence information of the quality control sample.

The information processing apparatus 1 creates a report on the basis of the analysis result obtained in step S109, and the index generated on the basis of the result of analyzing the quality control sample (step S111), and transmits the created report to the communication terminal 5 (step S112). For example, the report may include: data of an alignment result of the sequence information; data itself of the result of analysis by the information processing apparatus 1, such as data regarding identified gene mutations or the like; and information regarding the quality of the panel test.

The created report may be printed in the test institution 120. For example, the test institution 120 may send the created report in the form of a paper medium to the medical institution 210.

The information processing apparatus 1 of the test institution 120 that uses the gene analysis system 100 notifies the management server 3 of the gene panel information of the gene panel having been used in the analysis, information regarding the analyzed genes, an analysis record, the quality evaluation index generated for the gene test having been performed, and the like (step S114). S114 can be omitted. For example, in a case where the analysis system management institution 130 is identical to the test institution 120, S114 is omitted. In this case, the test institution 120 itself manages the analysis record, the quality evaluation index, and the like.

The management server 3 obtains a test institution ID, a gene panel ID, a gene ID, an analysis record, and the like, via, for example, a communication line 4 from the information processing apparatus 1 of each test institution 120 that uses the gene analysis system 100. The management server 3 stores the obtained test institution ID, gene panel ID, gene ID, analysis record, quality evaluation index, and the like so as to be associated with one another (step S115). S115 can be omitted. For example, in a case where the analysis system management institution 130 is identical to the test institution 120, S115 is omitted. In this case, the test institution 120 itself manages the analysis record, the quality evaluation index, and the like.

The test institution ID is information for specifying the test institution 120 that performs gene sequence analysis. The test institution ID may be an operator ID which is identification information provided to each operator who belongs to the test institution 120 that uses the information processing apparatus 1.

The gene panel ID is identification information provided for specifying a gene panel to be used in analysis of genes to be analyzed. The gene panel ID provided to the gene panel is associated with a gene panel name, the name of the company that provides the gene panel, and the like.

The gene ID is identification information provided to each gene for specifying a gene to be analyzed.

The analysis record is information regarding the analysis state of gene sequence information. For example, the analysis record may be the number of times of sequence analysis the analysis using a predetermined gene panel has been performed in the information processing apparatus 1, may be the number of genes that have been analyzed, or may be an accumulated total of the number of gene mutations that have been identified. Alternatively, the analysis record may be information regarding the amount of data that has been processed in the analysis.

The management server 3 aggregates, for each test institution 120, the analysis records in a predetermined period (for example, any period such as a day, week, month, or year) and determines a system usage fee in accordance with the aggregation result and the contract type (step S116). The analysis system management institution 130 may charge the determined system usage fee to the test institution 120, and request payment of the system usage fee to the analysis system management institution 130. S116 can be omitted. For example, in a case where the analysis system management institution 130 is identical to the test institution 120, S116 is omitted.

(Configuration example of gene analysis system 100)

The gene analysis system 100 is a system for analyzing gene sequence information, and includes at least the information processing apparatus 1 and the management server 3. The information processing apparatus 1 is connected to the management server 3 via the communication line 4 such as an intranet and the Internet.

(Sequencer 2)

The sequencer 2 is a base sequence analyzing apparatus that is used in order to read the base sequences of genes contained in a sample.

The sequencer 2 according to the present embodiment is preferably a next generation sequencer that performs sequencing using a next generation sequencing technology, or a third-generation sequencer. The next generation sequencer denotes one of base sequence analyzing apparatuses which have been developed in recent years. The next generation sequencer has a significantly improved analytical capability realized by performing, in a flow cell, parallel processing of a large amount of a single DNA molecule or a DNA template that has been clonally amplified.

Sequencing technology usable in the present embodiment can be a sequencing technology that obtains a plurality of reads by reading the same region multiple times (deep sequencing).

Examples of the sequencing technology usable in the present embodiment include sequencing technologies that can obtain a large number of reads per sequencing run, such as ionic semiconductor sequencing, pyrosequencing, sequencing-by-synthesis using a reversible dye terminator, sequencing-by-ligation, and sequencing that uses probe ligation of oligonucleotides. The present disclosure may be applied to whole genome sequencing which does not analyze the base sequences of a specific region but analyzes the base sequences of the entire genome. The whole genome sequencing can be applied to a gene panel to be used for analyzing genes related to a plurality of diseases. The whole genome sequencing can read base sequences from exon regions each having a base length of 10 Mb (10 million bases) or greater.

The sequence primer to be used in sequencing is not limited in particular, and is set as appropriate on the basis of a sequence that is suitable for amplifying a target region. Reagents to be used in sequencing may also be suitably selected in accordance with the sequencing technology and the sequencer 2 to be used. The procedure from the pretreatment to the sequencing is described later by using a specific example.

(Management server 3)

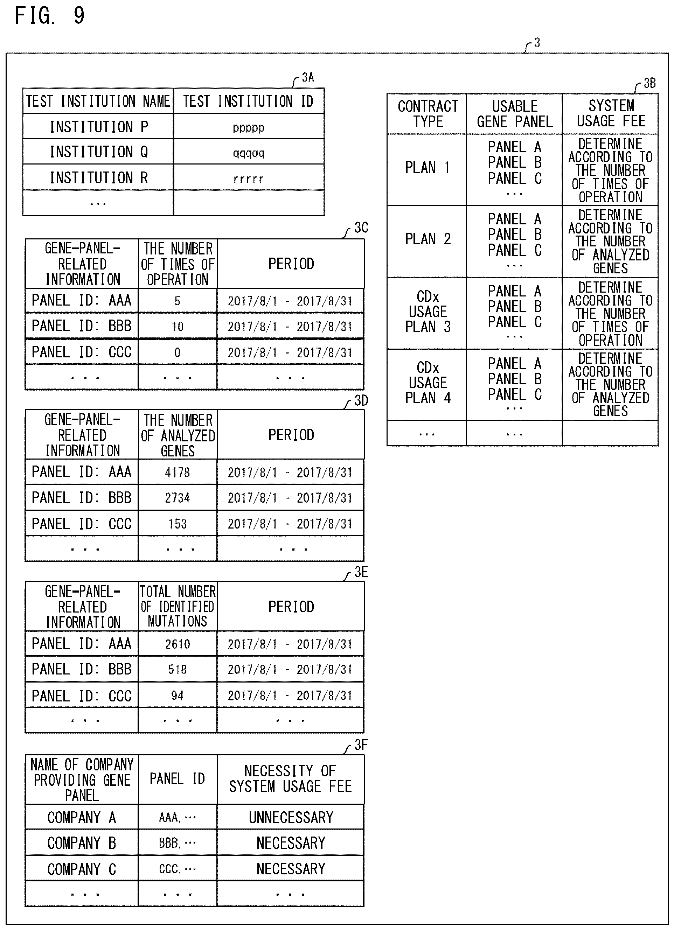

Next, data stored in the management server 3 is described with reference to FIG. 9. FIG. 9 shows an example of the structure of data stored in the management server 3. On the basis of each piece of data shown in FIG. 9, the analysis system management institution 130 determines a system usage fee to be charged to each test institution. The management server 3 receives, from the information processing apparatus 1 via the communication line 4, information that includes: information for specifying a test institution 120 that performs gene sequence analysis (for example, test institution ID); gene panel information of the gene panel that has been used; and information regarding the state of gene sequence analysis (for example, analysis record). In FIG. 9, "gene panel A" is indicated as "Panel A", "gene panel B" is indicated as "Panel B", and so on. "Gene panel ID" is indicated as "Panel ID".

In data 3A, the name of a test institution that uses the gene analysis system 100, and a test institution ID provided to the test institution are associated with each other. In data 3B, the type of contract concluded between the analysis system management institution 130 and a test institution 120, services to be provided to the test institution that has concluded the contract (for example, usable gene panel), and a system usage fee are associated with one another.

For example, in a case where a test institution "Institution P" has concluded a contract of "Plan 1" with the analysis system management institution 130, the analysis system management institution 130 charges the test institution P for a usage fee according to the number of times of operation. "The number of times of operation" is the number of times a panel test has been performed by the information processing apparatus 1, for example.

When the test institution P starts using the gene analysis system 100, the test institution P logs in the gene analysis system 100 by using the test institution ID and a password of the test institution P. On the basis of the test institution ID inputted at the time of log in, the management server 3 can specify the test institution name, the contract type, and the like.

"Plan 3" is a higher-order plan of "Plan 1". "Plan 3" is obtained by adding provision of auxiliary information usable for "CDx usage", to "Plan 1". Therefore, the cost for concluding a contract of "Plan 3" may be higher than the cost for concluding a contract of "Plan 1".

CDx information necessary for creating a report that includes auxiliary information related to the efficacy of drugs applicable to companion diagnostics (CDx) is provided to the test institution that has concluded the contract of "Plan 3" (see S104 in FIG. 6). For example, in a case where a test institution "Institution Q" has concluded the contract of "Plan 3" with the analysis system management institution 130, the management server 3 specifies the test institution name, the contract type, and the like on the basis of the test institution ID inputted at the time when the test institution Q has logged in the gene analysis system 100, and provides the test institution Q with auxiliary information related to the efficacy of drugs applicable to CDx. Thus, the test institution Q can provide the medical institution 210 with a report that includes auxiliary information related to the efficacy of drugs applicable to CDx.

Data 3C to 3E are analysis records regarding the number of times of operation that was performed, genes that were analyzed, and the total number of gene mutations that were identified, by the test institution using the gene analysis system 100 in a period from August 1, 2017 to August 31, 2017. These analysis records are transmitted from the information processing apparatus 1 to the management server 3, and are stored in the management server 3. On the basis of the data of these analysis records, the analysis system management institution 130 determines a system usage fee to be charged to each test institution. The record aggregation period is not limited to that mentioned above. The records may be aggregated in any period such as a day, week, month, or year.

When the analysis system management institution 130 determines a system usage fee, the system usage fee may be changed depending on whether the gene panel that was used in the test was from a company that provides (for example, produces or sells) the gene panel. In this case, it is sufficient that data 3F is stored in the management server 3. In data 3F, the name of a company that provides gene panels, such as "Company A" or "Company B", a gene panel ID, and an agreement regarding the system usage fee (for example, whether a system usage fee is required or not) are associated with one another.

An example in which "Institution P" concluded a contract of "Plan 1" with the analysis system management institution 130 and the analysis records are those shown in FIG. 9 is described. Institution P performed tests using a gene panel (gene panel ID "AAA") provided by Company A, five times, and tests using a gene panel (gene panel ID "BBB") provided by Company B, ten times. According to data 3F shown in FIG. 9, for the five tests using the gene panel provided by Company A, the system usage fee is not required. Therefore, for Institution P, the analysis system management institution 130 determines a system usage fee, excluding the number of times of test using the gene panel provided by Company A.

(Configuration example of information processing apparatus 1)

FIG. 10 is one example of the configuration of the information processing apparatus 1.

The information processing apparatus 1 includes: a controller 11 which obtains sequence information, to be analyzed, including base sequence data read by the sequencer 2 and gene panel information including a plurality of genes to be analyzed; and an output unit 13 which outputs a result of analysis, of the sequence information, based on the gene panel information obtained by the controller 11. The information processing apparatus 1 can be configured by use of a computer. For example, the controller 11 is implemented by a processor such as a CPU (central processing unit), and the storage unit 12 is implemented by a hard disk drive.

In the storage unit 12, a program for sequence analysis, a program for generating a single reference sequence, and the like are also stored. The output unit 13 includes a display, a printer, a speaker, and the like. An input unit 17 includes a keyboard, a mouse, a touch sensor, and the like. A device may be used that has both of the functions of an input unit and an output unit, such as a touch panel in which a touch sensor and a display are integrated. A communication unit 14 is an interface that allows the controller 11 to communicate with an external apparatus.

The information processing apparatus 1 includes: the controller 11 which comprehensively controls the components of the information processing apparatus 1; the storage unit 12 which stores various kinds of data to be used by an analysis execution unit 110; the output unit 13; the communication unit 14; and the input unit 17. The controller 11 includes the analysis execution unit 110 and a management unit 116. Further, the analysis execution unit 110 includes a sequence data reading unit 111, an information selection unit 112, a data adjustment unit 113, a mutation identification unit 114, the quality-control unit 117, a drug search unit 118, and a report creation unit 115. The storage unit 12 stores a gene-panel-related information database 121, a reference sequence database 122, a mutation database 123, a drug database 124, and an analysis record log 151.

The information processing apparatus 1 creates a report that includes an analysis result corresponding to the gene panel having been used, even when a different gene panel is used for each analysis. The operator who uses the gene analysis system 100 can analyze the result of the panel test by a common analysis program irrespective of the type of the gene panel, and can create a report. Accordingly, when a panel test is performed, a bothersome operation, such as selecting an analysis program to be used for each gene panel and performing specific setting for the analysis program for each gene panel to be used, is omitted. Thus, convenience for the operator is improved.

When the operator of the information processing apparatus 1 has inputted gene panel information through the input unit 17, the information selection unit 112 refers to the gene-panel-related information database 121, and controls the algorithm of the analysis program such that the analysis program performs analysis of genes to be analyzed, in accordance with the inputted gene panel information.

Here, the gene panel information may be any information that can specify the gene panel that has been used in measurement performed by the sequencer 2. Examples of the gene panel information include the gene panel name, the names of genes to be analyzed with the gene panel, the gene panel ID, and the like.

The sequence data reading unit 111 obtains sequence information generated by the sequencer 2. When the information processing apparatus 1 does not have the function of the auxiliary apparatus 2a shown in FIG. 2, the sequence data reading unit 111 obtains sequence information to be analyzed, from the auxiliary apparatus 2a. Meanwhile, when the information processing apparatus 1 has the function of the auxiliary apparatus 2a shown in FIG. 2, the sequence data reading unit 111 obtains sequence information from the sequencer 2, sorts the obtained sequence information for each subject ID or sample ID, and stores the sorted sequence information into the storage unit 12. The sequence data reading unit 111 reads out sequence information to be analyzed, from the storage unit 12, and does not read out sequence information that is not to be analyzed.

On the basis of the gene panel information inputted through the input unit 17, the information selection unit 112 changes the analysis algorithm for performing analysis so as to correspond to the genes to be analyzed with the gene panel indicated by the gene panel information.

The information selection unit 112 outputs an instruction based on the gene panel information, to at least one of the data adjustment unit 113, the mutation identification unit 114, the drug search unit 118, and the report creation unit 115. Through this configuration, the information processing apparatus 1 can output a result of analyzing the sequence information, on the basis of the inputted gene panel information.

That is, the information selection unit 112 is a function block that performs control so as to obtain gene panel information of a gene panel that includes a plurality of genes to be analyzed, and cause the output unit 13 to output the result of analyzing the sequence information on the basis of the obtained gene panel information.

When genes contained in various samples are analyzed in the test institution 120 which performs panel tests, various gene panels are used in accordance with the gene groups to be analyzed of the respective samples.

Even when various combinations of genes to be analyzed have been analyzed by use of various gene panels, the information processing apparatus 1 can appropriately output results of analyzing sequence information because the information processing apparatus 1 is provided with the information selection unit 112.

That is, if the operator merely selects gene panel information, without setting an analysis program to be used in analysis of sequence information and performing analysis for each gene to be analyzed, a result of analysis of each piece of sequence information can be appropriately outputted.

For example, when the information selection unit 112 outputs, to the data adjustment unit 113, an instruction based on the gene panel information, the data adjustment unit 113 performs an alignment process or the like reflecting the gene panel information.

In accordance with the gene panel information, the information selection unit 112 issues an instruction so that the reference sequence (reference sequence in which wild-type genome sequences and mutation sequences are incorporated) to be used by the data adjustment unit 113 when mapping the sequence information is limited only to the reference sequence for the genes that correspond to the gene panel information.

In this case, since the gene panel information has already been reflected in the result of the process performed by the data adjustment unit 113, the information selection unit 112 need not output an instruction based on the gene panel information to the mutation identification unit 114 which subsequently performs a process following the process performed by the data adjustment unit 113.

For example, in a case where the information selection unit 112 outputs an instruction based on the gene panel information to the mutation identification unit 114, the mutation identification unit 114 performs a process reflecting the gene panel information.

For example, in accordance with the gene panel information, the information selection unit 112 issues an instruction so that the region of the mutation database 123 referred to by the mutation identification unit 114 is limited to only mutations related to the genes that correspond to the gene panel information. Accordingly, the gene panel information is reflected in the result of the process performed by the mutation identification unit 114.

(Input of gene panel information)

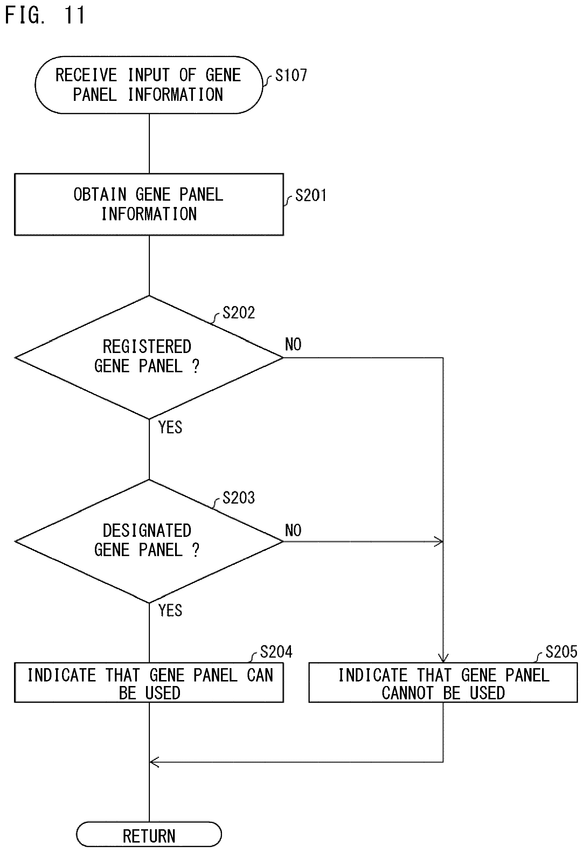

Here, a process for receiving an input of gene panel information shown in step S107 of FIG. 6 is described with reference to FIG. 11. FIG. 11 is a flow chart showing one example of the flow of a process for receiving an input of gene panel information.

Here, an example configuration is described in which the controller 11 causes the input unit 17 to display a GUI for inputting gene panel information, thereby allowing the operator to input gene panel information. Here, an example is described in which the input unit 17 is provided with a touch panel that allows the operator to perform an input operation onto the presented GUI.

First, the controller 11 of the information processing apparatus 1 causes the input unit 17 to display a GUI for allowing the operator to select gene panel information. On the basis of the input operation onto the GUI by the operator, the gene panel information is obtained (step S201).

On the basis of information selected by the operator in the information displayed as the GUI, the information selection unit 112 searches the gene-panel-related information database 121 and reads gene panel information that corresponds to the selected information.

In addition, the information processing apparatus 1 reads gene panel information that is included in the analysis request received from the medical institution 210.

When a gene panel corresponding to the selected information is already registered in the gene-panel-related information database 121 (YES in step S202), and the gene panel matches the gene panel included in the analysis request received from the medical institution 210 (YES in step S203), the information selection unit 112 receives the input. Then, the information selection unit 112 causes the input unit 17 to display a message to the effect that the inputted gene panel can be used (step S204).

Meanwhile, when the gene panel corresponding to the selected information is not registered in the gene-panel-related information database 121, i.e., when an unregistered gene panel has been selected (NO in step S202), the information selection unit 112 causes the input unit 17 to display a message to the effect that the inputted gene panel cannot be used (step S205), and prohibits analysis from being performed by the information processing apparatus 1.

In this case, instead of the message to the effect that the gene panel cannot be used, a message that indicates an error may be displayed. The message may be, for example, "The selected gene panel is not registered." and may further include a message that urges re-input, such as "Please input gene panel information again".

When the gene panel corresponding to the selected information does not match the gene panel included in the analysis request received from the medical institution 210 (NO in step S203), the information selection unit 112 causes the input unit 17 to display a message to the effect that the inputted gene panel cannot be used (step S205), and prohibits analysis from being performed by the information processing apparatus 1.

Also in this case, instead of the message that the gene panel cannot be used, a message that indicates an error may be displayed. The message may be, for example, "The selected gene panel is different from that in the order." and may further include a message that urges re-input, such as "Please input gene panel information again".

This process can prevent performing sequencing by use of an inappropriate gene panel and performing unnecessary analysis operation, and can eliminate wasteful use of gene panels and wasteful operation of the gene analysis system 100.

(Example of GUI used for inputting gene panel information)

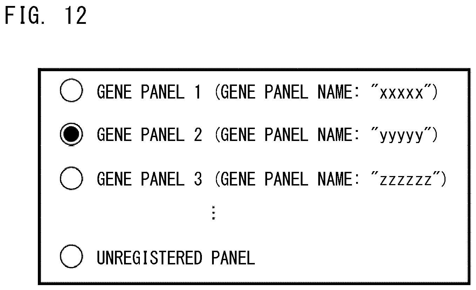

Next, a GUI for allowing the operator to input gene panel information is described with reference to FIG. 12. FIG. 12 shows an example of a GUI to be used for inputting gene panel information.

As shown in FIG. 12, as gene panel information, a list of gene panel names such as "xxxxx" and "yyyyy" is displayed on the GUI, and the operator may be allowed to select a desired gene panel from among the gene panels in the list.

The list of gene panel names on the GUI is displayed on the basis of gene panel names of gene panels that are provided with gene panel IDs and that are already registered in the gene-panel-related information database 121.

In the GUI shown in FIG. 12, "gene panel 2 (gene panel name "yyyyy")" has been selected by the operator. Using the gene panel ID associated with the selected gene panel name "yyyyy" as a key, the information selection unit 112 searches the gene-panel-related information database 121, and obtains gene panel information that corresponds to the inputted gene panel name.

(Gene-panel-related information database 121)

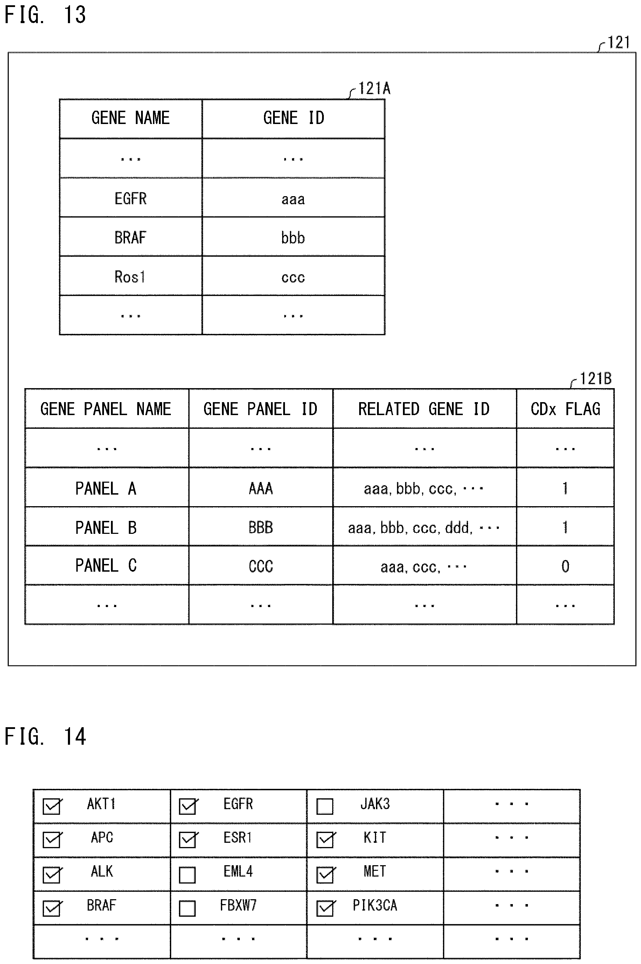

Next, data stored in the gene-panel-related information database 121 referred to by the information selection unit 112 when gene panel information has been inputted through the input unit 17 is described with reference to FIG. 13. FIG. 13 shows an example of a data structure of the gene-panel-related information database 121.

In the gene-panel-related information database 121, as shown in data 121A in FIG. 13, the name of each gene that can be a gene to be analyzed and a gene ID provided to the gene are stored for each gene panel. Gene panels "Panel A", "Panel B", and "Panel C" are each a gene panel (so-called "large panel") that allows batch analysis of a plurality of abnormalities being present in a plurality of genes and related to a plurality of diseases.

In the gene-panel-related information database 121, as shown in data 121B in FIG. 13, the name of each selectable gene panel, a gene panel ID provided to the gene panel, gene IDs of genes to be analyzed with the gene panel (related gene ID), and a CDx flag are stored in association with one another. The CDx flag is a flag that indicates whether the gene panel is for CDx or not. The gene panel for CDx is a gene panel that allows detection of gene mutations to be used for CDx. Each gene panel may also be associated with information regarding whether or not use of the gene panel is already approved by a public institution (for example, Japanese Ministry of Health, Labour and Welfare).

As shown in FIG. 12, when the operator has selected a desired gene panel from among the gene panels presented on the GUI, the information selection unit 112 may refer to the gene-panel-related information database 121 and extract the gene panel ID and the related gene IDs that are associated with the selected gene panel name.

As shown in FIG. 14, when genes to be analyzed have been selected from among the gene names presented on the GUI, the information selection unit 112 refers to the gene-panel-related information database 121 and extracts the gene IDs associated with the selected gene names, and the gene panel ID of the gene panel that includes these gene IDs as the related gene IDs.

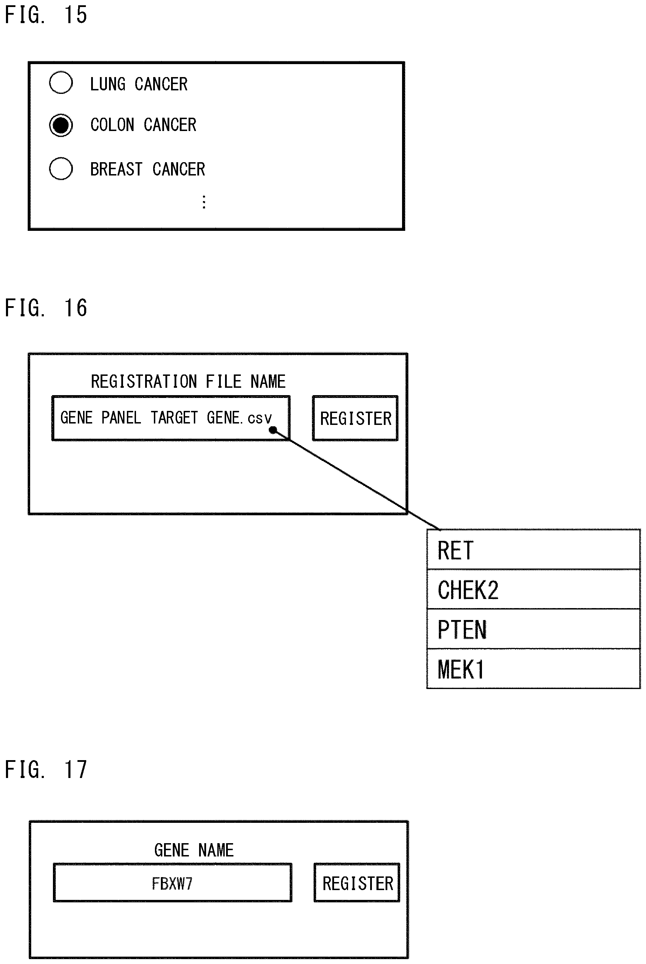

When performing a panel test using a gene panel that allows batch analysis of a plurality of abnormalities being present in a plurality of genes and related to a plurality of diseases, the disease to which each sample is related may be inputted. For example, as shown in FIG. 15, identification information for identifying a disease may be selected from a list of disease names presented on the GUI. The information selection unit 112 outputs the selected/inputted disease name (or disease ID) to the data adjustment unit 113, the mutation identification unit 114, the drug search unit 118, the quality-control unit 117, the report creation unit 115, and the like. On the basis of each sample ID, the information selection unit 112 may automatically obtain a disease name and a disease ID of the subject associated with the sample ID.

As shown in FIG. 7 and FIG. 8, the sample ID of each sample, the disease name of the subject, the disease ID, and the like may be read and obtained from a recording means such as the bar code L11 or the RFID tag L21 attached to the container that stores blood, tissue, or the like derived from the subject. When the sample ID, the disease ID, and the like are obtained by reading the bar code L11, the input unit 17 may be a bar-code reader. When the sample ID, the disease ID, and the like are obtained from the RFID tag L21, the input unit 17 may be a receiving device that has a function of receiving a signal from the RFID tag L21.

<Update of gene-panel-related information database 121>

Here, update of information stored in the gene-panel-related information database 121 is described with reference to FIG. 16 and FIG. 17. FIG. 16 and FIG. 17 each show an example of a GUI to be used when the operator updates the gene-panel-related information database 121.

Update of the information stored in the gene-panel-related information database 121 can be performed by use of an update patch provided from the analysis system management institution 130 to the test institution 120.

Provision of the update patch from the analysis system management institution 130 may be targeted to test institutions 120 that have paid the system usage fee. For example, the analysis system management institution 130 may notify each test institution 120 that the condition for providing an update patch is existence of an update patch that can be provided and payment of the system usage fee. Such a notification can appropriately urge each test institution 120 to pay the system usage fee.

As shown in FIG. 16, when a plurality of genes are updated as a batch, a column for inputting a "registration file name" may be displayed, and the name of a file describing gene names, such as "gene panel target gene.csv", may be inputted in the column. In the example shown in FIG. 16, this "gene panel target gene.csv" includes a plurality of gene names of RET, CHEK2, PTEN, and MEK1.

When a "register" button is pressed after the file name has been inputted, a request for updating the information regarding the genes that correspond to the gene names included in the file is associated with the test institution ID, and is transmitted to the management server 3 via the communication unit 14. The generation of the update request and the association of the update request with the test institution ID may be performed by the controller 11 shown in FIG. 10, for example.

The analysis system management institution 130 permits the information processing apparatus 1 to download information that includes: the gene IDs provided to the gene names included in the update request received by the management server 3; and the gene panel ID provided to the gene panel for analyzing the genes.

Alternatively, as shown in FIG. 17, when the operator performs update by inputting a gene name individually, a column for inputting a "gene name" may be displayed, and a gene name such as "FBXW7" may be inputted in the column.

When a "register" button is pressed after the gene name has been inputted, a request for updating the information regarding the gene that corresponds to the gene name is associated with the test institution ID, and is transmitted to the management server 3 via the communication unit 14. The analysis system management institution 130 permits the information processing apparatus 1 to download information that includes: the gene ID provided to the gene name included in the update request received by the management server 3; and the gene panel ID provided to the gene panel for analyzing the gene.

The column for inputting a "registration file name" in FIG. 16, and the column for inputting a "gene name" in FIG. 17 may include a configuration for displaying input candidates as a suggestion.

For example, information of input candidates to be displayed is provided from the management server 3 to the information processing apparatus 1 in advance, and is stored in the storage unit 12. Then, when a click operation onto the GUI in the input column has been detected, all of the gene names that can be updated may be presented as input candidates to allow the operator to select therefrom, or a gene name that can be updated and that matches the character string inputted by the operator may be presented as an input candidate. Alternatively, for example, at the time point when the operator has inputted one character "E" in the column for inputting a "gene name" shown in FIG. 17, a list of gene names that can be updated such as "EGFR" and "ESR" may be displayed so as to allow the operator to select from the list. By presenting input candidates in this manner, it is possible to prevent the operator from making an erroneous input.

The gene-panel-related information database 121 may store each gene name, the gene ID of the gene, and the name of a protein coded by the gene in association with one another.

In this case, even when the inputted character string is not a gene name but a protein or the like coded by the gene, the information selection unit 112 can obtain a gene name and a gene ID that are associated with the inputted protein name, with reference to the gene-panel-related information database 121.

When a protein name has been inputted in the column for inputting a "gene name" and the register button has been pressed, a GUI may be displayed that shows a gene name associated with the protein name to allow the operator to confirm that the displayed gene name is the correct one.

(Management unit 116)

The management unit 116 stores, in the analysis record log 151, whenever necessary, an analysis record which includes the number of times of operation performed by the analysis execution unit 110, the number of analyzed genes, the total number of identified mutations, and the like, in association with the gene panel IDs and the gene IDs. At a desired frequency (for example, each day, each week, or each month), the management unit 116 reads data including the analysis record and the like from the analysis record log 151, and transmits the data in association with the test institution ID, to the management server via the communication unit 14.

(Communication unit 14)

The communication unit 14 allows the information processing apparatus 1 to communicate with the management server 3 via the communication line 4. Data transmitted from the communication unit 14 to the management server 3 can include the test institution ID, gene panel IDs, gene IDs, analysis records, update requests, and the like. Data received from the management server 3 can include gene panel information, gene names that can be updated, and the like.

(Flow of process for analyzing gene base sequence of sample)

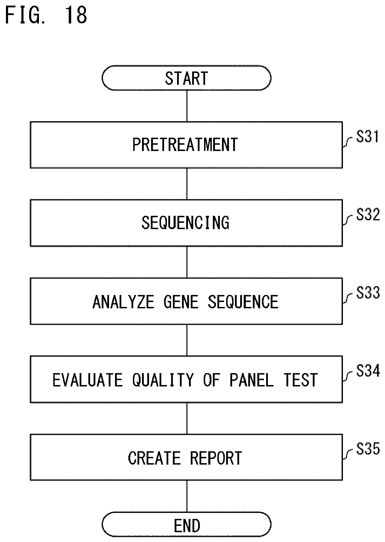

The flow of a process for analyzing base sequences of samples is described with reference to FIG. 18. FIG. 18 is a flow chart showing one example of the flow of the process for analyzing gene base sequences of samples.

First, in step S31 in FIG. 18, pretreatment for analyzing a sequence of each gene to be analyzed is performed. The pretreatment includes processes from fragmentation of nucleic acid such as DNA contained in a sample and a quality control sample to collection of the fragmented nucleic acid. When the sample provided from the medical institution 210 is, for example, a tissue and blood, processes for extracting nucleic acid from the tissue and the blood are also included. In this case, from one subject, a sample that contains nucleic acid extracted from a tissue, and a sample that contains nucleic acid extracted from blood are prepared.

Next, in step S32, base sequences of genes of the sample and nucleic acid contained in the quality control sample which have been subjected to the pretreatment are read by the sequencer 2.

Specifically, step S32 is a step of reading the base sequences of one or a plurality of fragmented genes, to be analyzed, which have been collected after the pretreatment. The sequence information includes the gene base sequences having been read in this step. One or a plurality of fragmented nucleic acids, to be analyzed, which have been collected after the pretreatment may also be referred to as a "library".

Subsequently, in step S33, the information processing apparatus 1 analyzes each gene base sequence having been read, and specifies the presence or absence of mutation in the sequence, the position of the mutation, the type of the mutation, and the like. By the read gene base sequence being analyzed, the detected gene mutation is identified.

Next, when the quality control sample has been measured, the quality-control unit 117 generates, in step S34, a quality evaluation index for evaluating the quality of the panel test. The information processing apparatus 1 may evaluate the quality of the panel test having been performed, on the basis of the generated quality evaluation index.

Lastly, the information processing apparatus 1 creates a report that includes an analysis result such as information related to the gene mutation identified in step S33, and information indicating the quality of the panel test, such as the quality evaluation index generated by the quality-control unit 117 in step S34. The created report is provided to the medical institution 210.

The type of the sequencer 2 that can be used in the present embodiment is not limited in particular, and any sequencer that can analyze a plurality of targets to be analyzed in one run can be suitably used. In the following, one example is described in which a sequencer of Illumina, Inc. (San Diego, CA) (for example, MySeq, HiSeq, NextSeq, or the like), or an apparatus that employs a similar method to that of the sequencer of Illumina, Inc. is used.

Through combination of a Bridge PCR method and a Sequencing-by-synthesis technique, the sequencer of Illumina, Inc. can perform sequencing, with a target DNA amplified and synthesized to a huge number on a flow cell. The sequencer of Illumina, Inc. can simultaneously analyze base sequences of genes of a plurality of subjects.

(a. pretreatment)

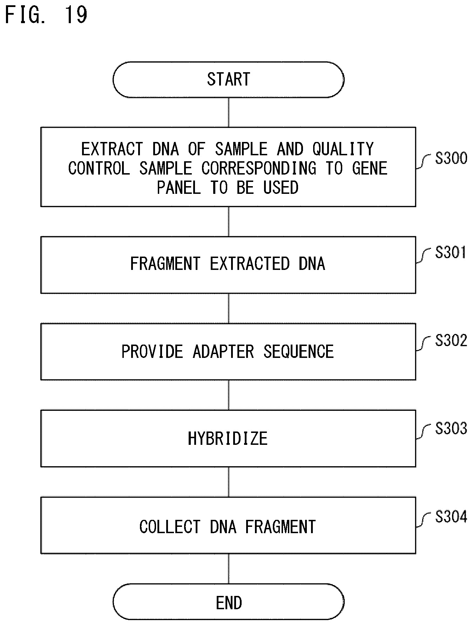

Next, the procedure of the pretreatment in step S31 in FIG. 18 is described with reference to the flow of processes shown in FIGS. 19 to 22. FIGS. 19 to 22 are each a flow chart that describes one example of the procedure of the pretreatment for analyzing gene base sequences of a sample by use of the sequencer 2. In the following, an example case in which the target nucleic acid of sequence analysis is DNA is described.

<Extraction>

When base sequences of each of a sample and the quality control sample are to be analyzed, DNA is firstly extracted from the sample that includes genes to be analyzed and the quality control sample that corresponds to the gene panel to be used (step S300 in FIG. 19).

In this case, the DNA derived from the sample and the DNA derived from the quality control sample are each subjected to the processes of step S301 and the subsequent steps.

Since the DNA extracted from the quality control sample is subjected to the same process as that for the DNA extracted from the sample, a quality evaluation index useful for evaluating the quality of the sequence analysis in the panel test can be generated.

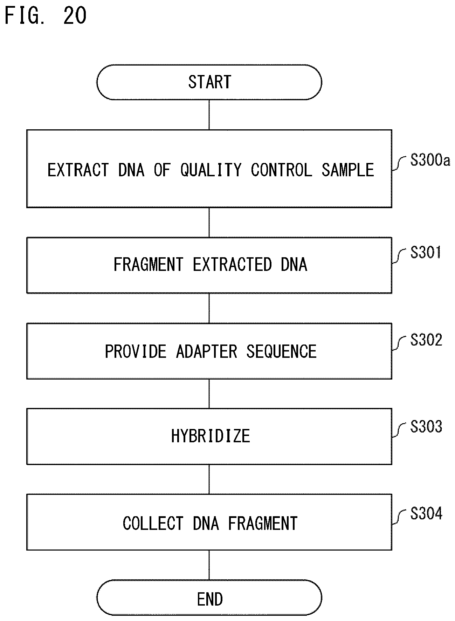

The usage of the quality control sample is not limited thereto. For example, as shown in FIG. 20, DNA of only the quality control sample may be extracted in step S300a, and subjected to the processes of step S301 and the subsequent steps.

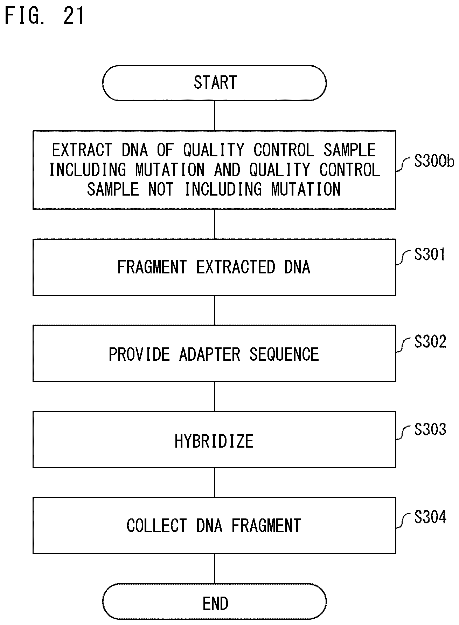

Alternatively, as shown in FIG. 21, a quality control sample that includes mutation and a quality control sample that does not include mutation are prepared as quality control samples, and DNA may be extracted therefrom (step S300b).

By comparison between a result of analysis of DNA derived from the quality control sample that includes mutation and a result of analysis of DNA derived from the quality control sample that does not include mutation, a quality evaluation index useful for evaluating the quality of the sequence analysis in the panel test can be generated.

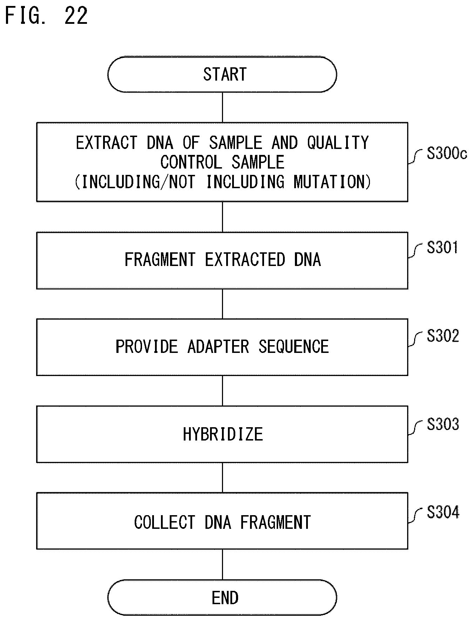

Furthermore, as shown in FIG. 22, DNA may be extracted from each of a sample that includes genes to be analyzed, a quality control sample that includes mutation, and a quality control sample that does not include mutation (step S300c).

The sample that includes genes to be analyzed may be a combination of a blood sample and a tissue (for example, tumor cell) sample. In this case, for one subject, a sample that contains nucleic acid extracted from the tissue, and a sample that contains nucleic acid extracted from the blood are subjected to sequencing as individual samples.

In the processes of step S301 and the subsequent steps, DNA derived from the sample and DNA derived from the quality control sample may be mixed to perform the processes of step S301 and the subsequent steps without individually processing the DNA derived from the sample and the DNA derived from the quality control sample. Accordingly, in all the processes of step S301 and the subsequent steps, the conditions for both of the samples are the same, and thus, a more accurate quality evaluation index can be generated. In addition, it is not necessary to use a part of the lanes in the flow cell used for the sequencer 2, only for the DNA fragments prepared from the quality control sample. Accordingly, the limited number of lanes can be effectively used for DNA fragments derived from the sample that include genes to be analyzed.

In this case, (1) a reagent for appropriately fragmenting a standard gene which is a gene included in the quality control sample and each gene to be analyzed in the panel test, to prepare a library, and (2) a reagent that contains RNA baits for appropriately capturing the respective DNA fragments after the standard gene included in the quality control sample and the gene to be analyzed in the panel test have been fragmented, are preferably used.

<Quality control sample>

In one embodiment, the quality control sample is a composition containing a plurality of standard genes. The quality control sample can be prepared by mixing a plurality of standard genes. A reagent obtained by these standard genes being mixed and stored in a single container can be provided as the quality control sample to the test institution 120. A plurality of standard genes that are stored in separate containers may be provided in the form of a kit as the quality control sample, to the test institution 120. The quality control sample may be in the form of a solution or may be in a solid (powder) state. When the quality control sample is provided in the form of a solution, an aqueous solvent, such as water or TE buffer, known to a person skilled in the art, can be used as the solvent.

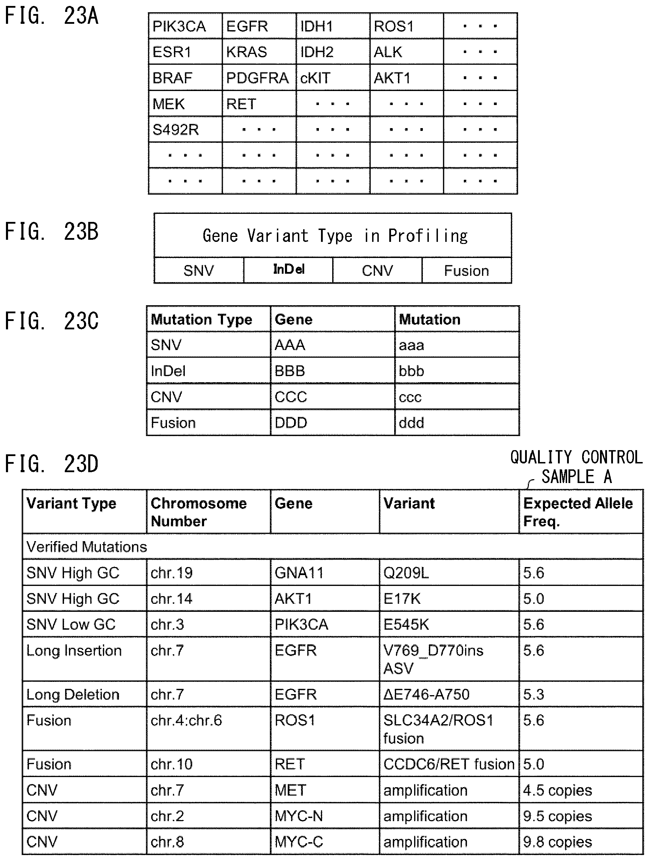

The quality control sample is described with reference to FIG. 23. FIG. 23 illustrates one example of the quality control sample.

FIG. 23A shows a list of genes that can be genes to be analyzed in the panel test using a gene panel. One or a plurality of genes in this list are associated as a gene or genes to be analyzed with a gene panel (see data 121B in FIG. 13).

FIGS. 23B and 23C each show an example of types of mutations to be detected in the panel test. The types of mutations to be detected are "SNV (single nucleotide polymorphism)", "Insertion" and "Deletion" (in the drawing, indicated as "InDel", CNV (copy number variation), and "Fusion".

A quality control sample A1 corresponding to a gene panel A includes at least two of a standard gene that includes SNV, a standard gene that includes Insertion, a standard gene that includes Deletion, a standard gene that includes CNV, and a standard gene that includes Fusion. For example, the quality control sample A1 includes, as the standard gene, a partial sequence of gene A that includes "SNV" with respect to a wild type, and a partial sequence of gene B that includes "Insertion" with respect to a wild type.

FIG. 23D is an example of output of a result of analysis of the quality control sample and a result of analysis of the gene test using the gene panel A. In this example, as the analysis result of the gene panel A, SNV of GNA11. AKT1, and PIK3CA, Long insertion and Long deletion of EGFR, SLC34A2/ROS1 fusion gene, CCDC6/RET fusion gene, gene amplification of MET, gene amplification of MYC-N, and gene amplification of MYC-C are detected. The quality control sample of the gene panel A includes a standard gene that includes SNV of GNA11, a standard gene that includes SNV of AKT1, a standard gene that includes SNV of PIK3CA, a standard gene that includes Long insertion of EGFR, a standard gene that includes Long deletion of EGFR, a standard gene that includes SLC34A2/ROS1 fusion sequence, a standard gene that includes CCDC6/RET fusion sequence, a standard gene that includes gene amplification of MET, a standard gene that includes gene amplification of MYC-N, and a standard gene that includes gene amplification of MYC-C. In this example, the quality control sample includes 10 kinds of standard genes. However, the quality control sample is not limited to this example.

A first standard gene and a second standard gene included in the quality control sample may be different DNA molecules, or may be connected to each other. When the first standard gene and the second standard gene are connected to each other, the sequence of the first standard gene and the sequence of the second standard gene may be directly connected to each other, or a spacer sequence may intervene between the sequence of the first standard gene and the sequence of the second standard gene.

The spacer sequence is preferably a sequence that is less likely to be included in the sample subjected to the gene test. For example, the spacer sequence can be a sequence in which only a plurality (for example, 100) of adenine bases are consecutive.

The standard gene may be a gene that is included in the gene panel to be analyzed, or a gene that is not included in the gene panel to be analyzed. The standard gene may be a gene of a biological species for which the gene test is performed, or a gene of a different biological species. For example, when the gene test is performed for a human, the standard gene can be a gene of an animal other than a human, a plant, a bacterium, or the like.

The method for synthesizing the standard gene is not limited in particular. For example, the standard gene can be synthesized by a known DNA synthesizer. Alternatively, a gene derived from an organism, which serves as a template, is amplified by PCR and purified, whereby the standard gene may be obtained. Alternatively, PCR amplification is performed by using, as a template, a standard gene synthesized by a DNA synthesizer and purification is performed, whereby the standard gene may be obtained.

The length of the standard gene is not limited in particular. For example, the length of the standard gene can be 50 nucleotides or greater. In the case of amplification by PCR, amplification can be advantageously performed with ease if the length of the standard gene is 2000 nucleotides or less. When the standard gene is synthesized by a DNA synthesizer, up to several kbp of the standard gene can be synthesized.

The concentration of the standard gene in the quality control sample is not limited in particular. For example, the concentration of the standard gene can be approximately the same as a DNA concentration in the sample.

The standard gene in the quality control sample may be single-stranded or doublestranded. The standard gene may be linear or circular.

For example, (1) a standard gene that includes substitution mutation is prepared, (2) a standard gene that includes fusion mutation is prepared, and (3) the quality control sample and the sample are mixed together, whereby a sequence analysis sample is prepared. Next, (4) the standard genes and the sample-derived genomic DNA in the sequence analysis sample are subjected to the pretreatment (fragmentation, DNA concentration, PCR amplification using tag primer, and the like) and the sequence analysis, to obtain sequence information of the target gene. In the sequence analysis, an index for quality control is obtained, and the quality of the result of analysis of the target gene is evaluated on the basis of the index of sequence analysis of the standard DNA molecules. The operator is allowed to determine reliability of the result of analysis of the gene to be analyzed, on the basis of the result of the quality evaluation.

In the example above, in (3), the quality control sample and the subject-derived sample are mixed together, but are not limited thereto. For example, the quality control sample and the sample may be separately subjected to the sequence analysis in (4) without being mixed together.

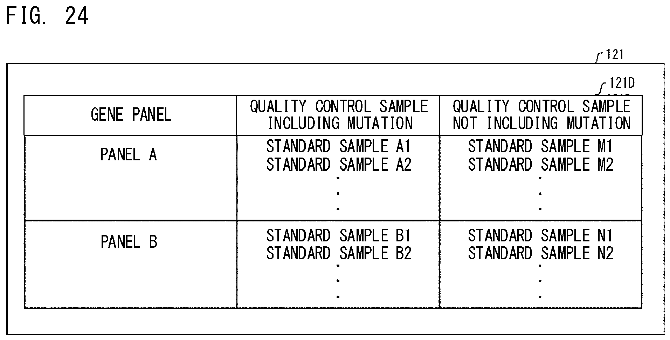

When the panel test using the same gene panel is repeatedly performed, the same quality control sample may be repeatedly used. As shown in data 121D in FIG. 24, a plurality of kinds of quality control samples including different types of mutations and different standard genes may be prepared as a plurality of quality control samples corresponding to each gene panel.

If a plurality of quality control samples having different combinations of standard genes are selectively used for each panel test, each week, or each month, the quality-control unit 117 can generate the quality evaluation index for evaluating the quality of the process for detecting mutations in the panel test, on the basis of detection of mutations of the increased number of kinds of standard genes. Therefore, the comprehensiveness of the quality control of the panel test is improved.

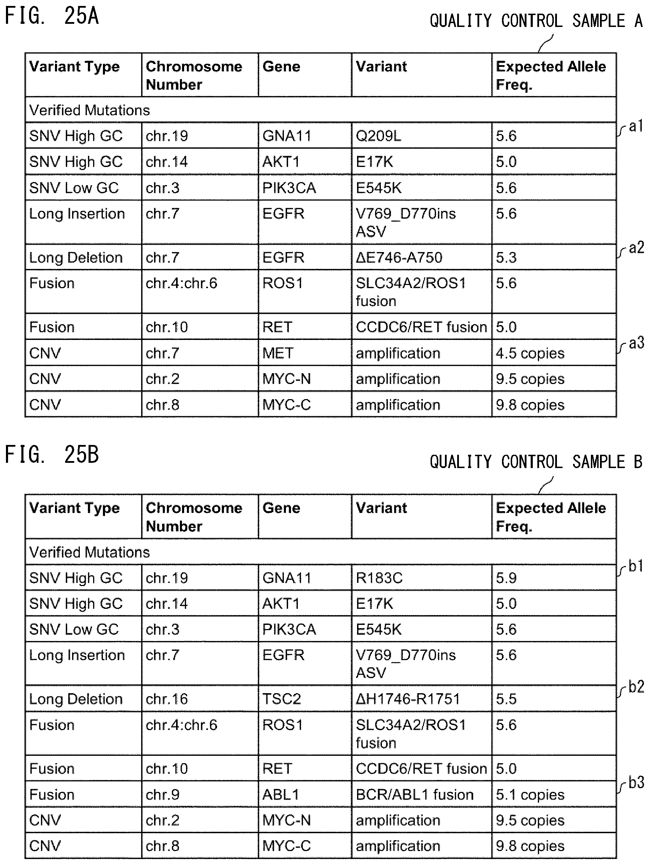

For example, FIGS. 25A and 25B show a quality control sample A and a quality control sample B which are quality control samples that correspond to a gene panel A. A standard gene a1, a standard gene a2, and a standard gene a3 included in the quality control sample A are respectively changed to a standard gene b1, a standard gene b2, and a standard gene b3 in a quality control sample B.

<Fragmentation>

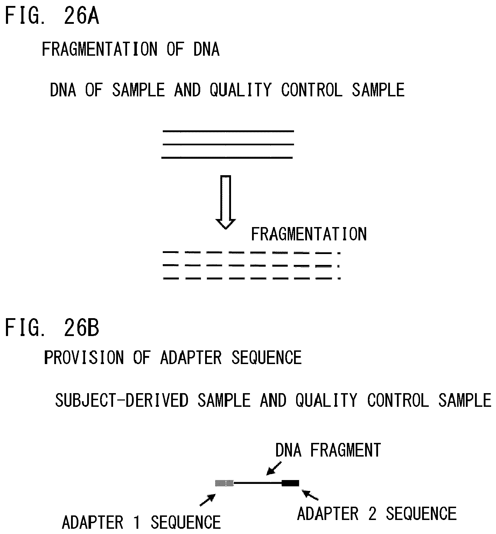

Next, as shown in FIG. 26A, a sample (sample-derived genome DNA and/or standard gene) is fragmented so as to have a length with which the sequencer 2 reads the sequence (step S301 in FIG. 19 to FIG. 22). The sample DNA can be fragmented by a known method such as ultrasonication and a process using a reagent that fragments nucleic acid. Each obtained DNA fragment (nucleic acid fragment) can have a length of, for example, several tens of bp to several hundred bp. When sequencing using a sequencer of Illumina, Inc. is performed, DNA fragments having a length of, for example, 150 to 200 bp are suitable.

<Provision of adapter sequence>

Next, as shown in FIG. 26B, adapter sequences according to the type of the sequencer 2 and the sequencing protocol that are used are added to both ends (3' end and 5' end) of the DNA fragment obtained in step S301 (step S302 in FIG. 19 to FIG. 22). This step is indispensable when the sequencer 2 is a sequencer of Illumina, Inc. or an apparatus that employs a similar method to that of the sequencer of Illumina, Inc. However, when another type of sequencer 2 is used, this step may be omitted in some cases.

The adapter sequence is a sequence to be used for performing sequencing in a later step. According to one embodiment, in Bridge PCR, the adapter sequence can be a sequence that is hybridized with oligo DNA which is the capture molecule immobilized on the flow cell.

In one aspect, as shown in the upper part of FIG. 26B, the adapter sequences (for example, adapter 1 sequence and adapter 2 sequence in FIG. 26) may be added directly to both ends of the DNA fragment. At least one side of each of the adapter 1 sequence and the adapter 2 sequence to be added includes the same sequence as the sequence of the sequence primer to be used in the later-performed sequencing. The adapter 1 sequence and the adapter 2 sequence may be the same base sequence.

The adapter sequence may be added to the DNA fragment by using a known technique in this technical field. For example, the adapter sequence may be added by subjecting the DNA fragment to PCR reaction using a PCR primer that includes the adapter sequence and a sequence of the gene to be analyzed. Alternatively, the DNA fragment may be blunted and the adapter sequence may be ligated.

<Concentration of DNA fragment>

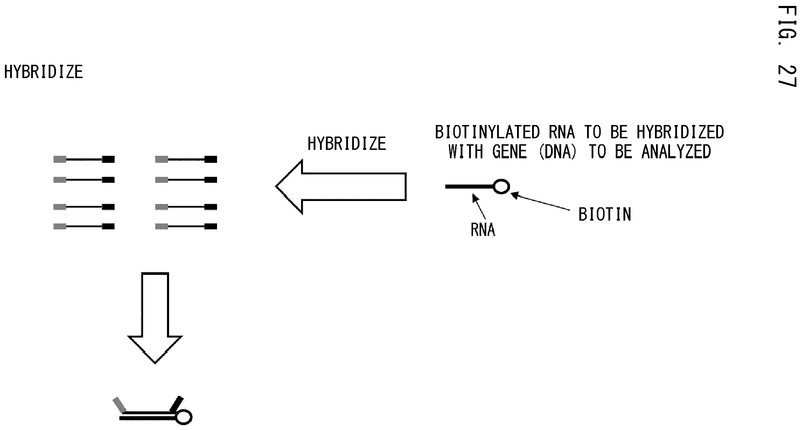

Next, as shown in FIG. 27, a biotinylated RNA bait library is hybridized with the DNA fragment to which the adapter sequences have been added (step S303 in FIG. 19 to FIG. 22).

The biotinylated RNA bait library is composed of biotinylated RNAs (hereinafter, referred to as RNA bait) that are hybridized with genes to be analyzed. The RNA bait may have any length. For example, long oligo RNA bait having about 120 bp may be used in order to enhance specificity.

The panel test using the sequencer 2 in the present embodiment may be a test in which a specific gene is to be analyzed, or may be a test in which a large number of genes (for example, 100 or greater) are to be analyzed.

The reagent to be used in the panel test includes a set of RNA baits that respectively correspond to the large number of genes. When the panel is different, the number and the kinds of genes to be tested are different, and thus, the set of RNA baits included in the reagent to be used in the panel test is also different. When a gene different from a gene to be analyzed is used as a standard gene, a bait that binds to the standard gene needs to be prepared.

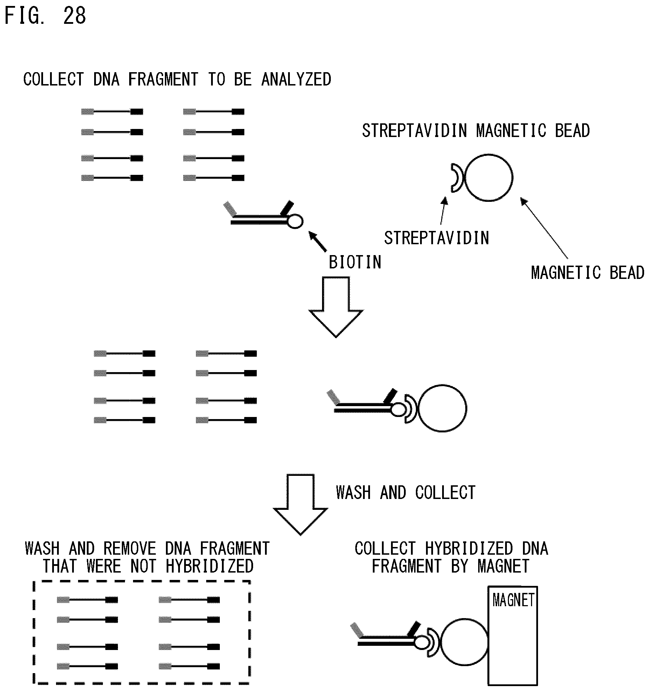

As shown in FIG. 28, DNA fragments to be analyzed are collected (step S304 in FIG. 19 to FIG. 22). Specifically, as shown in the upper part of FIG. 28, the DNA fragments hybridized with the biotinylated RNA bait library are mixed with streptavidin magnetic beads which are each composed of streptavidin and a magnetic bead bound to each other.

Accordingly, as shown in the middle part of FIG. 28, the streptavidin part of the streptavidin magnetic bead and the biotin part of the RNA bait are bound to each other. Then, as shown in the lower part of FIG. 28, the streptavidin magnetic beads are collected by a magnet, and the fragments that are not hybridized with the RNA baits (i.e., DNA fragments of genes that are not to be analyzed) are removed by washing.

Accordingly, the DNA fragments hybridized with the RNA baits, i.e., the DNA fragments to be analyzed, can be selectively collected and concentrated. This process is performed for each sample, whereby the library of each sample is prepared (see step I in FIG. 60).

(Preparation of measurement sample)

In a case where base sequences of genes of a plurality of subjects are to be simultaneously analyzed, the measurement sample to be applied to the flow cell is prepared by mixing libraries of a plurality of samples (see step II in FIG. 60). In the sequence information of base sequences that are read through sequencing, pieces of sequence information of DNA derived from samples derived from a plurality of subjects are present. Thus, prior to sequencing, an index sequence is added to each DNA fragment prepared from each subject-derived sample.

<Provision of index sequence>

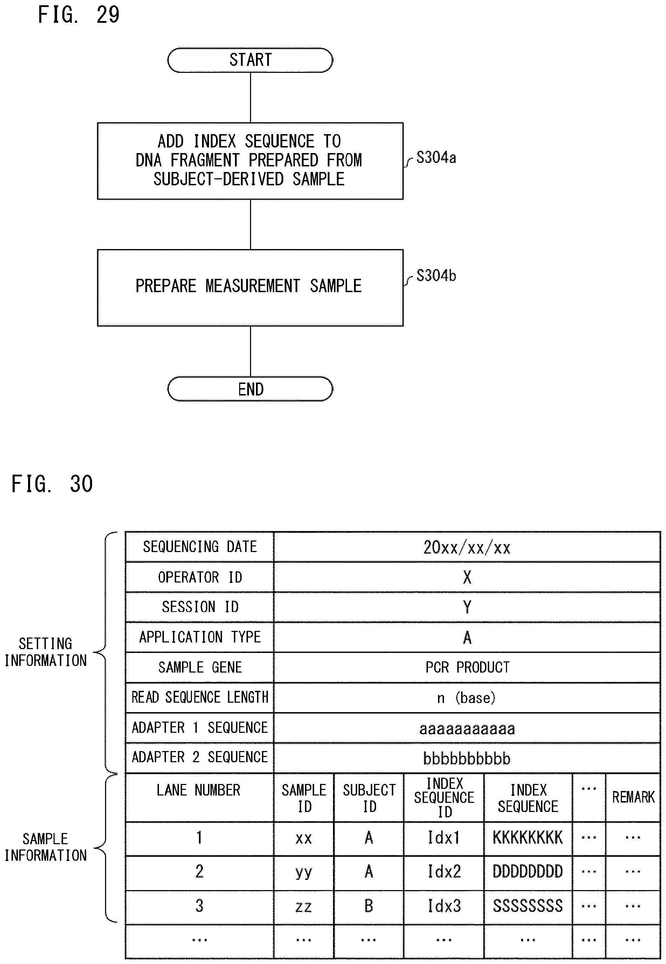

In order to enable sorting of base sequences for each subject or for each sample from the sequence information of DNA of samples derived from a plurality of subjects, an index sequence that is different for each library is added. FIG. 29 is a flow chart describing one example of the procedure of preparing a measurement sample to be applied to a flow cell.

In step S304a in FIG. 29, an index sequence is added to the DNA fragments prepared from each subject-derived sample. At this time, the libraries of subject-derived genes mixed in the measurement sample that is to be applied to the same flow cell have different index sequences added thereto, respectively.

Accordingly, pieces of sequence information of base sequences regarding genes of samples derived from different subjects can be distinguished from one another on the basis of the base sequences of the index sequences added thereto. If no index sequence is added to nucleic acid that is not to be analyzed (for example, non-subject-derived genes, genes derived from the quality control sample, and the like), only the sequence information of base sequences of a subject-derived sample can be made the target to be analyzed.

The index sequence can be added to the DNA fragments by use of a known technique in this technical field. For example, in a case where SureSelect XT of Agilent is used, if the DNA fragments collected in step S304 in FIG. 22 are subjected to PCR reaction using a PCR primer that includes an index sequence, the index sequence is added to each DNA fragment.

FIG. 29 shows an example in which the index sequence is added to each DNA fragment collected in step S304 in FIG. 22, but not limited thereto. The addition of the index sequence may be performed before step S304 in FIG. 22. For example, in a case where SureSelect XT2 of Agilent is used, if PCR reaction using a PCR primer including an index sequence is performed in step S302 in FIG. 22, the index sequence is added to each DNA fragment.

Alternatively, the index sequence may be added when the adapter sequence is added to each DNA fragment. For example, the index sequence may be added by subjecting the DNA fragments to PCR reaction using a PCR primer that includes the adapter sequence, the index sequence, and a sequence of the gene to be analyzed.

<Measurement sample sheet>

Next, in step S304b in FIG. 29, a measurement sample is prepared by mixing a plurality of libraries prepared from subject-derived samples, to each of which an index sequence has been added.

In preparation of a measurement sample, a measurement sample sheet in which a sample ID is associated with an index sequence ID and an index sequence added to the library of each sample is created and managed. FIG. 30 shows one example of a measurement sample sheet created at the time of preparation of a measurement sample that is to be subjected to sequencing.

The measurement sample sheet may include setting information which is common among the libraries of all the samples included in the measurement sample, and sample information specific to the library of each sample included in the measurement sample. As shown in FIG. 30, the setting information may include "sequencing date", "operator ID" which is an ID of the operator of the sequencer 2, "session ID" which is an ID assigned for each test, the type of application used in the sequencer 2, and the like.

The setting information may include "sample gene" which is information related to the method for preparing the libraries of samples used in preparation of a measurement sample. In the column "sample gene", "PCR product", "amplicon", or the like can be entered, for example.

Further, the setting information may include "read sequence length" which is the set value of the length of the base sequence read by the sequencer 2, information related to the adapter 1 sequence and the adapter 2 sequence, and the like. Here, the read sequence is the base sequence read through sequencing by the sequencer 2.

As shown in FIG. 30, the sample information may include, for each "lane number" of the flow cell, "sample ID", "subject ID", "index sequence ID" of the index sequence added to the sample, "index sequence" which is the base sequence of the index sequence, and the like.

As for the measurement sample sheet, the sequencer 2 or the auxiliary apparatus 2a shown in FIG. 2 may obtain the measurement sample sheet inputted by the operator. Alternatively, the measurement sample sheet may be inputted by the operator through the input unit 17 of the information processing apparatus 1 shown in FIG. 3.

<Method for preparing measurement sample>

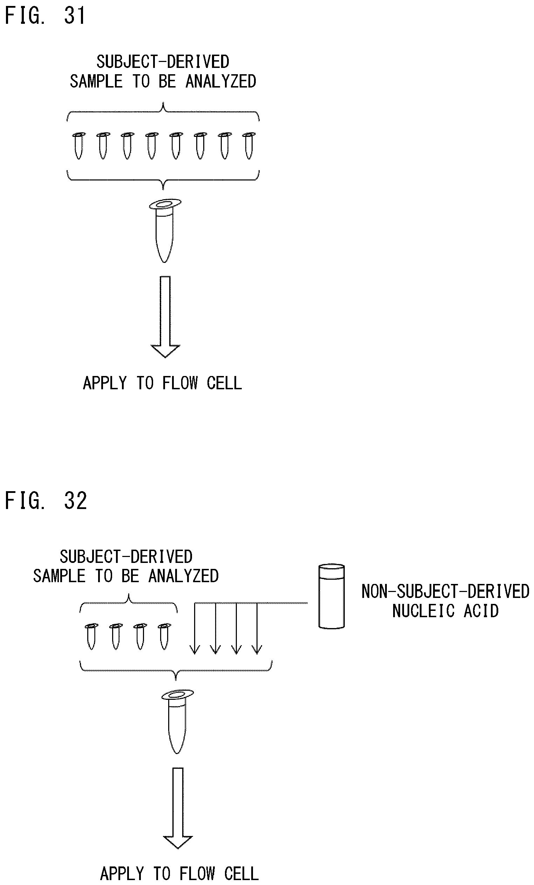

Here, a method for preparing a sample is described with reference to FIGS. 31 to 33. In FIGS. 31 to 33, an example case is described in which the number of samples recommended to be included in a measurement sample to be subjected to one sequencing run is 8.

FIG. 31 illustrates a method for preparing a measurement sample by mixing a plurality of libraries prepared from subject-derived samples to be analyzed. As shown in the drawing, when the number of subject-derived samples to be analyzed is the recommended number of samples, the measurement sample can be prepared by mixing these samples as shown in FIG. 31.

Accordingly, the measurement sample is prepared so as to contain a previously determined amount of nucleic acid, by mixing the libraries prepared from the recommended number of subject-derived samples. Here, the "previously determined amount of nucleic acid" means the amount of nucleic acid recommended in accordance with the specification of the flow cell that suits the sequencer 2 and the amounts of the primer, the probe, and the like included in the gene panel. Here, the amount of nucleic acid is the number of moles of nucleic acid.

The molar concentration of nucleic acid can be calculated on the basis of, for example, the absorbance at 260 nm, the molecular weight of the DNA fragment, and the molar absorption coefficient of nucleic acid. After purifying the PCR product after the PCR reaction for adding the index sequence is performed in step S304a in FIG. 29, the absorbance at 260 nm is measured. On the basis of the measured absorbance and the molar absorption coefficient, the concentration (for example, ng/$\mu$l) of the PCR product is calculated.

For example, in a case where the length of the library obtained as the PCR product is 100 bp, and the concentration is x (ng/$\mu$l), if 330 is used as the average molecular weight of deoxyribonucleotide, the molar concentration of the PCR product is calculated as x/33 (pmol/μl). When a previously determined amount (for example, y (pmol)) of the nucleic acid of this library is mixed, 33×x/y (μl) is used to prepare the measurement sample, with use of an autopipette or the like.

In a case where the number of subject-derived samples to be analyzed is insufficient, even if libraries prepared from the subject-derived samples are mixed by the same amount as the amount that is used when the number of subject-derived samples to be analyzed is the recommended number of samples, the amount of nucleic acid of the measurement sample does not become the previously determined amount of nucleic acid. However, if, in order to attain the previously determined amount of nucleic acid, the amount of libraries prepared from the subject-derived samples is increased or decreased to prepare a measurement sample, the data amount of sequence information obtained per sample will vary for each sequencing run.

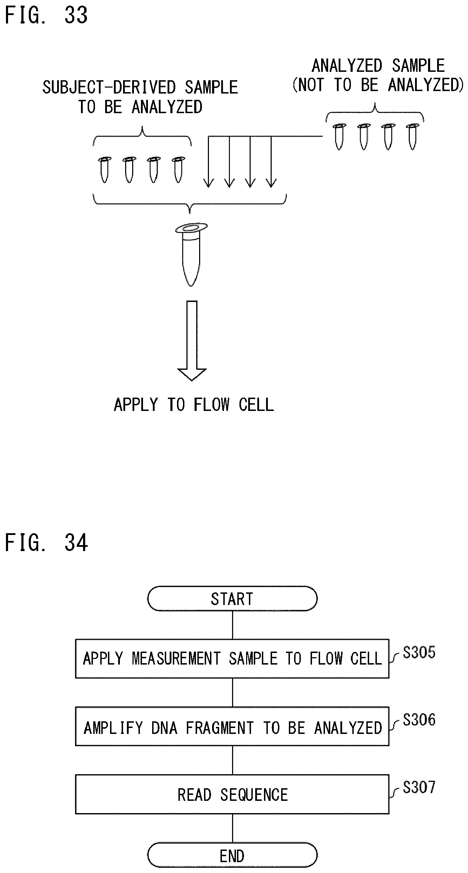

Therefore, even when the number of subject-derived samples to be analyzed is insufficient, it is preferable that the libraries prepared from the subject-derived samples are mixed by the same amount as the amount that is used when the number of subject-derived samples to be analyzed is the recommended number of samples, while the amount of nucleic acid of the measurement sample is made the previously determined amount of nucleic acid. Such a method for preparing the measurement sample is described with reference to FIGS. 32 and 33.

FIG. 32 illustrates one example of the method for preparing the measurement sample when the number of subject-derived samples to be analyzed is insufficient. As shown in the drawing, when the number of subject-derived samples to be analyzed is smaller than the recommended number of samples, it is sufficient that non-subject-derived nucleic acid that has the adapter sequence added thereto is used to make up for the insufficient amount, to prepare a measurement sample. The variation in the amount of nucleic acid derived from each sample included in the measurement sample is preferably in a range of ±10%.

In this case, the amount of the non-subject-derived nucleic acid included in the measurement sample may be at least an amount corresponding to, or greater than, the amount of nucleic acid per sample included in the measurement sample. Examples of the non-subject-derived nucleic acid include "PhiX DNA" or the like provided from Illumina, Inc. but is not limited thereto. For example, nucleic acid or the like, of a quality control sample for a gene panel, that has an adapter sequence added thereto may be used. In order not to hinder the reading of base sequences in the sequencer 2, it is preferable to use high diversity nucleic acid (i.e., nucleic acid having high diversity in sequences) in which nucleic acids having diverse base sequences are mixed, compared with low diversity nucleic acid (i.e., nucleic acid having low diversity in sequences) in which a large amount of nucleic acids having the same base sequences is included.

Alternatively, in a case where the number of subject-derived samples to be analyzed is smaller than the recommended number of samples, it is sufficient that libraries having been prepared from subject-derived samples and having already been analyzed (i.e., those not to be analyzed any more) are used as a substitute, to prepare a measurement sample. As the libraries prepared from subject-derived samples having already been analyzed, libraries each having added thereto an index sequence different from any of the index sequences added to libraries that have been prepared from subject-derived samples to be analyzed and that are to be mixed in order to prepare a measurement sample, are used.

For example, in a case where a first index sequence is added to a library prepared from a subject-derived sample to be analyzed, it is sufficient that a library prepared from a subject-derived sample that has been analyzed and that has added thereto a second index sequence different from the first index sequence is used to prepare a measurement sample.

Also in this case, the variation in the amount of nucleic acid derived from each sample included in the measurement sample is preferably in a range of ±10%. The amount of the subject-derived nucleic acid having been analyzed included in the measurement sample may be at least an amount corresponding to, or greater than, the amount of nucleic acid per sample included in the measurement sample.

When the measurement sample is prepared according to the methods shown in FIG. 32 and FIG. 33, the amount of each library prepared from a subject-derived sample in the measurement sample does not vary. Thus, the data amount of sequence information obtained per sample does not vary for each sequencing run. That is, even if the number of subjects in a first subject group to be analyzed in a sequencing run is different from the number of subjects in a second subject group to be analyzed in another sequencing run, it is sufficient that the amount of non-subject-derived nucleic acid in the measurement sample is adjusted in accordance with the difference between the number of subjects in the first subject group and the number of subjects in the second subject group.

(Reading of read sequence performed by sequencer 2)

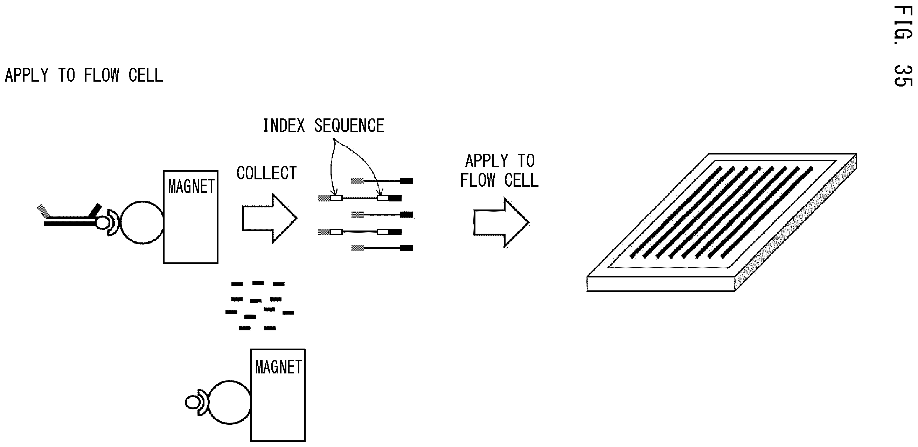

Next, with reference to FIG. 35 to FIG. 37 as appropriate, the procedure of step S32 shown in FIG. 18 is described on the basis of the flow of the process shown in FIG. 34. FIG. 34 is a flow chart describing one example of the procedure of analyzing base sequences of sample DNA by using the sequencer 2.

As shown in the left part to the center part of FIG. 35, the streptavidin magnetic beads and the RNA baits are removed from the concentrated DNA fragments, and the resultant DNA fragments are amplified through PCR, whereby the pretreatment is completed. Here, the amplified DNA fragments may be subjected to PCR reaction using a PCR primer that includes an index sequence, thereby adding the index sequence to the DNA fragments.

Although FIG. 35 shows an example in which the index sequence is added to both the 5' end side and the 3' end side of each DNA fragment, the addition is not limited thereto. The index sequence may be added to only the 5' end side or only the 3' end side of each DNA fragment.

Next, as shown in the right part of FIG. 35, the measurement sample prepared according to the methods shown in FIGS. 31 to 33 is applied to the flow cell (step S305 in FIG. 34). In the measurement sample applied to the flow cell, nucleic acid having adapter sequences and an index sequence added thereto and nucleic acid having only adapter sequences added thereto are mixed.

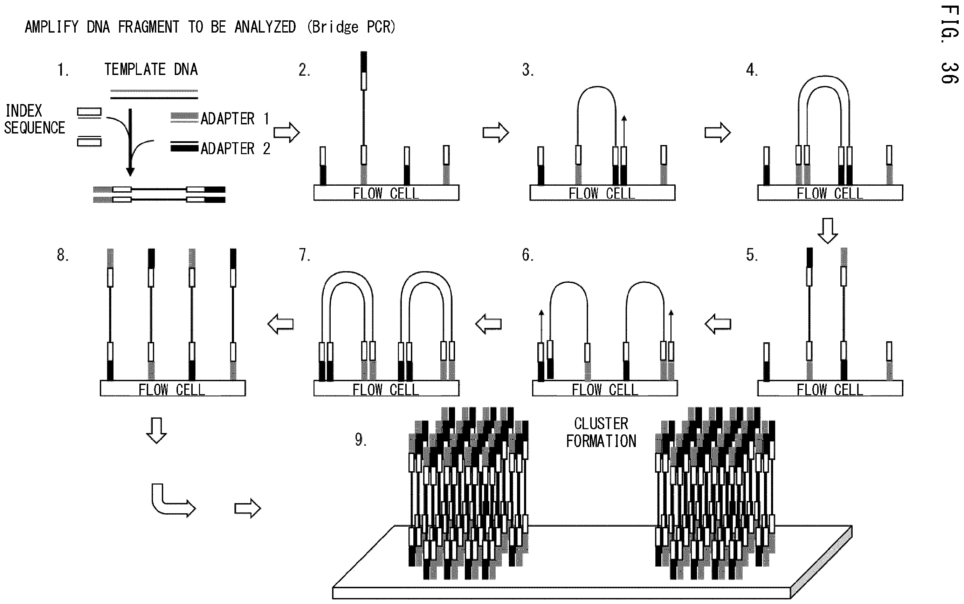

Subsequently, as shown in FIG. 36, the DNA fragments to be analyzed are amplified on the flow cell through Bridge PCR (step S306 in FIG. 34).

That is, each DNA fragment to be analyzed (for example, Template DNA in FIG. 36) is in a state where both ends of the DNA fragment have two different kinds of adapter sequences (for example, adapter 1 sequence and adapter 2 sequence in FIG. 36) added thereto through the pretreatment described above ("1" in FIG. 36). This DNA fragment is separated into single strands, and the adapter 1 sequence at the 5' end side is immobilized on the flow cell ("2" in FIG. 36).

On the flow cell, the adapter 2 sequence on the 3' end side is immobilized in advance, and the adapter 2 sequence on the 3' end side of the DNA fragment is bound to the adapter 2 sequence on the 3' end side on the flow cell to produce a bridge-like state, whereby a bridge is formed ("3" in FIG. 36).

When DNA elongation is caused by DNA polymerase in this state ("4" in FIG. 36), and denaturation is caused, two single-stranded DNA fragments are obtained ("5" in FIG. 36).

Through repetition of the bridge formation, the DNA elongation, and the denaturation in this order, a large number of single-stranded DNA fragments are locally amplified and immobilized, whereby clusters can be formed ("6" to "9" in FIG. 36).

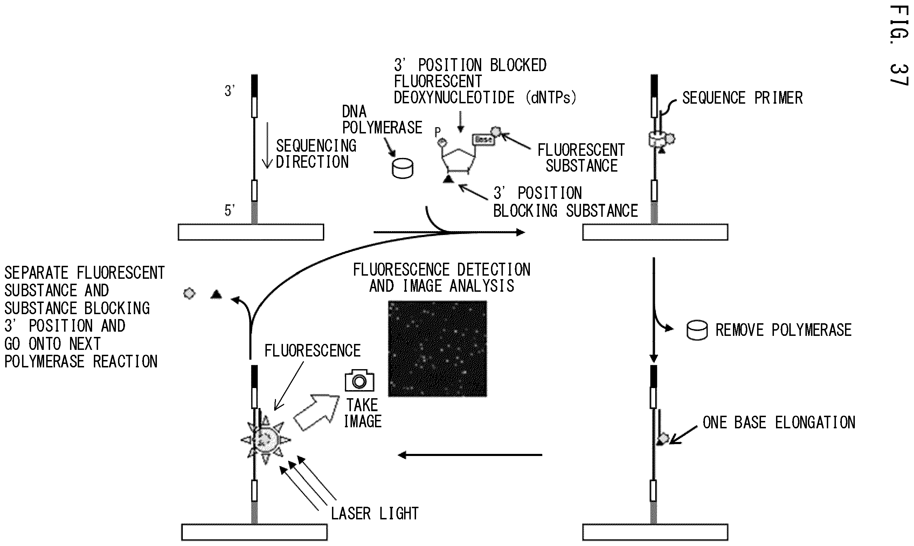

Then, as shown in FIG. 37, the single-stranded DNA forming the cluster is used as a template, and the sequence is read by sequencing-by-synthesis (step S307 in FIG. 34).

First, to the single-stranded DNA immobilized on the flow cell (the upper left part of FIG. 37), a DNA polymerase and dNTP that is labeled with fluorescence and that has the 3' end side blocked are added (the upper center part of FIG. 37), and a sequence primer is further added thereto (the upper right part of FIG. 37).

The sequence primer may be any sequence primer that is designed so as to be hybridized to a part of the adapter sequence, for example. In other words, it is sufficient that the sequence primer is designed to amplify the DNA fragment derived from the sample DNA. In a case where an index sequence is added, it is sufficient that the sequence primer is designed to further amplify the index sequence.

After the sequence primer is added, one base elongation is caused, by the DNA polymerase, for dNTP labeled with fluorescence and having the 3' end blocked. Since dNTP having the 3' end side blocked is used, polymerase reaction stops when one base elongation has been realized. Then, the DNA polymerase is removed (the right middle part of FIG. 37), laser light is applied to the single-stranded DNA elongated by one base (the lower right part of FIG. 37) to excite the fluorescent substance bound to the base, and a photograph of light generated at this time is taken and recorded (the lower left part of FIG. 37).

In order to determine four kinds of bases, the photographs are taken by a fluorescence microscope for the fluorescent colors respectively corresponding to A, C, G, and T, while a wavelength filter is changed. After all the photographs have been obtained, bases are determined from the photograph data. Then, the fluorescent substance and the protecting group blocking the 3' end side are removed, and the reaction goes onto the next polymerase reaction. With this flow assumed as one cycle, the second cycle, the third cycle, and so on are performed, whereby sequencing of the entire length can be performed.

According to the technique described above, the length of the chain that can be analyzed reaches 150 bases ×2, and analysis in a unit much smaller than the unit of a picotiter plate can be performed. Thus, due to the high density, a huge amount of sequence information of 40 to 200 Gb can be obtained in one analysis.

(Gene panel)

The gene panel used for reading the read sequences by the sequencer 2 means an analysis kit for analyzing a plurality of targets to be analyzed in one run as described above. In one embodiment, the gene panel can be an analysis kit for analyzing a plurality of gene sequences related to a plurality of diseases.

When used herein, the term "kit" is intended to mean a package that includes containers (for example, bottles, plate, tubes, and dishes) each containing a specific material. Preferably, the kit includes instructions for using each material. When used in the context of a kit herein, "include (is included)" is intended to mean a state of being included in any of individual containers that form a kit. The kit can be a package in which a plurality of different compositions are packed into one, and the forms of the compositions can be as described above. In the case of a solution form, the solution may be contained in a container.

The kit may include a substance A and a substance B that are mixed in one container or that are in separate containers. The "instructions" indicate the procedure of applying each component in the kit to a therapy and/or diagnosis. The "instructions" may be written or printed on paper or any other medium, or may be stored in an electronic medium such as a magnetic tape, a computer readable disk or tape, or a CD-ROM. The kit can include a container that contains a diluent, a solvent, a washing liquid, or another reagent. Further, the kit may also include an apparatus that is necessary for the kit to be applied to a therapy and/or diagnosis.

In one embodiment, the gene panel may be provided with one or more of the quality control sample, reagents such as the reagent for fragmenting nucleic acid, the reagent for ligation, the washing liquid, the PCR reagent (dNTP, DNA polymerase, etc.), and the magnetic beads, as described above. The gene panel may be provided with one or more of oligonucleotides for adding the adapter sequences to the fragmented DNA, oligonucleotides for adding the index sequence to the fragmented DNA, the RNA bait library, and the like.

The index sequence provided to each gene panel can be a sequence that is unique to the gene panel and that identifies the gene panel. The RNA bait library provided to each gene panel can be a library that is unique to the gene panel and that includes RNA baits that correspond to the test genes of the gene panel.

(Control by information processing apparatus 1 based on information of measurement sample sheet)

In a case where each piece of information included in the measurement sample sheet shown in FIG. 30 has been inputted by an operator through the input unit 17 of the information processing apparatus 1, the information selection unit 112 provides the inputted information to at least one of the data adjustment unit 113, the mutation identification unit 114, the drug search unit 118, and the report creation unit 115.

Accordingly, on the basis of the sample information in the measurement sample sheet, the information processing apparatus 1 can selectively analyze only the sequence information of a gene of a subject-derived sample having a predetermined index sequence added thereto, in the entire sequence information obtained from the sequencer 2.

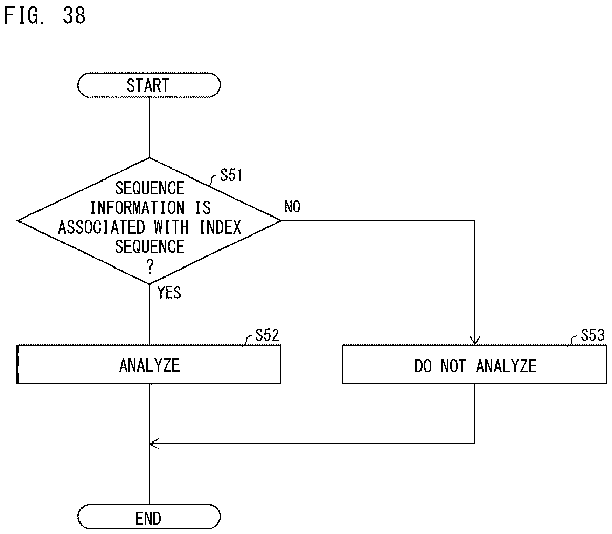

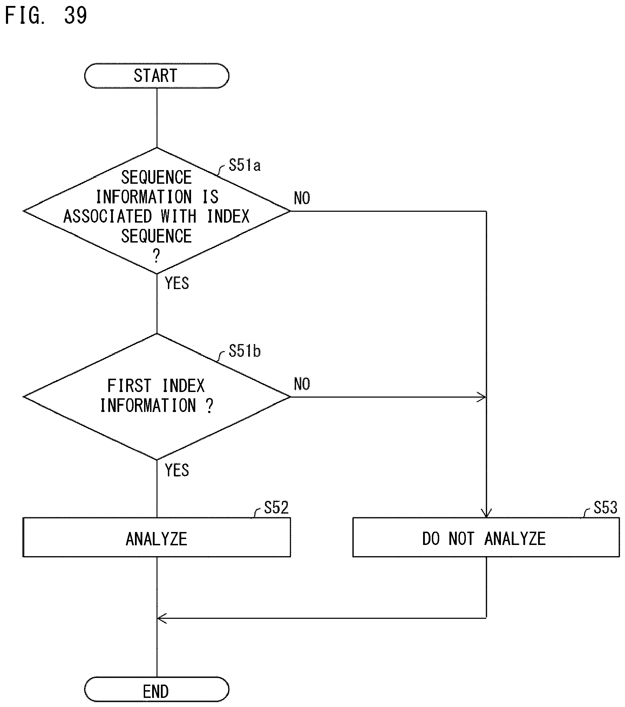

The control performed by the information processing apparatus 1 based on the information of the measurement sample sheet is described with reference to FIG. 38 and FIG. 39. FIG. 38 is a flow chart showing one example of the flow of processes performed by the information processing apparatus when a measurement sample is prepared according to the method shown in FIG. 32. FIG. 39 is a flow chart showing one example of the flow of the processes performed by the information processing apparatus when a measurement sample is prepared according to the method shown in FIG. 33.

When a measurement sample is prepared according to the method shown in FIG. 32, each subject-derived nucleic acid has both adapter sequences and an index sequence added thereto, but the non-subject-derived nucleic acid has only the adapter sequences added thereto.

For example, the aforementioned PhiX provided from Illumina, Inc. has adapter sequences already ligated thereto, and can be suitably used as non-subject-derived nucleic acid.

When the sequence information is associated with an index sequence in the measurement sample sheet (i.e., sequence information that includes an index sequence) (YES in step S51), the information processing apparatus 1 performs analysis (step S52). When the sequence information is not associated with any index sequence (NO in step S51), the information processing apparatus 1 does not perform at least a part of the analysis that is to be performed on the sequence information associated with an index sequence (step S53). That is, the information processing apparatus 1 selectively performs the processes of step S109 and the subsequent steps shown in FIG. 6 on the sequence information of nucleic acid of a library having an index sequence added thereto, in the entire sequence information obtained from the sequencer 2. The information processing apparatus 1 does not perform the processes of step S109 and the subsequent steps shown in FIG. 6 on the sequence information of nucleic acid not having any index sequence added thereto, in the entire sequence information obtained from the sequencer 2.

Meanwhile, when a measurement sample is prepared according to the method shown in FIG. 33, each subject-derived nucleic acid having been analyzed has an index sequence that is different from the index sequence added to each subject-derived nucleic acid to be analyzed. Here, the index sequence added to subject-derived nucleic acid to be analyzed is referred to as "first index sequence" for clarification.

When the sequence information is associated with an index sequence in the measurement sample sheet (YES in step S51a), the information processing apparatus 1 advances to step S51b, and when the sequence information is not associated with any index sequence (NO in step S51a), the information processing apparatus 1 advances to step S53.

In step S51b, the information processing apparatus 1 refers to the measurement sample sheet, and when the sequence information is associated with the first index sequence added to nucleic acid prepared from a sample to be analyzed (YES in step S51b), the information processing apparatus 1 performs analysis (step S52). Meanwhile, when the sequence information is not associated with the first index sequence (NO in step S51a), the information processing apparatus 1 does not perform at least a part of the analysis that is to be performed on the sequence information associated with an index sequence (step S53). That is, the information processing apparatus 1 selectively performs the processes of step S109 and the subsequent steps shown in FIG. 6 on the base sequence data of nucleic acid of a library having the first index sequence added thereto, in the entire sequence information obtained from the sequencer 2. The information processing apparatus 1 does not perform the process of step S109 and the subsequent steps shown in FIG. 6 on the base sequence data of nucleic acid of a library not having the first index sequence added thereto, in the entire sequence information obtained from the sequencer 2.

According to this configuration, the information processing apparatus 1 can efficiently perform the analysis only on the base sequences of the samples to be analyzed.

(Sequence data reading unit 111, data adjustment unit 113, and mutation identification unit 114)

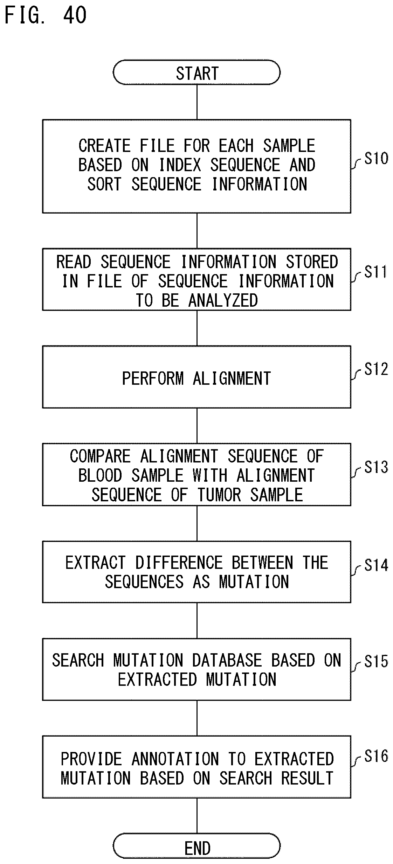

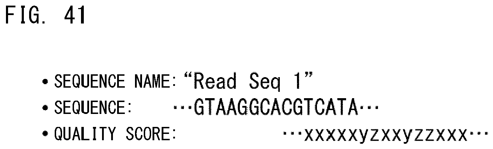

Next, the processes performed by the sequence data reading unit 111, the data adjustment unit 113, and the mutation identification unit 114 of the analysis execution unit 110 are described on the basis of the flow of the process shown in FIG. 40, with reference to FIG. 41 to FIG. 46 as appropriate.

FIG. 40 is a flow chart describing one example of the flow of analysis performed by the information processing apparatus 1. The process shown in FIG. 40 corresponds to step S109 shown in FIG. 6.

<Sequence data reading unit 111>

First, in step S10 shown in FIG. 40, the sequence data reading unit 111 obtains sequence information provided from the sequencer 2, creates a file for each sample on the basis of the index sequence, and sorts the sequence information. For each piece of sequence information that includes an index sequence, a file different for each index sequence is created. For sequence information that does not include any index sequence, a file separate from the file created for each index sequence is created.

The sequence information is data indicating base sequences read by the sequencer 2. The sequencer 2 performs sequencing on a large number of nucleic acid fragments obtained by use of a specific gene panel, reads the sequence information thereof, and provides the information processing apparatus 1 with the read sequence information as sequence information.

The sequence data reading unit 111 may obtain sequence information read from an exon region of a nucleic acid sequence, or may obtain sequence information read from an exon region having at least 10 Mb (10 million bases) or greater.

Next, in step S11, the sequence data reading unit 111 reads sequence information stored in a file of the sequence information to be analyzed.

In one aspect, the sequence information may include a quality score of each base in the sequence as well as the sequence having been read. Both the sequence information obtained by subjecting, to the sequencer 2, an FFPE sample from a lesion site of a subject and the sequence information obtained by subjecting a blood sample of the subject to the sequencer 2 are inputted to the information processing apparatus 1.

FIG. 41 shows one example of a file format used when sequence information to be analyzed is outputted to the information processing apparatus 1. In the example shown in FIG. 41, the sequence information includes a sequence name, a sequence, and a quality score. The sequence name may be a sequence ID or the like provided to the sequence information outputted by the sequencer 2. The sequence indicates the base sequence read by the sequencer 2. The quality score indicates the probability of incorrect base assignment performed by the sequencer 2. Any base sequence quality score (Q) is represented by the following equation.

$$Q = -10\log10E$$

In this equation, E represents an estimated value of the probability of incorrect base assignment. The greater the value of Q is, the lower the probability of the error is. The smaller the value of Q is, the greater the portion of the read that cannot be used is.

In addition, false-positive mutation assignment also increases, which could result in a lowered accuracy of the result. "False-positive" means that the read sequence is determined as having mutation although the read sequence does not have true mutation to be determined.

"Positive" means that the read sequence has true mutation to be determined, and "negative" means that the read sequence does not have mutation to be determined. For example, if the quality score is 20, the probability of error is 1/100. This means that the accuracy (also referred to as "base call accuracy") of each base in the gene sequence having been read is 99%.

<Data adjustment unit 113>

Subsequently, in step S12 shown in FIG. 40, on the basis of the sequence information read by the sequence data reading unit 111, the data adjustment unit 113 performs alignment of the base sequence of each nucleic acid fragment included in the sequence information.

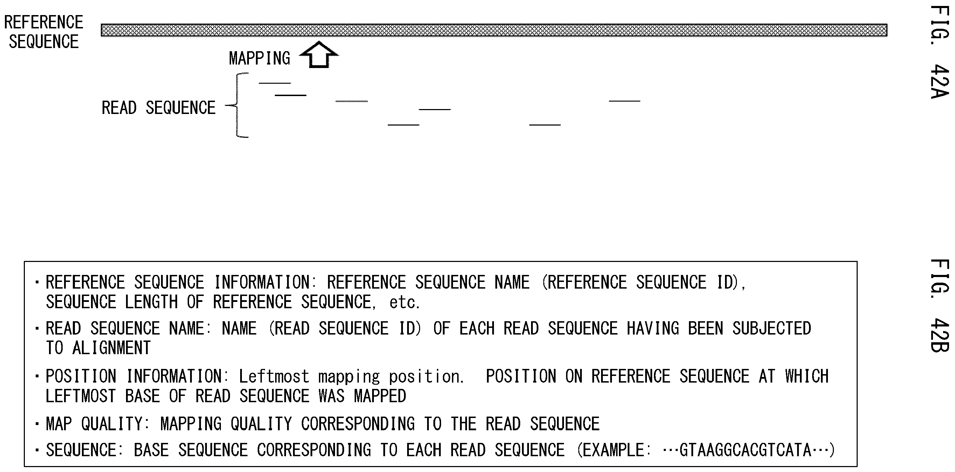

FIG. 42A illustrates alignment performed by the data adjustment unit 113. The data adjustment unit 113 refers to reference sequences stored in the reference sequence database 122, and performs mapping of the read sequence of each nucleic acid fragment, to the reference sequence to be compared with the sequence information, thereby performing alignment. In one aspect, a plurality of kinds of reference sequences that respectively correspond to the genes to be analyzed are stored in the reference sequence database 122.

The data adjustment unit 113 performs alignment for both the sequence information obtained by subjecting an FFPE sample from a lesion site of a subject to the sequencer 2, and the sequence information obtained by subjecting a blood sample of the same subject to the sequencer 2.

FIG. 42B shows one example of a format for a result of alignment performed by the data adjustment unit 113. The format for the alignment result is not limited in particular, and may be any format that can specify the read sequence, the reference sequence, and the mapping position. As shown in FIG. 42B, the format may include reference sequence information, read sequence name, position information, map quality, and sequence.

The reference sequence information indicates the reference sequence name (reference sequence ID), the sequence length of the reference sequence, and the like in the reference sequence database 122. The read sequence name is information that indicates the name (read sequence ID) of each read sequence for which alignment has been performed. The position information indicates the position (leftmost mapping position) on the reference sequence at which the leftmost base of the read sequence has been mapped. The map quality is information that indicates the quality of mapping corresponding to the read sequence. The sequence is information that indicates the base sequence (example: ...GTAAGGCACGTCATA...) corresponding to each read sequence.

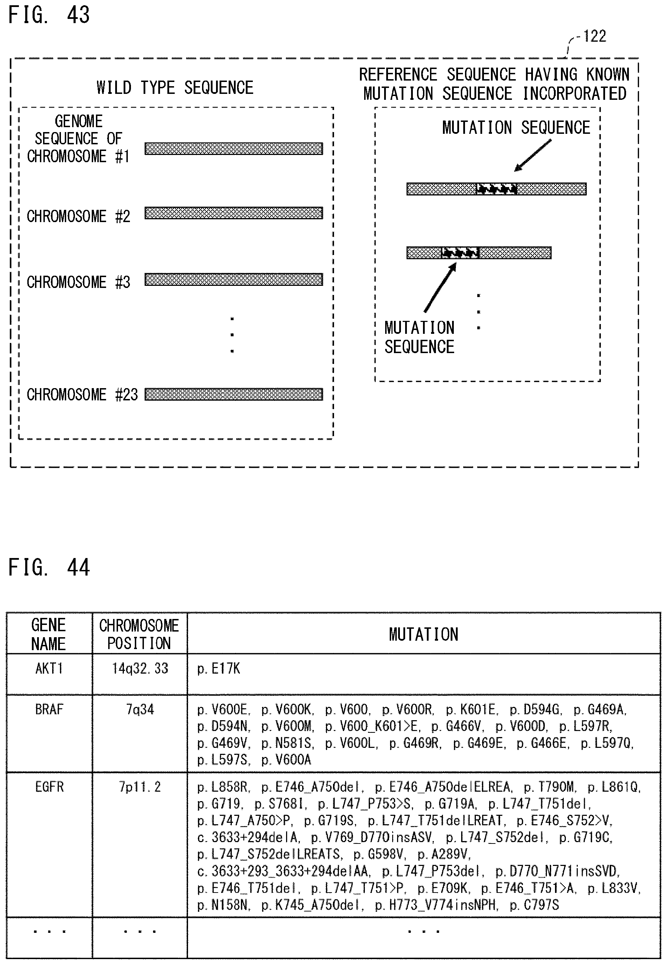

FIG. 43 shows an example of the structure of the reference sequence database 122. As shown in FIG. 43, the reference sequence database 122 stores reference sequences (for example, genome sequences of chromosomes #1 to 23) indicating wild-type sequences, and reference sequences in which known mutations are incorporated in the wild-type sequences.

Further, each reference sequence in the reference sequence database 122 is provided with metadata which indicates gene panel information. For example, the gene panel information provided to each reference sequence can directly or indirectly indicate the gene, to be analyzed, that corresponds to the reference sequence.

In one embodiment, the information selection unit 112 may perform control such that, when the data adjustment unit 113 obtains a reference sequence from the reference sequence database 122, the data adjustment unit 113 refers to the inputted gene panel information and the metadata of each reference sequence, and selects a reference sequence that corresponds to the gene panel information.

For example, in one aspect, the information selection unit 112 may control the data adjustment unit 113 so as to select a reference sequence that corresponds to the gene, to be analyzed, that is specified by the inputted gene panel information. This allows the data adjustment unit 113 to perform mapping only on the reference sequence related to the gene panel having been used, and thus efficiency of the analysis can be improved.

In another embodiment, the information selection unit 112 need not perform the above-described control. In this case, the information selection unit 112 merely controls the mutation identification unit 114 or the report creation unit 115 as described later.

FIG. 44 shows an example of known mutations to be incorporated into reference sequences (that are not wild-type sequences) included in the reference sequence database 122. The known mutations are gene mutations registered in external databases (for example, COSMIC, ClinVar, etc.), and as shown in FIG. 44, the chromosome positions, the gene names, and the mutations are specified. In the example shown in FIG. 44, mutations of amino acid are specified. However, mutations of nucleic acid may be specified. The types of mutations are not limited in particular, and the mutations may be various mutations such as substitution, insertion, and deletion, or the mutation may be a mutation in which a sequence of a part of another chromosome or reverse complement sequence is bound.

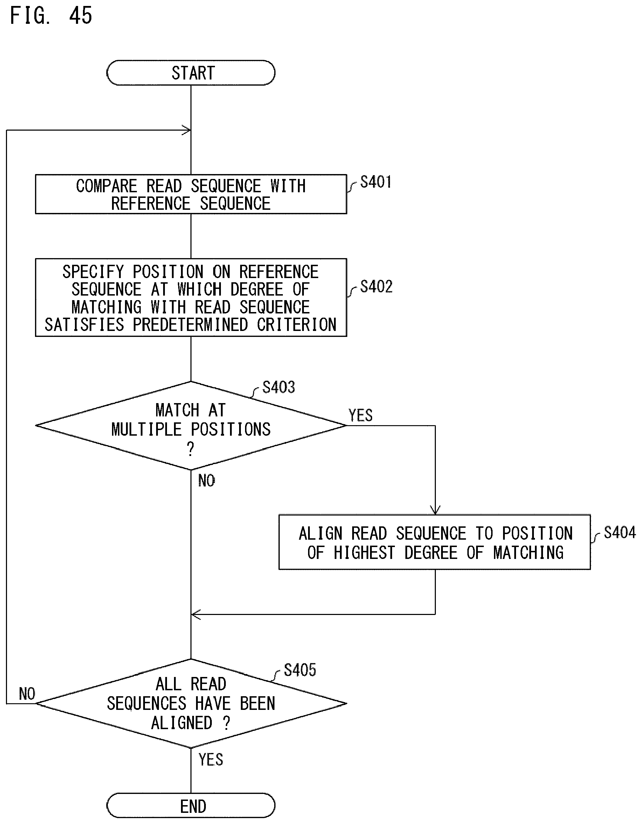

FIG. 45 is a flow chart describing in detail one example of a step of alignment performed in step S12 shown in FIG. 40. In one aspect, the alignment in step S12 shown in FIG. 40 is performed in steps S401 to S405 shown in FIG. 45.

In step S401 shown in FIG. 45, the data adjustment unit 113 selects a read sequence that has not been subjected to alignment from among the read sequences of nucleic acid fragments included in the sequence information obtained by the sequence data reading unit 111, and compares the selected read sequence with a reference sequence obtained from the reference sequence database 122. In step S402, the data adjustment unit 113 specifies a position, on the reference sequence, at which the degree of matching with the read sequence satisfies a predetermined criterion. The degree of matching is a value that indicates how much the obtained sequence information and the reference sequence match each other. Examples of the degree of matching include the number or proportion of bases that match each other.

In one aspect, the data adjustment unit 113 calculates a score that indicates the degree of matching between the read sequence and the reference sequence. The score indicating the degree of matching can be, for example, a percentage identity between two sequences. For example, the data adjustment unit 113 specifies positions at which bases of the read sequence and bases of the reference sequence are the same, obtains the number of the positions, and divides the number of the positions at which the bases are the same, by the number of bases (the number of bases in the comparison window) of the read sequence compared with the reference sequence, to calculate the percentage.

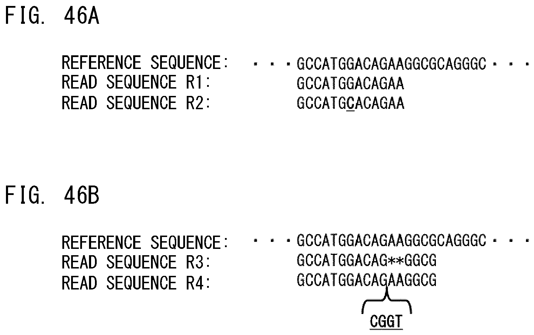

FIG. 46A shows one example of score calculation. In one aspect, at the position shown in FIG. 46A, the score of the degree of matching between the read sequence R1 and the reference sequence is 100% because 13 bases among 13 bases of the read sequence match the bases of the reference sequence. The score of the degree of matching between the read sequence R2 and the reference sequence is 92.3% because 12 bases among 13 bases of the read sequence match the bases of the reference sequence.

In the calculation of the score indicating the degree of matching between a read sequence and a reference sequence, the data adjustment unit 113 may perform calculation such that, when the read sequence includes a predetermined mutation (for example, InDel: Insertion/Deletion) with respect to the reference sequence, a score lower than that calculated in the normal calculation is obtained.

In one aspect, for a read sequence that includes at least one of insertion and deletion with respect to the reference sequence, the data adjustment unit 113 may correct the score by, for example, multiplying the score calculated in the above-described normal calculation, by a weighting factor according to the number of bases that correspond to the insertion/deletion. The weighting factor $W$ may be calculated as, for example, $W=\{1-(1/100)\times$ (the number of bases corresponding to insertion/deletion)$\}$.

FIG. 46B shows another example of score calculation. In one aspect, at the positions shown in FIG. 46B, the score of the degree of matching between the read sequence R3 and the reference sequence is 88% in the normal calculation because 15 bases among 17 bases of the read sequence (the symbol * indicating a deletion is also calculated as one base) match the bases of the reference sequence, and the corrected score is $86\%=88\%\times0.98$. The score of the degree of matching between the read sequence R4 and the reference sequence is 81% in the normal calculation because 17 bases among 21 bases of the read sequence match the bases of the reference sequence, and the corrected score is $77.8\%=81\%\times0.96$.

The data adjustment unit 113 calculates the score of the degree of matching while changing the mapping position of the read sequence with respect to each reference sequence, thereby specifying a position on the reference sequence at which the degree of matching with the read sequence satisfies a predetermined criterion. At this time, an algorithm known in this technical field, such as dynamic programming, the FASTA method, and the BLAST method, may be used.

With reference back to FIG. 45, next, when the degree of matching with the read sequence satisfies the predetermined criterion at a single position on the reference sequence (NO in step S403), the data adjustment unit 113 performs alignment of the read sequence at the position, and when the degree of matching with the read sequence satisfies the predetermined criterion at a plurality of positions on the reference sequence (YES in step S403), the data adjustment unit 113 performs alignment of the read sequence at the position at which the degree of matching is highest (step S404).

When alignment of all the read sequences included in the sequence information obtained by the sequence data reading unit 111 has not been performed (NO in step S405), the data adjustment unit 113 returns to step S401. When alignment of all the read sequences included in the sequence information has been performed (YES in step S405), the data adjustment unit 113 completes the process of step S12.

<Mutation identification unit 114>

With reference back to FIG. 40, subsequently, in step S13, the mutation identification unit 114 compares the sequence (alignment sequence) of the reference sequence with which the read sequence obtained from the sample collected from the lesion site of the subject has been aligned, with the sequence of the reference sequence with which the read sequence obtained from a blood sample of the subject has been aligned.

In step S14 shown in FIG. 40, a difference between the alignment sequences is extracted as mutation (mutation extraction process). For example, if, at the same position of the same gene to be analyzed, the alignment sequence derived from the blood sample is ATCGA and the alignment sequence derived from the tumor tissue is ATCCA, the mutation identification unit 114 extracts the difference of G and C as a mutation.

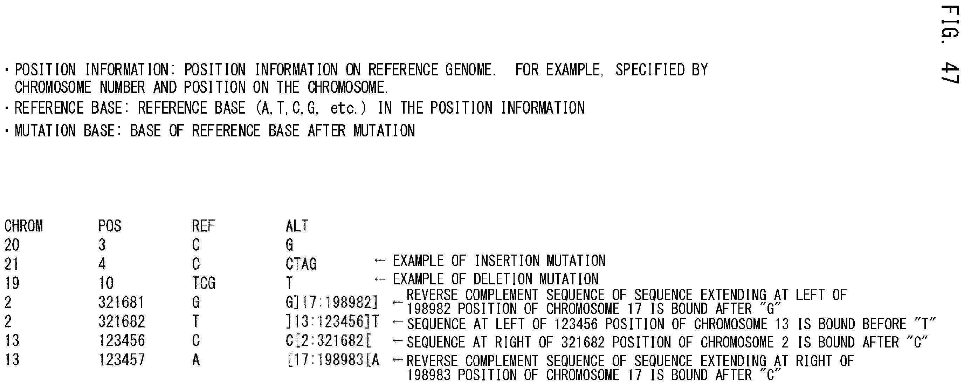

In one aspect, the mutation identification unit 114 generates a result file on the basis of the extracted gene mutation. FIG. 47 shows one example of a format of the result file generated by the mutation identification unit 114. The format can be, for example, based on Variant Call Format (VCF).

As shown in FIG. 47, the result file contains position information, reference base, and mutation base for each extracted gene mutation. The position information indicates the position on the reference genome, and includes the chromosome number and the position on the chromosome, for example. The reference base indicates the reference base (such as A, T, C, G) at the position indicated by the position information. The mutation base indicates the base of the reference base which is present after the mutation. The reference base is the base on the alignment sequence derived from the blood sample. The mutation base is the base on the alignment sequence derived from the tumor tissue.

In FIG. 47, the mutation in which the reference base is C and the mutation base is G is an example of substitution mutation, the mutation in which the reference base is C and the mutation base is CTAG is an example of insertion mutation, and the mutation in which the reference base is TCG and the mutation base is T is an example of deletion mutation. The mutation in which the mutation base is G]17:198982], ]13:123456]T, C[2:321682[, or [17:198983[A is an example of mutation in which a sequence of a part of another chromosome or reverse complement sequence is bound.

With reference back to FIG. 40, subsequently, in step S15, the mutation identification unit 114 searches the mutation database 123. In step S16, the mutation identification unit 114 refers to mutation information in the mutation database 123 and provides annotation to the mutation included in the result file, to identify the mutation.

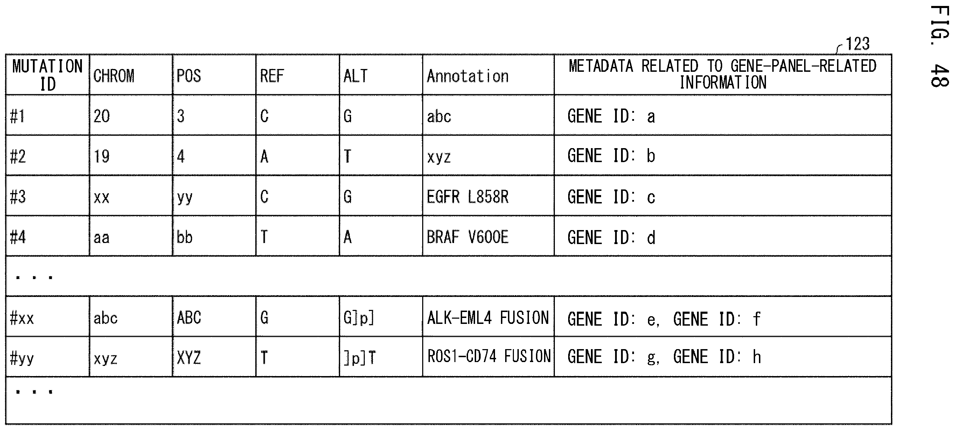

FIG. 48 shows one example of the structure of the mutation database 123. The mutation database 123 is constructed on the basis of an external database such as COSMIC or ClinVar, for example. In one aspect, metadata related to gene panel information is provided to each piece of mutation information in the database. In the example shown in FIG. 48, a gene ID of a gene to be analyzed is provided as metadata to each piece of mutation information in the database.

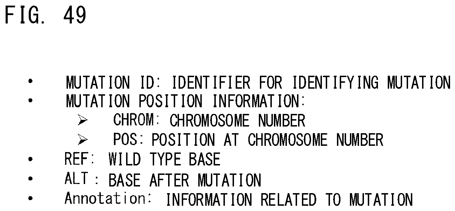

FIG. 49 shows in detail an example of the structure of mutation information in the mutation database 123. As shown in FIG. 49, in one aspect, the mutation information included in the mutation database 123 may include mutation ID, mutation position information (for example, "CHROM" and "POS"), "REF", "ALT", and "Annotation". The mutation ID is an identifier for identifying the mutation.

In the mutation position information, "CHROM" indicates the chromosome number, and "POS" indicates the position at the chromosome number. "REF" indicates the base in the wild type, and "ALT" indicates the base that is present after the mutation. "Annotation" indicates information related to mutation. "Annotation" may be information indicating mutation of amino acid such as "EGFR C2573G" or "EGFR L858R", for example. For example, "EGFR C2573G" indicates mutation in which cysteine at the 2573-th residue of protein "EGFR" is substituted with glycine.

As in the above-described example, "Annotation" of the mutation information may be information for converting mutation based on the base information to mutation based on amino acid information. In this case, the mutation identification unit 114 can convert the mutation based on the base information to the mutation based on the amino acid information, according to the information of "Annotation" which has been referred to.

The mutation identification unit 114 searches the mutation database 123 by using, as a key, information (for example, base information corresponding to mutation position information and mutation) that specifies the mutation included in the result file. For example, the mutation identification unit 114 may search the mutation database 123 by using, as a key, information of any of "CHROM", "POS", "REF", and "ALT". When the gene mutation extracted by comparison between the alignment sequence derived from the blood sample and the alignment sequence derived from the lesion site is already registered in the mutation database 123, the mutation identification unit 114 identifies the mutation as a mutation existing in the sample, and provides annotation (for example, "EGFR L858R", "BRAF V600E", or the like) to the mutation included in the result file.

In one embodiment, before the mutation identification unit 114 searches the mutation database 123 on the basis of the result file, the information selection unit 112 may cause mutations that do not correspond to the gene panel information having been inputted to the mutation identification unit 114, to be masked in (excluded from) the result file.

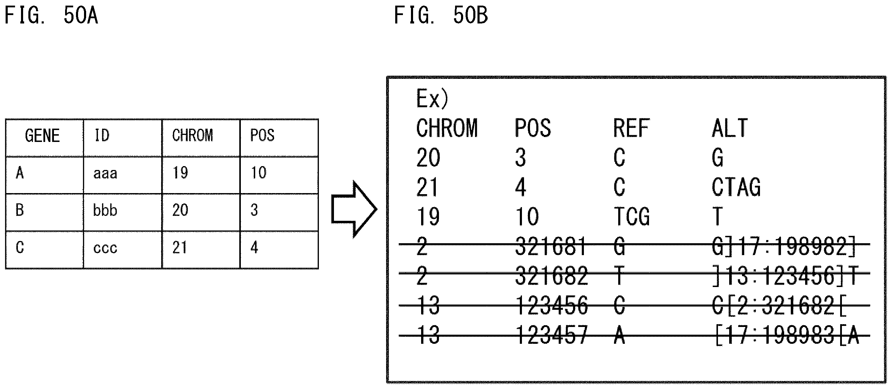

For example, in one aspect, the mutation identification unit 114 which has been notified of the gene panel information from the information selection unit 112 may refer to a table indicating the correspondence relationship between each gene to be analyzed and position information (for example, "CHROM" and "POS") as shown in FIG. 50A, may specify the position of mutation corresponding to the gene, to be analyzed, specified by the notified gene panel information, and may mask (exclude) mutations at the other positions in the result file as shown in FIG. 50B. Accordingly, the mutation identification unit 114 only has to provide annotation to the mutations, in the result file, that are related to the gene panel having been used, and thus, the mutation identifying and specifying efficiency can be improved.

(Drug search unit 118)

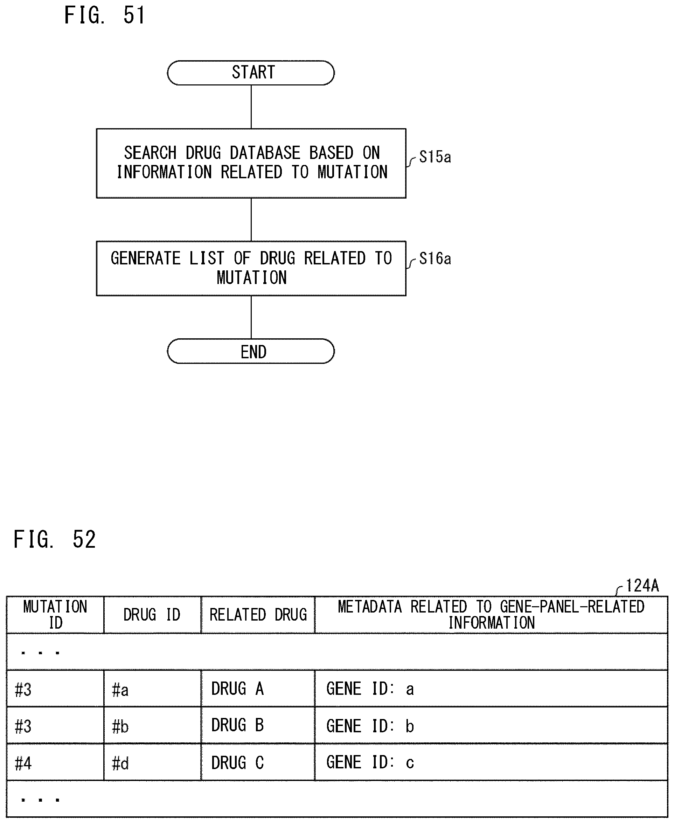

The flow of a process in which the drug search unit 118 generates a list including information related to drugs is described with reference to FIG. 51. FIG. 51 is a flow chart showing one example of the process in which the drug search unit 118 generates a list of drugs related to mutations.

The drug search unit 118 searches the drug database 124 by using, as a key, the mutation ID provided to each gene mutation identified by the mutation identification unit 114 (step S15a). On the basis of the search result, the drug search unit 118 generates a list including information regarding drugs related to the mutations (step S16a). The generated list is incorporated into the report created by the report creation unit 115.

(Drug database 124)

Data 124A stored in the drug database 124 and used when the drug search unit 118 searches the drug database 124 and generates a drug list is described with reference to FIG. 52. FIG. 52 shows an example of a data structure of the drug database 124.

As shown in FIG. 52, a mutation ID provided to each mutation, a related drug name, and a drug ID provided to each drug are stored in association with one another in the drug database 124. With reference to data 124A of FIG. 52, "drug A" and "drug B" are associated with mutation ID "#3". Similar to this, each mutation ID may have a plurality of related drugs associated therewith.

Each mutation ID in the drug database 124 may be provided with "metadata related to gene-panel-related information", which is metadata related to gene panel information. The drug search unit 118 refers to the "metadata related to gene-panel-related information" in accordance with an instruction from the information selection unit 112.

The drug search unit 118 changes the range in which the drug database 124 is searched, to a range indicated by the metadata. Accordingly, in accordance with the "metadata related to gene-panel-related information" provided to each drug and the inputted gene panel information, the drug search unit 118 can narrow the drugs that should be referred to in the drug database, and can generate a list that includes information regarding drugs according to the gene panel information.

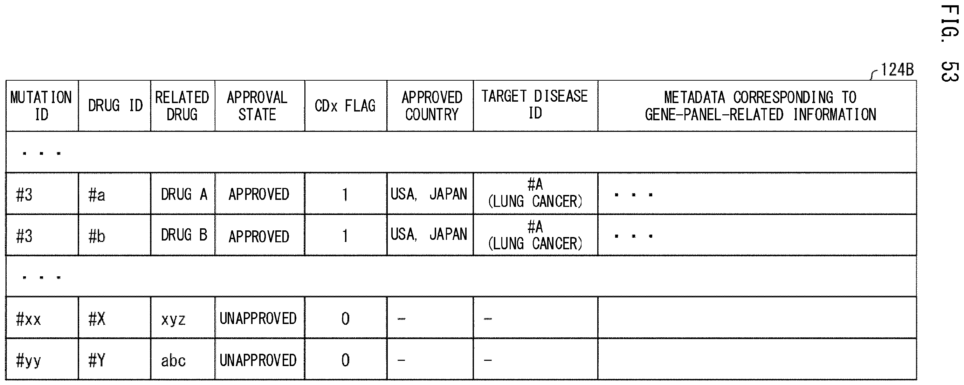

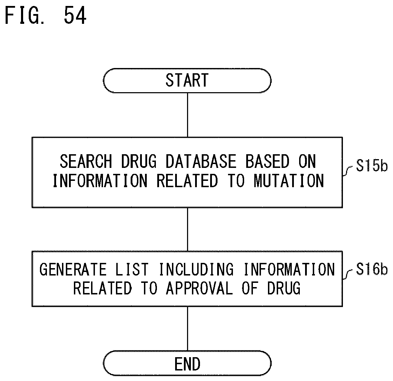

The drug search unit 118 may search the drug database 124 having a data structure shown in FIG. 53, and generate a list that includes another type of information regarding drugs related to mutations. This is described with reference to FIG. 54. FIG. 54 is a flow chart showing one example of a process in which the drug search unit 118 generates a list that includes information regarding drugs related to mutations.

The drug search unit 118 searches the drug database 124 which stores data 124B shown in FIG. 53, as to whether or not the related drug has been approved by an authority (FDA, PMDA, or the like). Specifically, for example, by using information related to a mutation such as "mutation ID" as a key, the drug search unit 118 searches for "approval state" which indicates whether the related drug corresponding to the mutation has been approved by the authority, and "approved country" which indicates which country's authority has provided its approval (step S15b).

On the basis of the search result, the drug search unit 118 generates a list that includes the mutation, the related drug that corresponds to the mutation, and information regarding the approval of the related drug (step S16b).

The drug search unit 118 may search the drug database 124 having a data structure shown in FIG. 53 and generate a list that includes still another type of information regarding drugs related to mutations. This is described with reference to FIG. 54. FIG. 54 is a flow chart showing one example of a process in which, on the basis of information obtained by searching the drug database 124, the drug search unit 118 determines the presence or absence of a drug having a possibility of off-label use and generates a list that includes the determination result.

The drug search unit 118 searches the drug database 124 which stores the data 124B shown in FIG. 53, as to whether or not the related drug has been approved by an authority (FDA, PMDA, or the like) (step S15b). When the searched drug has not been approved, the drug search unit 118 associates the drug as an unapproved drug, with the mutation, and creates a report of drugs related to the mutations (step S16b).

When the searched drug has been approved, the drug search unit 118 determines whether or not the disease (disease name or disease ID) of the subject from whom the sample has been collected, and the disease (for example, disease name or disease ID of the "target disease" shown in FIG. 53) that corresponds to the related drug searched from the drug database 124 match each other.

When the disease of the subject and the "target disease" match each other, the drug search unit 118 associates the drug of the search result, as an approved drug, with the mutation, and generates a list that includes the mutation, the related drug corresponding to the mutation, information regarding the approval of the related drug, and the like (step S16b).

Meanwhile, when the disease of the subject and the "target disease" are different from each other, the drug search unit 118 determines that the searched related drug is a drug having a possibility of off-label use, associates the determination result with the mutation, and generates a list that includes the mutation, the related drug corresponding to the mutation, information regarding the approval of the related drug, and the like (step S16b).

The identification information (for example, disease name, disease ID, or the like) for identifying the disease of the subject can be inputted through the input unit 17 by an operator or the like when performing gene analysis, for example. In this case, the information selection unit 112 obtains information related to the disease corresponding to the sample inputted by the operator, and identifies the disease. Alternatively, as shown in FIG. 7, a label L1 indicating a subject ID, a sample ID, and the like is attached to each container PI which stores a sample, and a recording means such as a bar code L11 indicated on the label L1 is read, whereby the disease ID which is identification information of the disease of the subject may be obtained. Alternatively, as shown in FIG. 8, a label L2 indicating a subject ID, a sample ID, and the like is attached to each container P1 which stores a sample, and a recording means such as an RFID tag L21 attached to the label L2 is read, whereby the disease ID which is identification information of the disease of the subject may be obtained.

Alternatively, in the test institution 120, a sample ID and a subject ID are managed so as to be associated with a disease ID, and the information selection unit 112 may obtain a disease ID that corresponds to a sample, on the basis of the subject ID or the sample ID. For example, the information selection unit 112 may obtain, via a communication line, a disease ID associated with a subject ID (or sample ID) obtained by reading a recording means of a label attached to each container which stores a sample. The disease ID may be included in a header region of sequence information shown in FIG. 41, and the information selection unit 112 may obtain the disease ID.

As in the data 124B shown in FIG. 53, the drug database 124 may have a "CDx flag" which indicates whether or not each drug in the database is a drug related to CDx in terms of the relationship of a predetermined gene mutation and a predetermined disease. When the drug search unit 118 has retrieved a drug whose CDx flag is "1" ("drug A" and "drug B" in FIG. 53), the drug search unit 118 may generate a list that includes auxiliary information that indicates the detection result of the predetermined gene mutation in the predetermined disease is applicable to the CDx for the retrieved drug. In accordance with the fact that a predetermined gene mutation has been detected in the sample collected from the subject having a predetermined disease (for example, cancer), the drug search unit 118 may create a list that includes information that the detected gene mutation and the drug corresponding to the gene mutation are related to CDx, and auxiliary information related to the efficacy of the drug.

In this manner, the drug search unit 118 searches the drug database 124 in which gene mutations, target diseases, and drugs are stored in association with one another, and checks a detected gene mutation against a disease specified by the information selection unit 112, thereby being able to create a list according to the disease corresponding to the sample. The report creation unit 115 creates a report by use of the list created by the drug search unit 118.

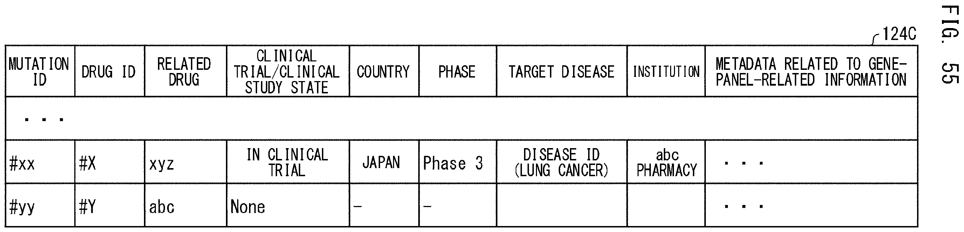

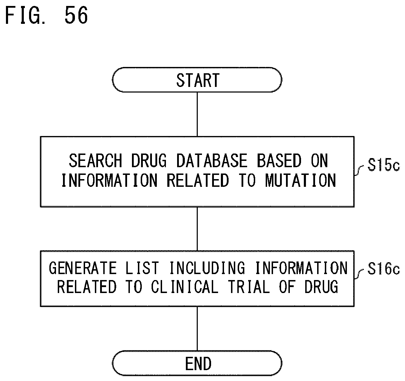

The drug search unit 118 may search the drug database 124 having a data structure shown in FIG. 55 and generate a list that includes information regarding clinical trials of drugs related to mutations. This is described with reference to FIG. 56. FIG. 56 is a flow chart showing one example of a process in which the drug search unit 118 generates a list that includes information regarding clinical trials of drugs.

The drug search unit 118 searches the drug database 124 which stores data 124C shown in FIG. 55, for information such as the progress of a clinical trial of a related drug. Specifically, by using a mutation ID or the like as a key, the drug search unit 118 searches for information regarding a clinical trial with respect to the mutation, such as, for example, "clinical trial/clinical study state", "country", and "institution" in which the clinical trial is being performed, as shown in FIG. 55 (step S15c in FIG. 56). On the basis of the search result, the drug search unit 118 generates a list that includes the mutation, the related drug corresponding to the mutation, and information regarding the clinical trial of the related drug (step S16c in FIG. 56).

The data 124A shown in FIG. 52, the data 124B shown in FIG. 53, and the data 124C shown in FIG. 55 may be integrated together and stored in the drug database 124, or may be discretely stored in a plurality of databases including the drug database 124.

(Report creation unit 115)

The report creation unit 115 creates a report (corresponding to step Sill in FIG. 6) on the basis of the information outputted by the mutation identification unit 114, the gene panel information provided from the information selection unit 112, and the drug list generated by the drug search unit 118. The information put on the created report includes gene panel information, information related to the identified gene mutation, and information of the drug related to the detected gene mutation. When the test institution 120 has concluded a contract of a "CDx usage" plan, the report creation unit 115 can create a report that includes auxiliary information related to the efficacy of the drug applicable to CDx, on the subject having a predetermined disease.

On the basis of the gene panel information from the information selection unit 112, the report creation unit 115 may select the target to be put on the report, and may delete, from the report, information that has not been selected. Alternatively, the information selection unit 112 may control the report creation unit 115 such that information related to genes that correspond to the gene panel information inputted through the input unit 17 is selected as the target to be put on the report and information that has not been selected is deleted from the report.

<Example of report>

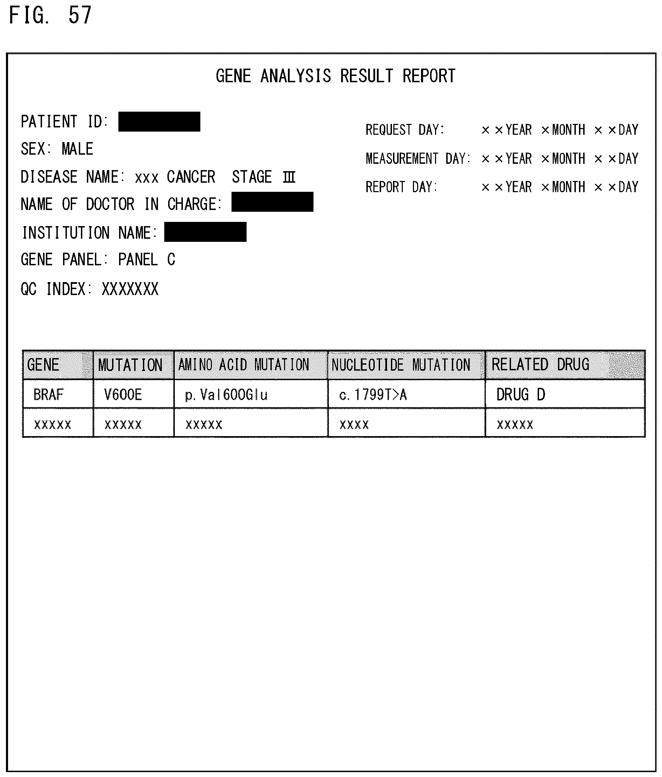

Next, a specific example of the report created by the report creation unit 115 is described with reference to FIG. 57. FIG. 57 shows one example of the report to be created.

In the upper left part of the example of the report shown in FIG. 57, "patient ID" indicating the subject ID, "sex of patient", "disease name of patient", "name of doctor in charge" which is the name of the doctor who is in charge of the subject in the medical institution 210, and "institution name" indicating the medical institution name are described.

Below these items, the gene panel name such as "Panel A" is also indicated as the gene panel information. Further, the quality evaluation index "QC index" obtained from the process using the quality control sample, the result of analysis thereof, and the like is also outputted in the report

(Output unit 13)

The report created by the report creation unit 115 may be transmitted in the form of data, from the output unit 13 to the communication terminal 5 (see FIG. 5) installed in the medical institution 210, as the analysis result of the sequence information (corresponding to step S112 in FIG. 6). Alternatively, the report may be transmitted to a printer (not shown) connected to the information processing apparatus 1, to be printed by the printer, and then, may be sent in the form of a paper medium, from the test institution 120 to the medical institution 210.

(Target to be analyzed by information processing apparatus 1)

As shown in FIG. 38, when sequence information includes an index sequence, the information processing apparatus 1 performs analysis (step S52), and when the sequence information is not associated with any index sequence, the information processing apparatus 1 does not perform at least a part of the analysis that is to be performed on the sequence information associated with an index sequence (step S53).

For example, in a case where PhiX DNA has been used as the non-subject-derived nucleic acid in preparation of a measurement sample, the measurement sample is not a target for the sorting performed for each index sequence in step S10 shown in FIG. 40, because PhiX DNA does not have an index sequence added thereto. Thus, the process of step S10 and the subsequent steps are not performed.

For example, in a case where a library prepared from a subject-derived sample having been analyzed is used in preparation of a measurement sample, the library is not a target to be analyzed, and thus, the process of step S11 and the subsequent steps in FIG. 40 are not performed.

For example, in a case where a quality control sample has been used in preparation of a measurement sample, there is no need to identify mutation, and thus, the process of step S15 and the subsequent steps in FIG. 40 are not performed.

That is, the information processing apparatus 1 selectively performs the process of step S10 and the subsequent steps shown in FIG. 40 with respect to sequence information of nucleic acid of a library that has an index sequence added thereto, in the entire sequence information obtained from the sequencer 2. However, the information processing apparatus 1 does not perform at least a part of the process of step S10 and the subsequent steps, with respect to sequence information of nucleic acid that does not have an index sequence added thereto.

(Quality evaluation index)

Here, a quality evaluation index for evaluating the quality of sequence information is described. Examples of the quality evaluation index include the following.

- Index (i): quality evaluation index indicating the quality of reading of base information performed by the sequencer 2.
- Index (ii): quality evaluation index indicating the proportion of bases read by the sequencer 2, to bases included in a plurality of genes to be analyzed.
- Index (iii): quality evaluation index indicating the depth of sequence information.
- Index (iv): quality evaluation index indicating variation in the depth of sequence information.

- Index (v): quality evaluation index indicating whether or not all the mutations in standard genes included in the quality control sample have been detected.

Index (i) can include

index (i-1): quality score, and
index (i-2): cluster concentration.

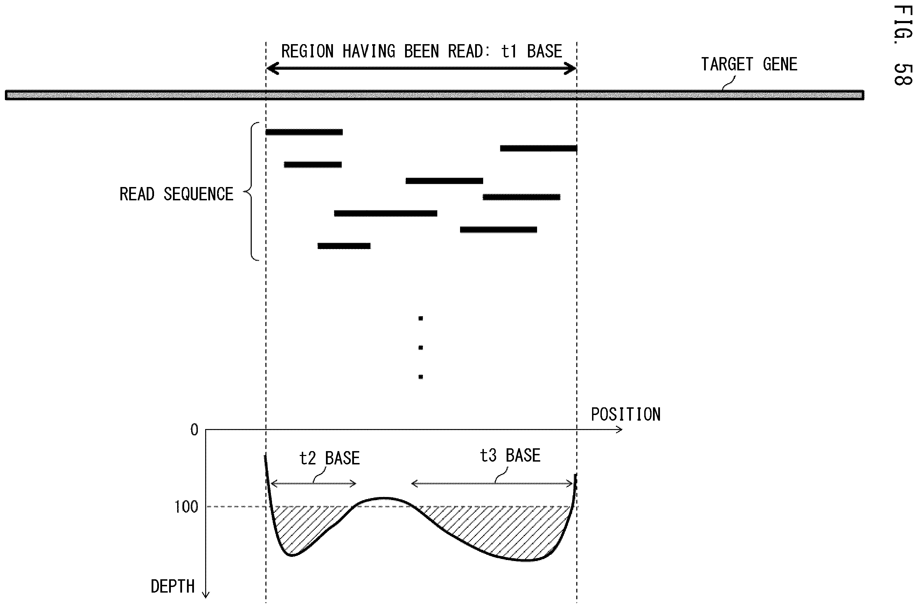

The above-described quality evaluation indexes are described with reference to FIG. 58.

Index (i-1): quality score

The quality score is an index indicating the accuracy of each base in the gene sequence read by the sequencer 2.

For example, when the sequence information is outputted as a FASTQ file from the sequencer 2, the quality score is also included in the sequence information (see FIG. 41). Since the quality score has already been described in detail, the description thereof is omitted here.

Index (i-2): cluster concentration

The sequencer 2 locally amplifies and immobilizes a large number of single-stranded DNA fragments on a flow cell to form a cluster (see "9" in FIG. 36). An image of the cluster group on the flow cell is taken by using a fluorescence microscope, and fluorescent colors (that is, fluorescences having different wavelengths) respectively corresponding to A, C, G, T are detected to read the sequence. The cluster density is an index indicating a degree to which the clusters of each gene formed on the flow cell are close to each other when the sequencing is performed.

For example, in a case where the cluster density becomes excessively high, and the clusters are excessively close to each other or overlap each other, the contrast of the taken image of the flow cell, i.e., the S/N ratio, is lowered, whereby focusing by the fluorescence microscope becomes difficult. Therefore, fluorescence cannot be accurately detected. As a result, the sequence cannot be accurately read.

Index (ii): quality evaluation index indicating the proportion of bases in a target region read by the sequencer 2, to bases read by the sequencer 2.

This index indicates how many bases in the target region have been read, among bases (also including bases other than those in the target region) read by the sequencer 2. This index can be calculated as a ratio between the total number of bases in the target region and the total number of bases having been read.

Index (iii): quality evaluation index indicating the depth of sequence information.

This index is an index based on the total number of pieces of the sequence information obtained by reading the bases included in a gene to be analyzed. This index can be calculated as a ratio between the total number of bases having depths greater than or equal to a predetermined value among the bases having been read, and the total number of bases having been read.

The depth means the total number of pieces of sequence information having been read for one base.

FIG. 58 shows a graph indicating the depth for each base having been read in a case where T base represents the entire length of the gene to be analyzed and t1 base represents the base in the read region. In this graph, the horizontal axis represents the position of each base, and the vertical axis represents the depth of each base. In the example shown in FIG. 58, in the t1 base in the region having been read, the total number of bases in the region in which the depth is greater than or equal to a predetermined value (for example, 100) is (t2+t3) bases. In this case, index (iii) is generated as a value of (t2+t3)/t1.

Index (iv): quality evaluation index indicating variation in the depth of sequence information.

This index is an index indicating the uniformity of the depth. When the number of pieces of the sequence information having been read in a certain portion in the region having been read is extremely great, uniformity of the depth is low. When the sequence information is relatively uniform over the region having been read, the uniformity of the depth is high. The uniformity of the depth is not limited thereto. For example, the uniformity can be expressed as a number by using the interquartile range (IQR). The greater the IQR is, the lower the uniformity is. The lower the IQR is, the higher the uniformity is.

Index (v): quality evaluation index indicating whether or not all the mutations in standard genes included in the quality control sample have been detected.

This index is an index indicating that the mutations in standard genes included in the quality control sample have been detected and accurately identified. For example, mutations (see the column of "Variant") in standard genes included in the quality control sample A shown in FIG. 25A and the quality control sample B shown in FIG. 25B are known mutations. The index for evaluating whether or not the position of the mutation, the type of the mutation, and the like have been accurately identified is used as the quality evaluation index.

The information processing apparatus 1 is a computer which performs commands of a program which is software that realizes each function. This computer includes one or more processors, for example, and also includes a computer-readable storage medium having the program stored therein. In the computer, the processor reads the program from the storage medium and performs the program, whereby the object of the present disclosure is achieved. As the processor, a CPU (Central Processing Unit) can be used, for example. As the storage medium, a "non-transitory and tangible medium", such as a ROM (Read Only Memory), tape, disk, card, semiconductor memory, or programmable logical circuit, can be used. The computer may further include a RAM (Random Access Memory) or the like onto which the program is developed. The program may be supplied to the computer via a desired transmission medium (communication network, broadcast wave, or the like) that can transmit the program. One aspect of the present disclosure can also be realized in the form of a data signal which is realized by electronic transmission of the program and which is embedded in a carrier wave.

The present disclosure is not limited to the above-described embodiments, and various modifications can be made without departing from the scope of the claims. Embodiments obtained by combining as appropriate technological means disclosed in different embodiments are also included in the technological scope of the present disclosure.

## Claims

1. An analysis method comprising:

   obtaining sequence information of nucleic acid contained in a measurement sample prepared by mixing a sample that contains subject-derived nucleic acid and a sample that contains non-subject-derived nucleic acid such that the measurement sample contains a previously determined amount of nucleic acid; and
   performing analysis on the obtained sequence information, wherein
   the performing of the analysis includes
   performing analysis on sequence information of the subject-derived nucleic acid in the obtained sequence information, and
   not performing, on sequence information of the non-subject-derived nucleic acid in the obtained sequence information, at least a part of the analysis to be performed on the sequence information of the subject-derived nucleic acid.

2. The analysis method of claim 1, wherein
   the sequence information of the subject-derived nucleic acid includes an index sequence, and
   the performing of the analysis includes performing analysis on sequence information that includes the index sequence, in the sequence information of the nucleic acid contained in the measurement sample.

3. The analysis method of claim 1 or 2, wherein
   the sequence information of the subject-derived nucleic acid includes a plurality of pieces of sequence information of nucleic acid derived from a plurality of subjects, and
   sequence information of nucleic acid derived from a different subject includes a different index sequence.

4. The analysis method of any one of claims 1 to 3, wherein
   the sequence information of the non-subject-derived nucleic acid does not include an index sequence.

**5.** The analysis method of any one of claims 1 to 4, wherein
the performing of the analysis includes performing analysis on sequence information that includes an index sequence in the sequence information of the nucleic acid contained in the measurement sample, and
at least a part of the analysis to be performed on the sequence information that includes the index sequence is not performed on sequence information that does not include the index sequence.

**6.** The analysis method of any one of claims 1 to 5, wherein
the sequence information of the nucleic acid contained in the measurement sample includes sequence information that includes a first index sequence and sequence information that includes a second index sequence different from the first index sequence, and
the performing of the analysis includes performing analysis on the sequence information that includes the first index sequence and not performing at least a part of the analysis to be performed on the sequence information that includes the first index sequence on the sequence information that includes the second index sequence.

**7.** The analysis method of any one of claims 1 to 6, wherein
the analysis includes obtaining information related to a gene of the subject, on the basis of the sequence information of the subject-derived nucleic acid.

**8.** The analysis method of claim 7, wherein
the information related to the gene of the subject includes a gene name corresponding to the sequence information and mutation information of the gene.

**9.** The analysis method of any one of claims 1 to 8, wherein

the measurement sample includes a quality control sample for evaluating quality of sequence information, and
the analysis method further comprises performing a process for obtaining information related to quality of the measurement sample from sequence information of the quality control sample.

**10.** The analysis method of claim 9, wherein

nucleic acid of the quality control sample is identical to the non-subject-derived nucleic acid, and
the analysis method further comprises performing a process for obtaining information related to quality, on at least a part of the sequence information of the non-subject-derived nucleic acid in the sequence information of the nucleic acid contained in the measurement sample.

**11.** The analysis method of any one of claims 1 to 10, wherein
the analysis includes an alignment process for mapping the obtained sequence information on a reference sequence, and
the performing of the analysis includes not performing the alignment process on the sequence information of the non-subject-derived nucleic acid.

**12.** The analysis method of any one of claims 1 to 11, wherein
the analysis includes a mutation extraction process for extracting a mutation of nucleic acid, and
the performing of the analysis includes not performing the mutation extraction process on the sequence information of the non-subject-derived nucleic acid.

**13.** An information processing apparatus comprising a controller programmed to:

obtain sequence information of nucleic acid of a measurement sample prepared by mixing a sample that contains subject-derived nucleic acid and a sample that contains non-subject-derived nucleic acid such that the measurement sample contains a previously determined amount of nucleic acid;
perform analysis on sequence information of the subject-derived nucleic acid in the obtained sequence information; and
not to perform, on sequence information of the non-subject-derived nucleic acid in the obtained sequence information, at least a part of the analysis to be performed on the sequence information of the subject-derived nucleic acid.

**14.** A gene analysis system comprising:

a sequencer configured to read sequence information of nucleic acid of a measurement sample prepared by mixing a sample that contains subject-derived nucleic acid and a sample that contains non-subject-derived nucleic acid such that the measurement sample contains a previously determined amount of nucleic acid; and
an information processing apparatus configured to perform analysis on the sequence information obtained by the sequencer, wherein
the information processing apparatus
performs analysis on sequence information of the subject-derived nucleic acid in the obtained sequence information, and

does not perform, on sequence information of the non-subject-derived nucleic acid in the obtained sequence information, at least a part of the analysis to be performed on the sequence information of the subject-derived nucleic acid.

**15.** A non-transitory storage medium that stores computer program executable by a computer:

to obtain sequence information of nucleic acid of a measurement sample prepared by mixing a sample that contains subject-derived nucleic acid and a sample that contains non-subject-derived nucleic acid such that the measurement sample contains a previously determined amount of nucleic acid;
to perform analysis on sequence information of the subject-derived nucleic acid in the obtained sequence information; and
not to perform, on sequence information of the non-subject-derived nucleic acid in the obtained sequence information, at least a part of the analysis to be performed on the sequence information of the subject-derived nucleic acid.

**16.** An analysis method comprising:

obtaining sequence information of nucleic acid contained in a measurement sample prepared by mixing a sample that contains subject-derived nucleic acid and a sample that contains non-subject-derived nucleic acid such that the measurement sample contains a previously determined amount of nucleic acid;
selecting sequence information of the subject-derived nucleic acid among the obtained sequence information; and
performing analysis on the selected sequence information.

FIG. 1

```
                    ┌─────────────────┐
                    │      START      │
                    └─────────────────┘
                             │
  ┌──────────────────────────────────────────────────────┐
  │ OBTAIN SEQUENCE INFORMATION OF NUCLEIC ACID CONTAINED │  ⌐ S1
  │ IN MEASUREMENT SAMPLE ADJUSTED SO AS TO CONTAIN       │
  │ PREVIOUSLY DETERMINED AMOUNT OF NUCLEIC ACID          │
  └──────────────────────────────────────────────────────┘
                             │
  ┌──────────────────────────────────────────────────────┐
  │ OUTPUT SEQUENCE INFORMATION IN WHICH DATA AMOUNT OF   │  ⌐ S2
  │ SEQUENCE INFORMATION PER SAMPLE IS PREDETERMINED      │
  │ AMOUNT, IRRESPECTIVE OF THE NUMBER OF SAMPLES         │
  │ CONTAINING SUBJECT-DERIVED NUCLEIC ACID HAVING BEEN   │
  │ USED IN PREPARING MEASUREMENT SAMPLE                  │
  └──────────────────────────────────────────────────────┘
                             │
  ┌──────────────────────────────────────────────────────┐
  │             ANALYZE BASE SEQUENCE DATA                │  ⌐ S3
  └──────────────────────────────────────────────────────┘
                             │
                    ┌─────────────────┐
                    │       END       │
                    └─────────────────┘
```

FIG. 2

```
┌─────────────────────────┐
│     INFORMATION         │  ┌ 1
│  PROCESSING APPARATUS   │
└─────────────────────────┘
            ▲
            │
   SEQUENCE INFORMATION
     TO BE ANALYZED
            │
┌─────────────────────────┐  ┌ 2a
│    AUXILIARY APPARATUS   │
│                          │
│           ┌ 21a          │
│   ┌──────────────────┐   │
│   │  STORAGE DEVICE  │   │
│   └──────────────────┘   │
└─────────────────────────┘
┌─────────────────────────────┐  ┌ 2
│        SEQUENCER            │
└─────────────────────────────┘
```

FIG. 3

```
┌─────────────────────────┐
│     INFORMATION         │  ┌ 1
│  PROCESSING APPARATUS   │
│          ┌ 12           │
│   ┌──────────────────┐  │
│   │   STORAGE UNIT   │  │
│   └──────────────────┘  │
└─────────────────────────┘
            ▲
            │
        SEQUENCE
      INFORMATION
            │
┌─────────────────────────────┐  ┌ 2
│        SEQUENCER            │
└─────────────────────────────┘
```

FIG. 4

```
                    ┌─────────────┐
                    │    START    │
                    └──────┬──────┘
                           │
                           │
                    ╱──────────────╲          S51
                  ╱   SEQUENCE       ╲      ╱
                ╱   INFORMATION OF     ╲   NO
               ╱   SUBJECT-DERIVED      ╲──────────────┐
                ╲    NUCLEIC ACID      ╱               │
                  ╲        ?         ╱                 │
                    ╲──────────────╱                   │
                           │                           │
                          YES                          │
                           │                           │
              ┌────────────────────────┐ S52   ┌───────────────────────┐ S53
              │        ANALYZE         │╱       │     DO NOT ANALYZE    │╱
              └───────────┬────────────┘        └───────────┬──────────┘
                          │                                 │
                          │←────────────────────────────────┘
                          │
                   ┌─────────────┐
                   │     END     │
                   └─────────────┘
```

FIG. 5

EP 3 627 513 A1

FIG. 6

## MEDICAL INSTITUTION ~210

COMMUNICATION TERMINAL 5

S105 — TRANSMIT ANALYSIS REQUEST

S113 — RECEIVE REPORT

## TEST INSTITUTION ~120

SEQUENCER 2    INFORMATION PROCESSING APPARATUS 1

ANALYSIS REQUEST (GENE PANEL INFORMATION), SAMPLE ID

SAMPLE, SAMPLE ID ⟹

S101 — FILE APPLICATION FOR USE OF GENE ANALYSIS SYSTEM

S104 — RECEIVE VARIOUS SERVICES

S106 — RECEIVE ANALYSIS REQUEST

SEQUENCE INFORMATION, QUALITY EVALUATION INDEX

S107 — RECEIVE INPUT OF GENE PANEL INFORMATION

S108 — SEQUENCING

S109 — ANALYZE GENE SEQUENCE

(QUALITY SCORE, CLUSTER CONCENTRATION)

S110 — GENERATE QUALITY EVALUATION INDEX BASED ON ANALYSIS RESULT OF QUALITY CONTROL SAMPLE

SAMPLE ID, REPORT (ANALYSIS RESULT, AUXILIARY INFORMATION)

S111 — CREATE REPORT

S112 — TRANSMIT REPORT

CHARGE ANALYSIS FEE ⟸

S114 — MAKE NOTIFICATION OF GENE PANEL INFORMATION OF GENE PANEL USED IN ANALYSIS, INFORMATION RELATED TO ANALYZED GENE, ANALYSIS RECORD, AND QUALITY EVALUATION INDEX

## ANALYSIS SYSTEM MANAGEMENT INSTITUTION ~130

MANAGEMENT SERVER 3

TEST INSTITUTION ID VARIOUS SERVICES CDx INFORMATION

S102 — SPECIFY CONTRACT TYPE

S103 — START PROVIDING VARIOUS SERVICES

TEST INSTITUTION ID, GENE PANEL ID, GENE ID, ANALYSIS RECORD, QUALITY EVALUATION INDEX

S115 — STORE ANALYSIS RECORD AND QUALITY EVALUATION INDEX

CHARGE SYSTEM USAGE FEE ⟸

S116 — DETERMINE SYSTEM USAGE FEE

FIG. 7

FIG. 8

# FIG. 9

3

## 3A

| TEST INSTITUTION NAME | TEST INSTITUTION ID |
|---|---|
| INSTITUTION P | ppppp |
| INSTITUTION Q | qqqqq |
| INSTITUTION R | rrrrr |
| ... | |

## 3C

| GENE-PANEL-RELATED INFORMATION | THE NUMBER OF TIMES OF OPERATION | PERIOD |
|---|---|---|
| PANEL ID: AAA | 5 | 2017/8/1 - 2017/8/31 |
| PANEL ID: BBB | 10 | 2017/8/1 - 2017/8/31 |
| PANEL ID: CCC | 0 | 2017/8/1 - 2017/8/31 |
| ... | ... | ... |

## 3D

| GENE-PANEL-RELATED INFORMATION | THE NUMBER OF ANALYZED GENES | PERIOD |
|---|---|---|
| PANEL ID: AAA | 4178 | 2017/8/1 - 2017/8/31 |
| PANEL ID: BBB | 2734 | 2017/8/1 - 2017/8/31 |
| PANEL ID: CCC | 153 | 2017/8/1 - 2017/8/31 |
| ... | ... | ... |

## 3E

| GENE-PANEL-RELATED INFORMATION | TOTAL NUMBER OF IDENTIFIED MUTATIONS | PERIOD |
|---|---|---|
| PANEL ID: AAA | 2610 | 2017/8/1 - 2017/8/31 |
| PANEL ID: BBB | 518 | 2017/8/1 - 2017/8/31 |
| PANEL ID: CCC | 94 | 2017/8/1 - 2017/8/31 |
| ... | ... | ... |

## 3F

| NAME OF COMPANY PROVIDING GENE PANEL | PANEL ID | NECESSITY OF SYSTEM USAGE FEE |
|---|---|---|
| COMPANY A | AAA, ... | UNNECESSARY |
| COMPANY B | BBB, ... | NECESSARY |
| COMPANY C | CCC, ... | NECESSARY |
| ... | ... | ... |

## 3B

| CONTRACT TYPE | USABLE GENE PANEL | SYSTEM USAGE FEE |
|---|---|---|
| PLAN 1 | PANEL A PANEL B PANEL C ... | DETERMINE ACCORDING TO THE NUMBER OF TIMES OF OPERATION |
| PLAN 2 | PANEL A PANEL B PANEL C ... | DETERMINE ACCORDING TO THE NUMBER OF ANALYZED GENES |
| CDx USAGE PLAN 3 | PANEL A PANEL B PANEL C ... | DETERMINE ACCORDING TO THE NUMBER OF TIMES OF OPERATION |
| CDx USAGE PLAN 4 | PANEL A PANEL B PANEL C ... | DETERMINE ACCORDING TO THE NUMBER OF ANALYZED GENES |
| ... | ... | |

FIG. 10

FIG. 11

```
         ┌─────────────────────────┐
         │  RECEIVE INPUT OF GENE  │  S107
         │     PANEL INFORMATION   │
         └────────────┬────────────┘
                      │
         ┌────────────┴────────────┐
         │    OBTAIN GENE PANEL    │  S201
         │       INFORMATION       │
         └────────────┬────────────┘
                      │
                      │            S202
                    ╱   ╲
                  ╱       ╲        NO
                ╱ REGISTERED╲──────────────────┐
                ╲ GENE PANEL?╱                  │
                  ╲       ╱                     │
                    ╲   ╱                       │
                     YES                        │
                      │                         │
                      │            S203         │
                    ╱   ╲                       │
                  ╱       ╲        NO           │
                ╱ DESIGNATED╲┄┄┄┄┄┄┄┄┄┄┄┄┄┄┄┄┄┄→│
                ╲ GENE PANEL?╱                   │
                  ╲       ╱                      │
                    ╲   ╱                        │
                     YES                         │
                      │                          ↓
   ┌──────────────────┴──────┐ S204   ┌──────────────────────┐ S205
   │ INDICATE THAT GENE PANEL CAN│     │ INDICATE THAT GENE PANEL │
   │        BE USED          │     │     CANNOT BE USED    │
   └──────────────┬──────────┘     └──────────┬───────────┘
                  │←──────────────────────────┘
                  │
         ┌────────┴────────┐
         │     RETURN      │
         └─────────────────┘
```

FIG. 12

○ GENE PANEL 1 (GENE PANEL NAME: "xxxxx")

◉ GENE PANEL 2 (GENE PANEL NAME: "yyyyy")

○ GENE PANEL 3 (GENE PANEL NAME: "zzzzzz")

⋮

○ UNREGISTERED PANEL

FIG. 13

121

121A

| GENE NAME | GENE ID |
|-----------|---------|
| . . . | . . . |
| EGFR | aaa |
| BRAF | bbb |
| Ros1 | ccc |
| . . . | . . . |

121B

| GENE PANEL NAME | GENE PANEL ID | RELATED GENE ID | CDx FLAG |
|-----------------|---------------|-----------------|----------|
| . . . | . . . | . . . | . . . |
| PANEL A | AAA | aaa, bbb, ccc, · · · | 1 |
| PANEL B | BBB | aaa, bbb, ccc, ddd, · · · | 1 |
| PANEL C | CCC | aaa, ccc, · · · | 0 |
| . . . | . . . | . . . | . . . |

FIG. 14

| ☑ AKT1 | ☑ EGFR | ☐ JAK3 | · · · |
|---------|---------|---------|-------|
| ☑ APC | ☑ ESR1 | ☑ KIT | · · · |
| ☑ ALK | ☐ EML4 | ☑ MET | · · · |
| ☑ BRAF | ☐ FBXW7 | ☑ PIK3CA | · · · |
| · · · | · · · | · · · | · · · |

FIG. 15

○ LUNG CANCER

● COLON CANCER

○ BREAST CANCER

⋮

FIG. 16

REGISTRATION FILE NAME

GENE PANEL TARGET GENE.csv     REGISTER

| RET |
| CHEK2 |
| PTEN |
| MEK1 |

FIG. 17

GENE NAME

FBXW7     REGISTER

FIG. 18

```
        ┌─────────────────┐
        │     START       │
        └─────────────────┘
                 │
    ┌─────────────────────────┐
    │      PRETREATMENT       │ ─ S31
    └─────────────────────────┘
                 │
    ┌─────────────────────────┐
    │       SEQUENCING        │ ─ S32
    └─────────────────────────┘
                 │
    ┌─────────────────────────┐
    │  ANALYZE GENE SEQUENCE  │ ─ S33
    └─────────────────────────┘
                 │
    ┌─────────────────────────┐
    │ EVALUATE QUALITY OF PANEL TEST │ ─ S34
    └─────────────────────────┘
                 │
    ┌─────────────────────────┐
    │      CREATE REPORT      │ ─ S35
    └─────────────────────────┘
                 │
        ┌─────────────────┐
        │      END        │
        └─────────────────┘
```

# FIG. 19

```
          START
            │
┌───────────────────────────────┐
│ EXTRACT DNA OF SAMPLE AND QUALITY  │  S300
│ CONTROL SAMPLE CORRESPONDING TO GENE│
│        PANEL TO BE USED            │
└───────────────────────────────┘
            │
┌───────────────────────────────┐
│     FRAGMENT EXTRACTED DNA         │  S301
└───────────────────────────────┘
            │
┌───────────────────────────────┐
│     PROVIDE ADAPTER SEQUENCE       │  S302
└───────────────────────────────┘
            │
┌───────────────────────────────┐
│          HYBRIDIZE                 │  S303
└───────────────────────────────┘
            │
┌───────────────────────────────┐
│       COLLECT DNA FRAGMENT         │  S304
└───────────────────────────────┘
            │
          END
```

FIG. 20

```
        ┌─────────────────┐
        │      START      │
        └─────────────────┘
                 │
 ┌───────────────────────────────────────┐
 │ EXTRACT DNA OF QUALITY CONTROL SAMPLE  │──⌐ S300a
 └───────────────────────────────────────┘
                 │
 ┌───────────────────────────────────────┐
 │        FRAGMENT EXTRACTED DNA          │──⌐ S301
 └───────────────────────────────────────┘
                 │
 ┌───────────────────────────────────────┐
 │        PROVIDE ADAPTER SEQUENCE        │──⌐ S302
 └───────────────────────────────────────┘
                 │
 ┌───────────────────────────────────────┐
 │              HYBRIDIZE                 │──⌐ S303
 └───────────────────────────────────────┘
                 │
 ┌───────────────────────────────────────┐
 │          COLLECT DNA FRAGMENT          │──⌐ S304
 └───────────────────────────────────────┘
                 │
        ┌─────────────────┐
        │       END       │
        └─────────────────┘
```

FIG. 21

```
            ┌─────────────────────┐
            │        START        │
            └─────────────────────┘
                       │
    ┌──────────────────────────────────────┐
    │ EXTRACT DNA OF QUALITY CONTROL SAMPLE │
    │ INCLUDING MUTATION AND QUALITY CONTROL│ ⌐ S300b
    │    SAMPLE NOT INCLUDING MUTATION      │
    └──────────────────────────────────────┘
                       │
    ┌──────────────────────────────────────┐
    │        FRAGMENT EXTRACTED DNA         │ ⌐ S301
    └──────────────────────────────────────┘
                       │
    ┌──────────────────────────────────────┐
    │        PROVIDE ADAPTER SEQUENCE       │ ⌐ S302
    └──────────────────────────────────────┘
                       │
    ┌──────────────────────────────────────┐
    │              HYBRIDIZE                │ ⌐ S303
    └──────────────────────────────────────┘
                       │
    ┌──────────────────────────────────────┐
    │         COLLECT DNA FRAGMENT          │ ⌐ S304
    └──────────────────────────────────────┘
                       │
            ┌─────────────────────┐
            │         END         │
            └─────────────────────┘
```

FIG. 22

```
┌─────────────────────────┐
│          START          │
└─────────────────────────┘
             │
┌─────────────────────────┐
│  EXTRACT DNA OF SAMPLE AND QUALITY  │  ⌿ S300c
│         CONTROL SAMPLE              │
│ (INCLUDING/NOT INCLUDING MUTATION)  │
└─────────────────────────┘
             │
┌─────────────────────────┐
│   FRAGMENT EXTRACTED DNA    │  ⌿ S301
└─────────────────────────┘
             │
┌─────────────────────────┐
│   PROVIDE ADAPTER SEQUENCE  │  ⌿ S302
└─────────────────────────┘
             │
┌─────────────────────────┐
│         HYBRIDIZE           │  ⌿ S303
└─────────────────────────┘
             │
┌─────────────────────────┐
│    COLLECT DNA FRAGMENT     │  ⌿ S304
└─────────────────────────┘
             │
┌─────────────────────────┐
│           END           │
└─────────────────────────┘
```

FIG. 23A

| PIK3CA | EGFR | IDH1 | ROS1 | · · · |
|--------|------|------|------|-------|
| ESR1 | KRAS | IDH2 | ALK | · · · |
| BRAF | PDGFRA | cKIT | AKT1 | · · · |
| MEK | RET | · · · | · · · | · · · |
| S492R | · · · | · · · | · · · | · · · |
| · · · | · · · | · · · | · · · | · · · |
| · · · | · · · | · · · | · · · | · · · |

FIG. 23B

| Gene Variant Type in Profiling | | | |
|--------|--------|--------|--------|
| SNV | **InDel** | CNV | Fusion |

FIG. 23C

| Mutation Type | Gene | Mutation |
|---------------|------|----------|
| SNV | AAA | aaa |
| InDel | BBB | bbb |
| CNV | CCC | ccc |
| Fusion | DDD | ddd |

FIG. 23D

QUALITY CONTROL
SAMPLE A

| Variant Type | Chromosome Number | Gene | Variant | Expected Allele Freq. |
|--------------|-------------------|------|---------|------------------------|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

FIG. 24

121

121D

| GENE PANEL | QUALITY CONTROL SAMPLE INCLUDING MUTATION | QUALITY CONTROL SAMPLE NOT INCLUDING MUTATION |
|---|---|---|
| PANEL A | STANDARD SAMPLE A1<br>STANDARD SAMPLE A2<br>. <br>. <br>. | STANDARD SAMPLE M1<br>STANDARD SAMPLE M2<br>. <br>. <br>. |
| PANEL B | STANDARD SAMPLE B1<br>STANDARD SAMPLE B2<br>. <br>. <br>. | STANDARD SAMPLE N1<br>STANDARD SAMPLE N2<br>. <br>. <br>. |

## FIG. 25A

QUALITY CONTROL SAMPLE A

| Variant Type | Chromosome Number | Gene | Variant | Expected Allele Freq. |
|---|---|---|---|---|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

a1, a2, a3

## FIG. 25B

QUALITY CONTROL SAMPLE B

| Variant Type | Chromosome Number | Gene | Variant | Expected Allele Freq. |
|---|---|---|---|---|
| Verified Mutations | | | | |
| SNV High GC | chr.19 | GNA11 | R183C | 5.9 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.16 | TSC2 | ΔH1746-R1751 | 5.5 |
| Fusion | chr.4:chr.6 | ROS1 | SLC34A2/ROS1 fusion | 5.6 |
| Fusion | chr.10 | RET | CCDC6/RET fusion | 5.0 |
| Fusion | chr.9 | ABL1 | BCR/ABL1 fusion | 5.1 copies |
| CNV | chr.2 | MYC-N | amplification | 9.5 copies |
| CNV | chr.8 | MYC-C | amplification | 9.8 copies |

b1, b2, b3

FIG. 26A

FRAGMENTATION OF DNA

DNA OF SAMPLE AND QUALITY CONTROL SAMPLE

FRAGMENTATION

FIG. 26B

PROVISION OF ADAPTER SEQUENCE

SUBJECT-DERIVED SAMPLE AND QUALITY CONTROL SAMPLE

DNA FRAGMENT

ADAPTER 1 SEQUENCE

ADAPTER 2 SEQUENCE

# FIG. 27

FIG. 28

COLLECT DNA FRAGMENT TO BE ANALYZED

STREPTAVIDIN MAGNETIC BEAD

STREPTAVIDIN

BIOTIN

MAGNETIC BEAD

WASH AND COLLECT

WASH AND REMOVE DNA FRAGMENT
THAT WERE NOT HYBRIDIZED

COLLECT HYBRIDIZED DNA
FRAGMENT BY MAGNET

MAGNET

FIG. 29

```
        ┌──────────────┐
        │    START     │
        └──────┬───────┘
               │
    ┌──────────┴───────────┐
    │   ADD INDEX SEQUENCE TO   │
    │  DNA FRAGMENT PREPARED FROM │ ⌐S304a
    │   SUBJECT-DERIVED SAMPLE    │
    └──────────┬───────────┘
               │
    ┌──────────┴───────────┐
    │                      │ ⌐S304b
    │ PREPARE MEASUREMENT SAMPLE │
    └──────────┬───────────┘
               │
        ┌──────┴───────┐
        │     END      │
        └──────────────┘
```

FIG. 30

| | | |
|---|---|---|---|---|---|---|
| SEQUENCING DATE | 20xx/xx/xx | | | | | |
| OPERATOR ID | X | | | | | |
| SESSION ID | Y | | | | | |
| APPLICATION TYPE | A | | | | | |
| SAMPLE GENE | PCR PRODUCT | | | | | |
| READ SEQUENCE LENGTH | n (base) | | | | | |
| ADAPTER 1 SEQUENCE | aaaaaaaaaa | | | | | |
| ADAPTER 2 SEQUENCE | bbbbbbbbbb | | | | | |
| LANE NUMBER | SAMPLE ID | SUBJECT ID | INDEX SEQUENCE ID | INDEX SEQUENCE | ··· | REMARK |
| 1 | xx | A | Idx1 | KKKKKKKK | ··· | ··· |
| 2 | yy | A | Idx2 | DDDDDDDD | ··· | ··· |
| 3 | zz | B | Idx3 | SSSSSSSS | ··· | ··· |
| ··· | ··· | ··· | ··· | ··· | ··· | ··· |

SETTING INFORMATION

SAMPLE INFORMATION

FIG. 31

SUBJECT-DERIVED
SAMPLE TO BE ANALYZED

APPLY TO FLOW CELL

FIG. 32

SUBJECT-DERIVED
SAMPLE TO BE ANALYZED

NON-SUBJECT-DERIVED
NUCLEIC ACID

APPLY TO FLOW CELL

FIG. 33

SUBJECT-DERIVED SAMPLE
TO BE ANALYZED

ANALYZED SAMPLE
(NOT TO BE ANALYZED)

APPLY TO FLOW CELL

FIG. 34

START

APPLY MEASUREMENT SAMPLE TO FLOW CELL — S305

AMPLIFY DNA FRAGMENT TO BE ANALYZED — S306

READ SEQUENCE — S307

END

FIG. 35

FIG. 36

AMPLIFY DNA FRAGMENT TO BE ANALYZED (Bridge PCR)

FIG. 37

FIG. 38

```
            ┌─────────────┐
            │    START    │
            └─────────────┘
                   │
                   │
                  ╱ ╲  S51
                 ╱   ╲
                ╱ SEQUENCE ╲
               ╱INFORMATION IS╲    NO
              ◄ ASSOCIATED WITH INDEX ├──────────────┐
               ╲  SEQUENCE  ╱                         │
                ╲    ?    ╱                           │
                 ╲      ╱                             │
                  ╲ YES╱                              │
                   │                                  │
                   │                                  ▼
      S52 ┌─────────────────┐          S53 ┌──────────────────┐
          │     ANALYZE     │              │  DO NOT ANALYZE  │
          └─────────────────┘              └──────────────────┘
                   │                                  │
                   │◄─────────────────────────────────┘
                   │
            ┌─────────────┐
            │     END     │
            └─────────────┘
```

FIG. 39

```
        ┌─────────────┐
        │    START    │
        └──────┬──────┘
               │
               ▼
          ╱─────────╲  S51a
         ╱ SEQUENCE  ╲
        ╱ INFORMATION ╲   NO
       ╱ IS ASSOCIATED ╲──────────────┐
       ╲ WITH INDEX    ╱              │
        ╲ SEQUENCE    ╱               │
         ╲    ?      ╱                │
          ╲─────────╱                 │
               │ YES                  │
               ▼                      │
          ╱─────────╲  S51b           │
         ╱           ╲   NO           │
        ╱ FIRST INDEX ╲───────────────┤
        ╲ INFORMATION?╱               │
         ╲           ╱                │
          ╲─────────╱                 │
               │ YES                  ▼
        ┌──────┴──────┐ S52    ┌──────────────┐ S53
        │   ANALYZE   │        │ DO NOT ANALYZE│
        └──────┬──────┘        └───────┬──────┘
               │                       │
               ◄───────────────────────┘
               │
               ▼
        ┌─────────────┐
        │     END     │
        └─────────────┘
```

FIG. 40

```
                    START

┌─────────────────────────────────┐
│   CREATE FILE FOR EACH SAMPLE    │
│   BASED ON INDEX SEQUENCE AND    │ ─ S10
│     SORT SEQUENCE INFORMATION    │
└─────────────────────────────────┘

┌─────────────────────────────────┐
│  READ SEQUENCE INFORMATION STORED│
│  IN FILE OF SEQUENCE INFORMATION │ ─ S11
│          TO BE ANALYZED          │
└─────────────────────────────────┘

┌─────────────────────────────────┐
│                                  │
│       PERFORM ALIGNMENT          │ ─ S12
│                                  │
└─────────────────────────────────┘

┌─────────────────────────────────┐
│   COMPARE ALIGNMENT SEQUENCE OF  │
│   BLOOD SAMPLE WITH ALIGNMENT    │ ─ S13
│     SEQUENCE OF TUMOR SAMPLE     │
└─────────────────────────────────┘

┌─────────────────────────────────┐
│   EXTRACT DIFFERENCE BETWEEN THE │ ─ S14
│       SEQUENCES AS MUTATION      │
└─────────────────────────────────┘

┌─────────────────────────────────┐
│  SEARCH MUTATION DATABASE BASED ON│ ─ S15
│        EXTRACTED MUTATION        │
└─────────────────────────────────┘

┌─────────────────────────────────┐
│  PROVIDE ANNOTATION TO EXTRACTED │ ─ S16
│   MUTATION BASED ON SEARCH RESULT│
└─────────────────────────────────┘

                    END
```

FIG. 41

- SEQUENCE NAME: "Read Seq 1"
- SEQUENCE:     ···GTAAGGCACGTCATA···
- QUALITY SCORE:            ···xxxxxyzxxyzzxxx···

REFERENCE
SEQUENCE

MAPPING

READ SEQUENCE

- REFERENCE SEQUENCE INFORMATION: REFERENCE SEQUENCE NAME (REFERENCE SEQUENCE ID),
  SEQUENCE LENGTH OF REFERENCE SEQUENCE, etc.

- READ SEQUENCE NAME: NAME (READ SEQUENCE ID) OF EACH READ SEQUENCE HAVING BEEN SUBJECTED
  TO ALIGNMENT

- POSITION INFORMATION: Leftmost mapping position.  POSITION ON REFERENCE SEQUENCE AT WHICH
  LEFTMOST BASE OF READ SEQUENCE WAS MAPPED

- MAP QUALITY: MAPPING QUALITY CORRESPONDING TO THE READ SEQUENCE

- SEQUENCE: BASE SEQUENCE CORRESPONDING TO EACH READ SEQUENCE (EXAMPLE: …GTAAGGCACGTCATA…)

FIG. 43

FIG. 44

| GENE NAME | CHROMOSOME POSITION | MUTATION |
|---|---|---|
| AKT1 | 14q32.33 | p.E17K |
| BRAF | 7q34 | p.V600E, p.V600K, p.V600, p.V600R, p.K601E, p.D594G, p.G469A, p.D594N, p.V600M, p.V600_K601>E, p.G466V, p.V600D, p.L597R, p.G469V, p.N581S, p.V600L, p.G469R, p.G469E, p.G466E, p.L597Q, p.L597S, p.V600A |
| EGFR | 7p11.2 | p.L858R, p.E746_A750del, p.E746_A750delELREA, p.T790M, p.L861Q, p.G719, p.S768I, p.L747_P753>S, p.G719A, p.L747_T751del, p.L747_A750>P, p.G719S, p.L747_T751delLREAT, p.E746_S752>V, c.3633+294delA, p.V769_D770insASV, p.L747_S752del, p.G719C, p.L747_S752delLREATS, p.G598V, p.A289V, c.3633+293_3633+294delAA, p.L747_P753del, p.D770_N771insSVD, p.E746_T751del, p.L747_T751>P, p.E709K, p.E746_T751>A, p.L833V, p.N158N, p.K745_A750del, p.H773_V774insNPH, p.C797S |
| . . . | . . . | . . . |

FIG. 45

```
                    ┌──────────────┐
                    │    START     │
                    └──────────────┘
                            │
    ┌───────────────────────┼──────────────────────────────┐
    │                       ▼                                │
    │     ┌─────────────────────────────────┐  ┌ S401       │
    │     │  COMPARE READ SEQUENCE WITH     │               │
    │     │     REFERENCE SEQUENCE          │               │
    │     └─────────────────────────────────┘               │
    │                       │                                │
    │     ┌─────────────────────────────────┐               │
    │     │  SPECIFY POSITION ON REFERENCE  │  ┌ S402        │
    │     │  SEQUENCE AT WHICH DEGREE OF    │               │
    │     │  MATCHING WITH READ SEQUENCE    │               │
    │     │ SATISFIES PREDETERMINED CRITERION│              │
    │     └─────────────────────────────────┘               │
```

COMPARE READ SEQUENCE WITH REFERENCE SEQUENCE ┌S401

SPECIFY POSITION ON REFERENCE SEQUENCE AT WHICH DEGREE OF MATCHING WITH READ SEQUENCE SATISFIES PREDETERMINED CRITERION ┌S402

┌S403 MATCH AT MULTIPLE POSITIONS ? — YES

NO

ALIGN READ SEQUENCE TO POSITION OF HIGHEST DEGREE OF MATCHING ┌S404

┌S405 ALL READ SEQUENCES HAVE BEEN ALIGNED ? — NO

YES

END

FIG. 46A

REFERENCE SEQUENCE:   ···GCCATGGACAGAAGGCGCAGGGC···
READ SEQUENCE R1:        GCCATGGACAGAA
READ SEQUENCE R2:        GCCATGCACAGAA

FIG. 46B

REFERENCE SEQUENCE:   ···GCCATGGACAGAAGGCGCAGGGC···
READ SEQUENCE R3:        GCCATGGACAG**GGCG
READ SEQUENCE R4:        GCCATGGACAGAAGGCG
                                        ⌣
                                       CGGT

FIG. 47

EP 3 627 513 A1

· POSITION INFORMATION: POSITION INFORMATION ON REFERENCE GENOME. FOR EXAMPLE, SPECIFIED BY CHROMOSOME NUMBER AND POSITION ON THE CHROMOSOME.

· REFERENCE BASE: REFERENCE BASE (A,T,C,G, etc.) IN THE POSITION INFORMATION

· MUTATION BASE: BASE OF REFERENCE BASE AFTER MUTATION

| CHROM | POS | REF | ALT | |
|---|---|---|---|---|
| 20 | 3 | C | G | |
| 21 | 4 | C | CTAG | ← EXAMPLE OF INSERTION MUTATION |
| 19 | 10 | TCG | T | ← EXAMPLE OF DELETION MUTATION |
| 2 | 321681 | G | G]17:198982] | ← REVERSE COMPLEMENT SEQUENCE OF SEQUENCE EXTENDING AT LEFT OF 198982 POSITION OF CHROMOSOME 17 IS BOUND AFTER "G" |
| 2 | 321682 | T | ]13:123456]T | ← SEQUENCE AT LEFT OF 123456 POSITION OF CHROMOSOME 13 IS BOUND BEFORE "T" |
| 13 | 123456 | C | C[2:321682[ | ← SEQUENCE AT RIGHT OF 321682 POSITION OF CHROMOSOME 2 IS BOUND AFTER "C" |
| 13 | 123457 | A | [17:198983[A | ← REVERSE COMPLEMENT SEQUENCE OF SEQUENCE EXTENDING AT RIGHT OF 198983 POSITION OF CHROMOSOME 17 IS BOUND AFTER "C" |

FIG. 48

EP 3 627 513 A1

123

| MUTATION ID | CHROM | POS | REF | ALT | Annotation | METADATA RELATED TO GENE-PANEL-RELATED INFORMATION |
|---|---|---|---|---|---|---|
| #1 | 20 | 3 | C | G | abc | GENE ID: a |
| #2 | 19 | 4 | A | T | xyz | GENE ID: b |
| #3 | xx | yy | C | G | EGFR L858R | GENE ID: c |
| #4 | aa | bb | T | A | BRAF V600E | GENE ID: d |
| . . . | | | | | | |
| #xx | abc | ABC | G | G]p] | ALK-EML4 FUSION | GENE ID: e, GENE ID: f |
| #yy | xyz | XYZ | T | ]p]T | ROS1-CD74 FUSION | GENE ID: g, GENE ID: h |
| . . . | | | | | | |

FIG. 49

- MUTATION ID: IDENTIFIER FOR IDENTIFYING MUTATION
- MUTATION POSITION INFORMATION:
  - ➤ CHROM: CHROMOSOME NUMBER
  - ➤ POS: POSITION AT CHROMOSOME NUMBER
- REF: WILD TYPE BASE
- ALT : BASE AFTER MUTATION
- Annotation: INFORMATION RELATED TO MUTATION

FIG. 50A

| GENE | ID | CHROM | POS |
|------|-----|-------|-----|
| A | aaa | 19 | 10 |
| B | bbb | 20 | 3 |
| C | ccc | 21 | 4 |

FIG. 50B

```
Ex)
CHROM      POS          REF          ALT
20         3            C            G
21         4            C            CTAG
19         10           TCG          T
2          321681       G            G]17:198982]
2          321682       T            ]13:123456]T
13         123456       C            C[2:321682[
13         123457       A            [17:198983[A
```

EP 3 627 513 A1

FIG. 51

```
        ┌─────────────────┐
        │      START      │
        └─────────────────┘
                 │
    ┌────────────────────────────┐
    │ SEARCH DRUG DATABASE BASED ON │  ⌐S15a
    │ INFORMATION RELATED TO MUTATION │
    └────────────────────────────┘
                 │
    ┌────────────────────────────┐
    │ GENERATE LIST OF DRUG RELATED TO │  ⌐S16a
    │          MUTATION          │
    └────────────────────────────┘
                 │
        ┌─────────────────┐
        │       END       │
        └─────────────────┘
```

FIG. 52

⌐124A

| MUTATION ID | DRUG ID | RELATED DRUG | METADATA RELATED TO GENE-PANEL-RELATED INFORMATION |
|---|---|---|---|
| . . . | | | |
| #3 | #a | DRUG A | GENE ID: a |
| #3 | #b | DRUG B | GENE ID: b |
| #4 | #d | DRUG C | GENE ID: c |
| . . . | | | |

FIG. 53

124B

| MUTATION ID | DRUG ID | RELATED DRUG | APPROVAL STATE | CDx FLAG | APPROVED COUNTRY | TARGET DISEASE ID | METADATA CORRESPONDING TO GENE-PANEL-RELATED INFORMATION |
|---|---|---|---|---|---|---|---|
| ... | | | | | | | |
| #3 | #a | DRUG A | APPROVED | 1 | USA, JAPAN | #A (LUNG CANCER) | ... |
| #3 | #b | DRUG B | APPROVED | 1 | USA, JAPAN | #A (LUNG CANCER) | ... |
| ... | | | | | | | |
| #xx | #X | xyz | UNAPPROVED | 0 | - | - | |
| #yy | #Y | abc | UNAPPROVED | 0 | - | - | |

FIG. 54

```
        ( START )
            |
  ┌──────────────────────────────┐
  │ SEARCH DRUG DATABASE BASED ON │ ⌐ S15b
  │ INFORMATION RELATED TO MUTATION│
  └──────────────────────────────┘
            |
  ┌──────────────────────────────┐
  │ GENERATE LIST INCLUDING INFORMATION │ ⌐ S16b
  │ RELATED TO APPROVAL OF DRUG   │
  └──────────────────────────────┘
            |
         ( END )
```

FIG. 55

_124C

| MUTATION ID | DRUG ID | RELATED DRUG | CLINICAL TRIAL/CLINICAL STUDY STATE | COUNTRY | PHASE | TARGET DISEASE | INSTITUTION | METADATA RELATED TO GENE-PANEL-RELATED INFORMATION |
|---|---|---|---|---|---|---|---|---|
| . . . | | | | | | | | |
| #xx | #X | xyz | IN CLINICAL TRIAL | JAPAN | Phase 3 | DISEASE ID (LUNG CANCER) | abc PHARMACY | . . . |
| #yy | #Y | abc | None | – | – | | | . . . |

FIG. 56

```
            ┌─────────────────┐
            │      START       │
            └────────┬─────────┘
                     │
     ┌───────────────────────────────┐
     │  SEARCH DRUG DATABASE BASED ON │ ┌ S15c
     │  INFORMATION RELATED TO MUTATION│
     └───────────────┬───────────────┘
                     │
     ┌───────────────────────────────┐
     │ GENERATE LIST INCLUDING INFORMATION│ ┌ S16c
     │  RELATED TO CLINICAL TRIAL OF DRUG │
     └───────────────┬───────────────┘
                     │
            ┌─────────────────┐
            │       END        │
            └─────────────────┘
```

## FIG. 57

### GENE ANALYSIS RESULT REPORT

PATIENT ID: ███████

SEX: MALE

DISEASE NAME: xxx CANCER   STAGE Ⅲ

NAME OF DOCTOR IN CHARGE: ███████

INSTITUTION NAME: ███████

GENE PANEL: PANEL C

QC INDEX: XXXXXXX

REQUEST DAY:        × ×YEAR ×MONTH × ×DAY

MEASUREMENT DAY: × ×YEAR ×MONTH × ×DAY

REPORT DAY:        × ×YEAR ×MONTH × ×DAY

| GENE | MUTATION | AMINO ACID MUTATION | NUCLEOTIDE MUTATION | RELATED DRUG |
|------|----------|---------------------|---------------------|--------------|
| BRAF | V600E | p.Val600Glu | c.1799T>A | DRUG D |
| XXXXX | XXXXX | XXXXX | XXXX | XXXXX |

FIG. 58

FIG. 59

```
            ╭──────────────────────╮
            │      GENE TEST       │
            ╰──────────────────────╯
                      │
    ┌─────────────────────────────────────┐
    │ CONSENT BY SUBJECT TO USE GENE TEST │ ⌐S91
    └─────────────────────────────────────┘
                      │
    ┌─────────────────────────────────────┐
    │       COLLECT TISSUE AS SAMPLE      │ ⌐S92
    └─────────────────────────────────────┘
                      │
    ┌─────────────────────────────────────┐
    │ PRETREATMENT OF GENE EXTRACTED FROM │ ⌐S93
    │        SAMPLE AND SEQUENCING        │
    └─────────────────────────────────────┘
                      │
    ┌─────────────────────────────────────┐
    │  ANALYZE SEQUENCE INFORMATION AND   │ ⌐S94
    │   DETECT ABNORMALITY IN GENE TO BE  │
    │               ANALYZED              │
    └─────────────────────────────────────┘
                      │
    ┌─────────────────────────────────────┐
    │     CREATE REPORT INCLUDING QUALITY │
    │ EVALUATION INDEX INDICATING QUALITY │ ⌐S95
    │ OF GENE TEST AND INFORMATION RELATED│
    │        TO DETECTED ABNORMALITY      │
    └─────────────────────────────────────┘
                      │
    ┌─────────────────────────────────────┐
    │       DETERMINE SIGNIFICANCE OF     │ ⌐S96
    │    INFORMATION INCLUDED IN REPORT BY│
    │             EXPERT PANEL            │
    └─────────────────────────────────────┘
                      │
    ┌─────────────────────────────────────┐
    │     EXPLAIN RESULT OF GENE TEST TO  │ ⌐S97
    │   SUBJECT AND SELECT THERAPEUTIC    │
    │               STRATEGY              │
    └─────────────────────────────────────┘
                      │
            ╭──────────────────────╮
            │         END          │
            ╰──────────────────────╯
```

FIG. 60

FIG. 61

FLOW CELL

**EUROPEAN SEARCH REPORT**

Application Number

EP 19 19 3572

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVID W CRAIG ET AL: "Identification of genetic variants using bar-coded multiplexed sequencing", NATURE METHODS, NATURE PUB. GROUP, NEW YORK, vol. 5, no. 10, 1 October 2008 (2008-10-01), pages 887-893, XP002662394, ISSN: 1548-7091, DOI: 10.1038/NMETH.1251 [retrieved on 2008-09-14] | 1-8, 11-16 | INV. G16B20/20 G16B30/20 |
| Y | * whole document, in particular title, p. 888, 892, fig. 1-4 * | 9,10 | |
| A | ABHISHEK MITRA ET AL: "Strategies for Achieving High Sequencing Accuracy for Low Diversity Samples and Avoiding Sample Bleeding Using Illumina Platform", PLOS ONE, vol. 10, no. 4, 1 January 2015 (2015-01-01), page e0120520, XP055669098, DOI: 10.1371/journal.pone.0120520 * the whole document * | 1-15 | |
| Y | US 2017/220735 A1 (DUENWALD SVEN [US] ET AL) 3 August 2017 (2017-08-03) * whole doc, in particular [0340] * | 9,10 | |

TECHNICAL FIELDS SEARCHED (IPC)

G16B

The present search report has been drawn up for all claims

| Place of search | Date of completion of the search | Examiner |
|---|---|---|
| Munich | 18 February 2020 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 3572

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2020

| Patent document cited in search report | Publication date | Patent family member(s) | Publication date |
|---|---|---|---|
| US 2017220735 A1 | 03-08-2017 | AR 107192 A1 | 28-03-2018 |
| | | AU 2016391100 A1 | 27-09-2018 |
| | | AU 2019203491 A1 | 06-06-2019 |
| | | BR 112018015913 A2 | 22-01-2019 |
| | | CA 3013572 A1 | 10-08-2017 |
| | | CN 108884491 A | 23-11-2018 |
| | | DK 3202915 T3 | 24-06-2019 |
| | | EA 201891580 A1 | 31-01-2019 |
| | | EP 3202915 A1 | 09-08-2017 |
| | | EP 3517626 A1 | 31-07-2019 |
| | | KR 20180123020 A | 14-11-2018 |
| | | KR 20190132558 A | 27-11-2019 |
| | | NZ 745637 A | 31-05-2019 |
| | | SG 11201806595U A | 27-09-2018 |
| | | TW 201805429 A | 16-02-2018 |
| | | TW 201930598 A | 01-08-2019 |
| | | US 2017220735 A1 | 03-08-2017 |
| | | US 2019065676 A1 | 28-02-2019 |
| | | WO 2017136059 A1 | 10-08-2017 |
| | | ZA 201805753 B | 24-04-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- An introduction to Next-Generation Sequencing Technology. Illumina, Inc, 30 August 2018